# EUROPEAN PATENT APPLICATION

(11) **EP 3 536 707 A1**
(43) Date of publication of application: **11.09.2019**
(21) Application number: 19152625.0
(22) Date of filing: 05.11.2015
(51) Int. Cl.: C07K 16/28, C07K 16/30, C07K 14/705, C07K 14/725, A61K 35/17, A61K 39/00

(54) **GENE MODIFIED IMMUNE EFFECTOR CELLS AND ENGINEERED CELLS FOR EXPANSION OF IMMUNE EFFECTOR CELLS**

(30) Priority: 05.11.2014 US 201462075561 P; 05.11.2014 US 201462075642 P; 05.11.2014 US 201462075667 P; 02.06.2015 US 201562169979 P
(62) Divisional of application: 15795304.3
(71) Applicant: Board of Regents, The University of Texas System, Austin, TX 78701 (US)
(72) Inventor: Cooper, Lawrence J.N., Houston, TX 77030 (US); Singh, Harjeet, Houston, TX 77030 (US); Huls, Helen, Houston, TX 77030 (US); Oliverares, Simon, Houston, TX 77030 (US); Jena, Bipulendu, Houston, TX 77030 (US); Patel, Krina, Houston, TX 77030 (US)
(74) Representative: Grünecker Patent- und Rechtsanwälte PartG mbB

(57) **Abstract**

Chimeric antigen receptor (CAR) polypeptides (and immune effector cells expressing the CARs) are provided comprising modified hinge domain sequences. Also provided are engineered antigen presenting cells (APCs) that express transgenes encoding target antigen and human leukocyte antigen (HLA). In further aspect, immune effector cells are provided that have been selected for elevated mitochondrial spare respiratory capacity.

SEQUENCE LISTING

<110>  BOARD OF REGENTS, THE UNIVERSITY OF TEXAS SYSTEM

<120>  GENE MODIFIED IMMUNE EFFECTOR CELLS AND ENGINEERED CELLS FOR
       EXPANSION OF IMMUNE EFFECTOR CELLS

<130>  EP112439IFZMGpau

<140>  not yet assigned
<141>  2015-11-05

<150>  EP15795304.3
<151>  2015-11-05

<150>  PCT/US2015/059293
<151>  2015-11-05

<150>  US 62/075,561
<151>  2014-11-05

<150>  US 62/075,642
<151>  2014-11-05

<150>  US 62/075,667
<151>  2014-11-05

<150>  US 62/169,979
<151>  2015-06-02

<160>  28

<170>  PatentIn version 3.5

<210>  1
<211>  230
<212>  PRT
<213>  Artificial Sequence

<220>
<223>  Synthetic IgG4 Fc region

<400>  1

Glu Ser Lys Tyr Gly Pro Pro Cys Pro Pro Cys Pro Ala Pro Glu Phe
1               5                   10                  15

Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Gln Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Met
225                 230


<210>  2
<211>  75
<212>  PRT
<213>  Homo sapiens

<400>  2

Lys Pro Thr Thr Thr Pro Ala Pro Arg Pro Pro Thr Pro Ala Pro Thr
1               5                   10                  15

Ile Ala Ser Gln Pro Leu Ser Leu Arg Pro Glu Ala Cys Arg Pro Ala
            20                  25                  30

Ala Gly Gly Ala Val His Thr Arg Gly Leu Asp Phe Ala Cys Asp Ile
        35                  40                  45

Tyr Ile Trp Ala Pro Leu Ala Gly Thr Cys Gly Val Leu Leu Leu Ser
    50                  55                  60

Leu Val Ile Thr Leu Tyr Cys Asn His Arg Asn
65                  70                  75

<210>  3
<211>  12
<212>  PRT
<213>  Artificial Sequence

<220>
<223>  Synthetic IgG4 hinge region

<400>  3

Glu Ser Lys Tyr Gly Pro Pro Cys Pro Pro Cys Pro
1               5                   10

<210>  4
<211>  422
<212>  PRT
<213>  Artificial Sequence

<220>
<223>  Synthetic IL-15-IL-15 Receptor Fusion

<400>  4

Met Asp Trp Thr Trp Ile Leu Phe Leu Val Ala Ala Ala Thr Arg Val
1               5                   10                  15

His Ser Asn Trp Val Asn Val Ile Ser Asp Leu Lys Lys Ile Glu Asp
            20                  25                  30

Leu Ile Gln Ser Met His Ile Asp Ala Thr Leu Tyr Thr Glu Ser Asp
        35                  40                  45

Val His Pro Ser Cys Lys Val Thr Ala Met Lys Cys Phe Leu Leu Glu
    50                  55                  60

Leu Gln Val Ile Ser Leu Glu Ser Gly Asp Ala Ser Ile His Asp Thr
65                  70                  75                  80

Val Glu Asn Leu Ile Ile Leu Ala Asn Asn Ser Leu Ser Ser Asn Gly
                85                  90                  95

Asn Val Thr Glu Ser Gly Cys Lys Glu Cys Glu Glu Leu Glu Glu Lys
            100                 105                 110

Asn Ile Lys Glu Phe Leu Gln Ser Phe Val His Ile Val Gln Met Phe
        115                 120                 125

Ile Asn Thr Ser Ser Gly Gly Gly Ser Gly Gly Gly Gly Ser Gly Gly
    130                 135                 140

Gly Gly Ser Gly Gly Gly Gly Ser Gly Gly Gly Ser Leu Gln Ile Thr
145                 150                 155                 160

Cys Pro Pro Pro Met Ser Val Glu His Ala Asp Ile Trp Val Lys Ser
                165                 170                 175

Tyr Ser Leu Tyr Ser Arg Glu Arg Tyr Ile Cys Asn Ser Gly Phe Lys
            180                 185                 190

Arg Lys Ala Gly Thr Ser Ser Leu Thr Glu Cys Val Leu Asn Lys Ala
        195                 200                 205

Thr Asn Val Ala His Trp Thr Thr Pro Ser Leu Lys Cys Ile Arg Asp
    210                 215                 220

Pro Ala Leu Val His Gln Arg Pro Ala Pro Pro Ser Thr Val Thr Thr
225                 230                 235                 240

Ala Gly Val Thr Pro Gln Pro Glu Ser Leu Ser Pro Ser Gly Lys Glu
                245                 250                 255

Pro Ala Ala Ser Ser Pro Ser Ser Asn Asn Thr Ala Ala Thr Thr Ala
            260                 265                 270

Ala Ile Val Pro Gly Ser Gln Leu Met Pro Ser Lys Ser Pro Ser Thr
        275                 280                 285

Gly Thr Thr Glu Ile Ser Ser His Glu Ser Ser His Gly Thr Pro Ser
    290                 295                 300

Gln Thr Thr Ala Lys Asn Trp Glu Leu Thr Ala Ser Ala Ser His Gln
305                 310                 315                 320

Pro Pro Gly Val Tyr Pro Gln Gly His Ser Asp Thr Thr Val Ala Ile
                325                 330                 335

Ser Thr Ser Thr Val Leu Leu Cys Gly Leu Ser Ala Val Ser Leu Leu
            340                 345                 350

Ala Cys Tyr Leu Lys Ser Arg Gln Thr Pro Pro Leu Ala Ser Val Glu
        355                 360                 365

Met Glu Ala Met Glu Ala Leu Pro Val Thr Trp Gly Thr Ser Ser Arg
    370                 375                 380

Asp Glu Asp Leu Glu Asn Cys Ser His His Leu Ser Arg Met Asp Tyr

385                 390                 395                 400

Lys Asp Asp Asp Asp Lys Asp Tyr Lys Asp Asp Asp Asp Lys Asp Tyr
                405                 410                 415

Lys Asp Asp Asp Asp Lys
            420

<210>  5
<211>  22
<212>  PRT
<213>  Homo sapiens

<400>  5

Met Leu Leu Leu Val Thr Ser Leu Leu Leu Cys Glu Leu Pro His Pro
1               5                   10                  15

Ala Phe Leu Leu Ile Pro
            20

<210>  6
<211>  107
<212>  PRT
<213>  Artificial Sequence

<220>
<223>  Synthetic VL domain

<400>  6

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr
            100                 105

<210>  7
<211>  18
<212>  PRT
<213>  Artificial Sequence

<220>
<223>  Whitlow linker peptide

<400>  7

Gly Ser Thr Ser Gly Ser Gly Lys Pro Gly Ser Gly Glu Gly Ser Thr
1               5                   10                  15

Lys Gly

<210>  8
<211>  120
<212>  PRT
<213>  Artificial Sequence

<220>
<223>  Synthetic VH domain

<400>  8

Glu Val Lys Leu Gln Glu Ser Gly Pro Gly Leu Val Ala Pro Ser Gln
1               5                   10                  15

Ser Leu Ser Val Thr Cys Thr Val Ser Gly Val Ser Leu Pro Asp Tyr
            20                  25                  30

Gly Val Ser Trp Ile Arg Gln Pro Pro Arg Lys Gly Leu Glu Trp Leu
        35                  40                  45

Gly Val Ile Trp Gly Ser Glu Thr Thr Tyr Tyr Asn Ser Ala Leu Lys
    50                  55                  60

Ser Arg Leu Thr Ile Ile Lys Asp Asn Ser Lys Ser Gln Val Phe Leu
65                  70                  75                  80

Lys Met Asn Ser Leu Gln Thr Asp Asp Thr Ala Ile Tyr Tyr Cys Ala
                85                  90                  95

Lys His Tyr Tyr Tyr Gly Gly Ser Tyr Ala Met Asp Tyr Trp Gly Gln
            100                 105                 110

Gly Thr Ser Val Thr Val Ser Ser
        115                 120

<210>  9
<211>  230

<212>  PRT
<213>  Homo sapiens

<400>  9

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Met
225                 230

<210>  10
<211>  230
<212>  PRT
<213>  Artificial Sequence

<220>
<223>  Synthetic IgG4 hinge

<400>  10

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys Met
225                 230

<210>  11
<211>  68
<212>  PRT
<213>  Homo sapiens

<400>  11

Phe Trp Val Leu Val Val Val Gly Gly Val Leu Ala Cys Tyr Ser Leu
1               5                   10                  15

Leu Val Thr Val Ala Phe Ile Ile Phe Trp Val Arg Ser Lys Arg Ser
            20                  25                  30

Arg Leu Leu His Ser Asp Tyr Met Asn Met Thr Pro Arg Arg Pro Gly
        35                  40                  45

Pro Thr Arg Lys His Tyr Gln Pro Tyr Ala Pro Pro Arg Asp Phe Ala
    50                  55                  60

Ala Tyr Arg Ser
65

<210>  12
<211>  68
<212>  PRT
<213>  Artificial Sequence

<220>
<223>  Synthetic CD28 transmembrane domain

<400>  12

Phe Trp Val Leu Val Val Val Gly Gly Val Leu Ala Cys Tyr Ser Leu
1               5                   10                  15

Leu Val Thr Val Ala Phe Ile Ile Phe Trp Val Arg Ser Lys Arg Ser
            20                  25                  30

Arg Gly Gly His Ser Asp Tyr Met Asn Met Thr Pro Arg Arg Pro Gly
        35                  40                  45

Pro Thr Arg Lys His Tyr Gln Pro Tyr Ala Pro Pro Arg Asp Phe Ala
    50                  55                  60

Ala Tyr Arg Ser

65

<210>  13
<211>  112
<212>  PRT
<213>  Homo sapiens

<400>  13

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  14
<211>  27
<212>  PRT
<213>  Homo sapiens

<400>  14

Phe Trp Val Leu Val Val Val Gly Gly Val Leu Ala Cys Tyr Ser Leu
1               5                   10                  15

Leu Val Thr Val Ala Phe Ile Ile Phe Trp Val
            20                  25

<210>  15
<211>  42
<212>  PRT
<213>  Homo sapiens

<400>  15

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210> 16
<211> 41
<212> PRT
<213> Homo sapiens

<400> 16

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 17
<211> 41
<212> PRT
<213> Artificial Sequence

<220>
<223> Synthentic CD28 cytoplasmic domain

<400> 17

Arg Ser Lys Arg Ser Arg Gly Gly His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210> 18
<211> 12
<212> PRT
<213> Homo sapiens

<400> 18

Glu Ser Lys Tyr Gly Pro Pro Cys Pro Ser Cys Pro
1               5                   10

<210> 19
<211> 32

<212>  PRT
<213>  Homo sapiens

<400>  19

Phe Ala Cys Asp Ile Tyr Ile Trp Ala Pro Leu Ala Gly Thr Cys Gly
1               5                   10                  15

Val Leu Leu Leu Ser Leu Val Ile Thr Leu Tyr Cys Asn His Arg Asn
            20                  25                  30

<210>  20
<211>  43
<212>  PRT
<213>  Homo sapiens

<400>  20

Lys Pro Thr Thr Thr Pro Ala Pro Arg Pro Pro Thr Pro Ala Pro Thr
1               5                   10                  15

Ile Ala Ser Gln Pro Leu Ser Leu Arg Pro Glu Ala Cys Arg Pro Ala
            20                  25                  30

Ala Gly Gly Ala Val His Thr Arg Gly Leu Asp
        35                  40

<210>  21
<211>  2037
<212>  DNA
<213>  Artificial sequence

<220>
<223>  Synthetic CAR oligonucleotide

<400>  21
atgctgctgc tggtgaccag cctgctgctg tgtgagctgc cccaccccgc ctttctgctg      60

atcccccagt ccgagctgac ccagcccaga tccgtgtccg gctccccagg ccagtccgtg     120

accatctcct gcaccggcac cgagagggac gtgggcggct acaactacgt gtcctggtat     180

cagcagcacc ccggcaaggc ccccaagctg atcatccacg acgtgatcga gaggtcctcc     240

ggcgtgcccg acaggttctc cggctccaag tccggcaaca ccgcctccct gaccatcagc     300

ggactgcagg ccgaggacga ggccgactac tactgctggt ccttcgctgg cggctactac     360

gtgttcggca ccggaaccga cgtgaccgtg ctgggcagca cctccggcag cggcaagcct     420

ggcagcggcg agggcagcac caagggcgaa gtgcagctgc tggaatccgg cggaggactg     480

gtgcagccag gcggctccct gagactgtct tgcgccgcct ccggcttcac cttctccacc     540

taccagatgt cctgggtgcg ccaggcccca ggcaagggcc tggaatgggt gtccggaatc     600

gtgtcctctg gcggctccac cgcctacgcc gactccgtga agggcaggtt caccatctcc     660

agagacaact ccaagaacac cctgtacctg cagatgaact ccctgagggc cgaggacacc 720

gccgtgtact actgtgccgg cgagctgctg ccctactacg gcatggacgt gtggggccag 780

ggcaccaccg tgaccgtgtc ctccgagagc aagtacggcc ctccctgccc cccttgccct 840

gcccccgagt tcctgggcgg acccagcgtg ttcctgttcc cccccaagcc caaggacacc 900

ctgatgatca gccggacccc cgaggtgacc tgtgtggtgg tggacgtgtc ccaggaggac 960

cccgaggtcc agttcaactg gtacgtggac ggcgtggagg tgcacaacgc caagaccaag 1020

ccccgggagg agcagttcaa tagcacctac cgggtggtgt ccgtgctgac cgtgctgcac 1080

caggactggc tgaacggcaa ggaatacaag tgtaaggtgt ccaacaaggg cctgcccagc 1140

agcatcgaga aaaccatcag caaggccaag ggccagcctc gggagcccca ggtgtacacc 1200

ctgcccccta gccaagagga gatgaccaag aatcaggtgt ccctgacctg cctggtgaag 1260

ggcttctacc ccagcgacat cgccgtggag tgggagagca acggccagcc cgagaacaac 1320

tacaagacca ccccccctgt gctggacagc gacggcagct tcttcctgta cagcaggctg 1380

accgtggaca agagccggtg gcaggagggc aacgtcttta gctgctccgt gatgcacgag 1440

gccctgcaca accactacac ccagaagagc ctgtccctga gcctgggcaa gatgttctgg 1500

gtgctggtcg tggtgggtgg cgtgctggcc tgctacagcc tgctggtgac agtggccttc 1560

atcatctttt gggtgaagag aggccggaag aaactgctgt acatcttcaa gcagcccttc 1620

atgcggcccg tgcagaccac ccaggaagag gacggctgca gctgccggtt ccccgaggaa 1680

gaggaaggcg gctgcgaact gcgggtgaag ttcagccgga gcgccgacgc ccctgcctac 1740

cagcagggcc agaaccagct gtacaacgag ctgaacctgg gccggaggga ggagtacgac 1800

gtgctggaca agcggagagg ccgggaccct gagatgggcg gcaagccccg gagaaagaac 1860

cctcaggagg gcctgtataa cgaactgcag aaagacaaga tggccgaggc ctacagcgag 1920

atcggcatga agggcgagcg gcggaggggc aagggccacg acggcctgta ccagggcctg 1980

agcaccgcca ccaaggatac ctacgacgcc ctgcacatgc aggccctgcc ccccaga 2037

<210> 22
<211> 679
<212> PRT
<213> Artificial sequence

<220>
<223> Synthetic CAR polypeptide

<400> 22

Met Leu Leu Leu Val Thr Ser Leu Leu Leu Cys Glu Leu Pro His Pro
1               5                   10                  15

Ala Phe Leu Leu Ile Pro Gln Ser Glu Leu Thr Gln Pro Arg Ser Val
            20                  25                  30

Ser Gly Ser Pro Gly Gln Ser Val Thr Ile Ser Cys Thr Gly Thr Glu
        35                  40                  45

Arg Asp Val Gly Gly Tyr Asn Tyr Val Ser Trp Tyr Gln Gln His Pro
    50                  55                  60

Gly Lys Ala Pro Lys Leu Ile Ile His Asp Val Ile Glu Arg Ser Ser
65                  70                  75                  80

Gly Val Pro Asp Arg Phe Ser Gly Ser Lys Ser Gly Asn Thr Ala Ser
                85                  90                  95

Leu Thr Ile Ser Gly Leu Gln Ala Glu Asp Glu Ala Asp Tyr Tyr Cys
            100                 105                 110

Trp Ser Phe Ala Gly Gly Tyr Tyr Val Phe Gly Thr Gly Thr Asp Val
        115                 120                 125

Thr Val Leu Gly Ser Thr Ser Gly Ser Gly Lys Pro Gly Ser Gly Glu
    130                 135                 140

Gly Ser Thr Lys Gly Glu Val Gln Leu Leu Glu Ser Gly Gly Gly Leu
145                 150                 155                 160

Val Gln Pro Gly Gly Ser Leu Arg Leu Ser Cys Ala Ala Ser Gly Phe
                165                 170                 175

Thr Phe Ser Thr Tyr Gln Met Ser Trp Val Arg Gln Ala Pro Gly Lys
            180                 185                 190

Gly Leu Glu Trp Val Ser Gly Ile Val Ser Ser Gly Gly Ser Thr Ala
        195                 200                 205

Tyr Ala Asp Ser Val Lys Gly Arg Phe Thr Ile Ser Arg Asp Asn Ser
    210                 215                 220

Lys Asn Thr Leu Tyr Leu Gln Met Asn Ser Leu Arg Ala Glu Asp Thr
225                 230                 235                 240

Ala Val Tyr Tyr Cys Ala Gly Glu Leu Leu Pro Tyr Tyr Gly Met Asp
                245                 250                 255

Val Trp Gly Gln Gly Thr Thr Val Thr Val Ser Ser Glu Ser Lys Tyr
            260                 265                 270

Gly Pro Pro Cys Pro Pro Cys Pro Ala Pro Glu Phe Leu Gly Gly Pro

275                 280                 285

Ser Val Phe Leu Phe Pro Pro Lys Pro Lys Asp Thr Leu Met Ile Ser
    290                 295                 300

Arg Thr Pro Glu Val Thr Cys Val Val Val Asp Val Ser Gln Glu Asp
305                 310                 315                 320

Pro Glu Val Gln Phe Asn Trp Tyr Val Asp Gly Val Glu Val His Asn
                325                 330                 335

Ala Lys Thr Lys Pro Arg Glu Glu Gln Phe Asn Ser Thr Tyr Arg Val
            340                 345                 350

Val Ser Val Leu Thr Val Leu His Gln Asp Trp Leu Asn Gly Lys Glu
        355                 360                 365

Tyr Lys Cys Lys Val Ser Asn Lys Gly Leu Pro Ser Ser Ile Glu Lys
    370                 375                 380

Thr Ile Ser Lys Ala Lys Gly Gln Pro Arg Glu Pro Gln Val Tyr Thr
385                 390                 395                 400

Leu Pro Pro Ser Gln Glu Glu Met Thr Lys Asn Gln Val Ser Leu Thr
                405                 410                 415

Cys Leu Val Lys Gly Phe Tyr Pro Ser Asp Ile Ala Val Glu Trp Glu
            420                 425                 430

Ser Asn Gly Gln Pro Glu Asn Asn Tyr Lys Thr Thr Pro Pro Val Leu
        435                 440                 445

Asp Ser Asp Gly Ser Phe Phe Leu Tyr Ser Arg Leu Thr Val Asp Lys
    450                 455                 460

Ser Arg Trp Gln Glu Gly Asn Val Phe Ser Cys Ser Val Met His Glu
465                 470                 475                 480

Ala Leu His Asn His Tyr Thr Gln Lys Ser Leu Ser Leu Ser Leu Gly
                485                 490                 495

Lys Met Phe Trp Val Leu Val Val Val Gly Gly Val Leu Ala Cys Tyr
            500                 505                 510

Ser Leu Leu Val Thr Val Ala Phe Ile Ile Phe Trp Val Lys Arg Gly
        515                 520                 525

Arg Lys Lys Leu Leu Tyr Ile Phe Lys Gln Pro Phe Met Arg Pro Val
    530                 535                 540

Gln Thr Thr Gln Glu Glu Asp Gly Cys Ser Cys Arg Phe Pro Glu Glu
545                 550                 555                 560

Glu Glu Gly Gly Cys Glu Leu Arg Val Lys Phe Ser Arg Ser Ala Asp
                565                 570                 575

Ala Pro Ala Tyr Gln Gln Gly Gln Asn Gln Leu Tyr Asn Glu Leu Asn
            580                 585                 590

Leu Gly Arg Arg Glu Glu Tyr Asp Val Leu Asp Lys Arg Arg Gly Arg
        595                 600                 605

Asp Pro Glu Met Gly Gly Lys Pro Arg Arg Lys Asn Pro Gln Glu Gly
    610                 615                 620

Leu Tyr Asn Glu Leu Gln Lys Asp Lys Met Ala Glu Ala Tyr Ser Glu
625                 630                 635                 640

Ile Gly Met Lys Gly Glu Arg Arg Arg Gly Lys Gly His Asp Gly Leu
                645                 650                 655

Tyr Gln Gly Leu Ser Thr Ala Thr Lys Asp Thr Tyr Asp Ala Leu His
            660                 665                 670

Met Gln Ala Leu Pro Pro Arg
        675

<210>  23
<211>  1488
<212>  DNA
<213>  Artificial sequence

<220>
<223>  Synthetic CAR oligonucleotide

<400>  23
atgctgctgc tggtgaccag cctgctgctg tgtgagctgc cccaccccgc ctttctgctg      60

atcccccagt ccgagctgac ccagcccaga tccgtgtccg gctccccagg ccagtccgtg     120

accatctcct gcaccggcac cgagagggac gtgggcggct acaactacgt gtcctggtat     180

cagcagcacc ccggcaaggc ccccaagctg atcatccacg acgtgatcga gaggtcctcc     240

ggcgtgcccg acaggttctc cggctccaag tccggcaaca ccgcctccct gaccatcagc     300

ggactgcagg ccgaggacga ggccgactac tactgctggt ccttcgctgg cggctactac     360

gtgttcggca ccggaaccga cgtgaccgtg ctgggcagca cctccggcag cggcaagcct     420

ggcagcggcg agggcagcac caagggcgaa gtgcagctgc tggaatccgg cggaggactg 480

gtgcagccag gcggctccct gagactgtct tgcgccgcct ccggcttcac cttctccacc 540

taccagatgt cctgggtgcg ccaggcccca ggcaagggcc tggaatgggt gtccggaatc 600

gtgtcctctg gcggctccac cgcctacgcc gactccgtga agggcaggtt caccatctcc 660

agagacaact ccaagaacac cctgtacctg cagatgaact ccctgagggc cgaggacacc 720

gccgtgtact actgtgccgg cgagctgctg ccctactacg gcatggacgt gtggggccag 780

ggcaccaccg tgaccgtgtc ctccaagccc accaccaccc ctgcccccag acctccaacc 840

ccagccccta caatcgccag ccagcccctg agcctgaggc ccgaagcctg tagacctgcc 900

gctggcggag ccgtgcacac cagaggcctg gatttcgcct gcgacatcta catctgggcc 960

cctctggccg gcacctgtgg cgtgctgctg ctgagcctgg tcatcaccct gtactgcaac 1020

caccggaaca ggagcaagcg gagcagaggc ggccacagcg actacatgaa catgaccccc 1080

cggaggcctg gccccacccg gaagcactac cagccctacg cccctcccag ggacttcgcc 1140

gcctaccgga gccgggtgaa gttcagccgg agcgccgacg cccctgccta ccagcagggc 1200

cagaaccagc tgtacaacga gctgaacctg ggccggaggg aggagtacga cgtgctggac 1260

aagcggagag gccgggaccc tgagatgggc ggcaagcccc ggagaaagaa ccctcaggag 1320

ggcctgtata acgaactgca gaaagacaag atggccgagg cctacagcga gatcggcatg 1380

aagggcgagc ggcggagggg caagggccac gacggcctgt accagggcct gagcaccgcc 1440

accaaggata cctacgacgc cctgcacatg caggccctgc cccccaga 1488

<210> 24
<211> 496
<212> PRT
<213> Artificial sequence

<220>
<223> Synthetic CAR polypeptide

<400> 24

Met Leu Leu Leu Val Thr Ser Leu Leu Leu Cys Glu Leu Pro His Pro
1               5                   10                  15

Ala Phe Leu Leu Ile Pro Gln Ser Glu Leu Thr Gln Pro Arg Ser Val
            20                  25                  30

Ser Gly Ser Pro Gly Gln Ser Val Thr Ile Ser Cys Thr Gly Thr Glu
        35                  40                  45

Arg Asp Val Gly Gly Tyr Asn Tyr Val Ser Trp Tyr Gln Gln His Pro
    50                  55                  60

Gly Lys Ala Pro Lys Leu Ile Ile His Asp Val Ile Glu Arg Ser Ser
65                  70                  75                  80

Gly Val Pro Asp Arg Phe Ser Gly Ser Lys Ser Gly Asn Thr Ala Ser
                85                  90                  95

Leu Thr Ile Ser Gly Leu Gln Ala Glu Asp Glu Ala Asp Tyr Tyr Cys
            100                 105                 110

Trp Ser Phe Ala Gly Gly Tyr Tyr Val Phe Gly Thr Gly Thr Asp Val
        115                 120                 125

Thr Val Leu Gly Ser Thr Ser Gly Ser Gly Lys Pro Gly Ser Gly Glu
    130                 135                 140

Gly Ser Thr Lys Gly Glu Val Gln Leu Leu Glu Ser Gly Gly Gly Leu
145                 150                 155                 160

Val Gln Pro Gly Gly Ser Leu Arg Leu Ser Cys Ala Ala Ser Gly Phe
                165                 170                 175

Thr Phe Ser Thr Tyr Gln Met Ser Trp Val Arg Gln Ala Pro Gly Lys
            180                 185                 190

Gly Leu Glu Trp Val Ser Gly Ile Val Ser Ser Gly Gly Ser Thr Ala
        195                 200                 205

Tyr Ala Asp Ser Val Lys Gly Arg Phe Thr Ile Ser Arg Asp Asn Ser
    210                 215                 220

Lys Asn Thr Leu Tyr Leu Gln Met Asn Ser Leu Arg Ala Glu Asp Thr
225                 230                 235                 240

Ala Val Tyr Tyr Cys Ala Gly Glu Leu Leu Pro Tyr Tyr Gly Met Asp
                245                 250                 255

Val Trp Gly Gln Gly Thr Thr Val Thr Val Ser Ser Lys Pro Thr Thr
            260                 265                 270

Thr Pro Ala Pro Arg Pro Pro Thr Pro Ala Pro Thr Ile Ala Ser Gln
        275                 280                 285

Pro Leu Ser Leu Arg Pro Glu Ala Cys Arg Pro Ala Ala Gly Gly Ala
    290                 295                 300

Val His Thr Arg Gly Leu Asp Phe Ala Cys Asp Ile Tyr Ile Trp Ala
305                 310                 315                 320

Pro Leu Ala Gly Thr Cys Gly Val Leu Leu Leu Ser Leu Val Ile Thr
                325                 330                 335

Leu Tyr Cys Asn His Arg Asn Arg Ser Lys Arg Ser Arg Gly Gly His
            340                 345                 350

Ser Asp Tyr Met Asn Met Thr Pro Arg Arg Pro Gly Pro Thr Arg Lys
        355                 360                 365

His Tyr Gln Pro Tyr Ala Pro Pro Arg Asp Phe Ala Ala Tyr Arg Ser
    370                 375                 380

Arg Val Lys Phe Ser Arg Ser Ala Asp Ala Pro Ala Tyr Gln Gln Gly
385                 390                 395                 400

Gln Asn Gln Leu Tyr Asn Glu Leu Asn Leu Gly Arg Arg Glu Glu Tyr
                405                 410                 415

Asp Val Leu Asp Lys Arg Arg Gly Arg Asp Pro Glu Met Gly Gly Lys
            420                 425                 430

Pro Arg Arg Lys Asn Pro Gln Glu Gly Leu Tyr Asn Glu Leu Gln Lys
        435                 440                 445

Asp Lys Met Ala Glu Ala Tyr Ser Glu Ile Gly Met Lys Gly Glu Arg
    450                 455                 460

Arg Arg Gly Lys Gly His Asp Gly Leu Tyr Gln Gly Leu Ser Thr Ala
465                 470                 475                 480

Thr Lys Asp Thr Tyr Asp Ala Leu His Met Gln Ala Leu Pro Pro Arg
                485                 490                 495

<210>  25
<211>  1491
<212>  DNA
<213>  Artificial sequence

<220>
<223>  Synthetic CAR oligonucleotide

<400>  25
atgctgctgc tggtgaccag cctgctgctg tgtgagctgc cccaccccgc ctttctgctg      60

atcccccagt ccgagctgac ccagcccaga tccgtgtccg gctccccagg ccagtccgtg     120

accatctcct gcaccggcac cgagagggac gtgggcggct acaactacgt gtcctggtat     180

cagcagcacc ccggcaaggc ccccaagctg atcatccacg acgtgatcga gaggtcctcc     240

ggcgtgcccg acaggttctc cggctccaag tccggcaaca ccgcctccct gaccatcagc     300

ggactgcagg ccgaggacga ggccgactac tactgctggt ccttcgctgg cggctactac 360

gtgttcggca ccggaaccga cgtgaccgtg ctgggcagca cctccggcag cggcaagcct 420

ggcagcggcg agggcagcac caagggcgaa gtgcagctgc tggaatccgg cggaggactg 480

gtgcagccag gcggctccct gagactgtct tgcgccgcct ccggcttcac cttctccacc 540

taccagatgt cctgggtgcg ccaggcccca ggcaagggcc tggaatgggt gtccggaatc 600

gtgtcctctg gcggctccac cgcctacgcc gactccgtga agggcaggtt caccatctcc 660

agagacaact ccaagaacac cctgtacctg cagatgaact ccctgagggc cgaggacacc 720

gccgtgtact actgtgccgg cgagctgctg ccctactacg gcatggacgt gtggggccag 780

ggcaccaccg tgaccgtgtc ctccaagccc accaccaccc ctgcccccag acctccaacc 840

ccagccccta caatcgccag ccagcccctg agcctgaggc ccgaagcctg tagacctgcc 900

gctggcggag ccgtgcacac cagaggcctg gatttcgcct gcgacatcta catctgggcc 960

cctctggccg gcacctgtgg cgtgctgctg ctgagcctgg tcatcaccct gtactgcaac 1020

caccggaaca agagaggccg gaagaaactg ctgtacatct tcaagcagcc cttcatgcgg 1080

cccgtgcaga ccacccagga agaggacggc tgcagctgcc ggttccccga ggaagaggaa 1140

ggcggctgcg aactgcgggt gaagttcagc cggagcgccg acgcccctgc ctaccagcag 1200

ggccagaacc agctgtacaa cgagctgaac ctgggccgga gggaggagta cgacgtgctg 1260

gacaagcgga gaggccggga ccctgagatg ggcggcaagc cccggagaaa gaaccctcag 1320

gagggcctgt ataacgaact gcagaaagac aagatggccg aggcctacag cgagatcggc 1380

atgaagggcg agcggcggag gggcaagggc cacgacggcc tgtaccaggg cctgagcacc 1440

gccaccaagg atacctacga cgccctgcac atgcaggccc tgccccccag a 1491

<210> 26
<211> 497
<212> PRT
<213> Artificial sequence

<220>
<223> Synthetic CAR polypeptide

<400> 26

Met Leu Leu Leu Val Thr Ser Leu Leu Leu Cys Glu Leu Pro His Pro
1               5                   10                  15

Ala Phe Leu Leu Ile Pro Gln Ser Glu Leu Thr Gln Pro Arg Ser Val
            20                  25                  30

Ser Gly Ser Pro Gly Gln Ser Val Thr Ile Ser Cys Thr Gly Thr Glu
        35                  40                  45

Arg Asp Val Gly Gly Tyr Asn Tyr Val Ser Trp Tyr Gln Gln His Pro
    50                  55                  60

Gly Lys Ala Pro Lys Leu Ile Ile His Asp Val Ile Glu Arg Ser Ser
65                  70                  75                  80

Gly Val Pro Asp Arg Phe Ser Gly Ser Lys Ser Gly Asn Thr Ala Ser
                85                  90                  95

Leu Thr Ile Ser Gly Leu Gln Ala Glu Asp Glu Ala Asp Tyr Tyr Cys
            100                 105                 110

Trp Ser Phe Ala Gly Gly Tyr Tyr Val Phe Gly Thr Gly Thr Asp Val
        115                 120                 125

Thr Val Leu Gly Ser Thr Ser Gly Ser Gly Lys Pro Gly Ser Gly Glu
    130                 135                 140

Gly Ser Thr Lys Gly Glu Val Gln Leu Leu Glu Ser Gly Gly Gly Leu
145                 150                 155                 160

Val Gln Pro Gly Gly Ser Leu Arg Leu Ser Cys Ala Ala Ser Gly Phe
                165                 170                 175

Thr Phe Ser Thr Tyr Gln Met Ser Trp Val Arg Gln Ala Pro Gly Lys
            180                 185                 190

Gly Leu Glu Trp Val Ser Gly Ile Val Ser Ser Gly Gly Ser Thr Ala
        195                 200                 205

Tyr Ala Asp Ser Val Lys Gly Arg Phe Thr Ile Ser Arg Asp Asn Ser
    210                 215                 220

Lys Asn Thr Leu Tyr Leu Gln Met Asn Ser Leu Arg Ala Glu Asp Thr
225                 230                 235                 240

Ala Val Tyr Tyr Cys Ala Gly Glu Leu Leu Pro Tyr Tyr Gly Met Asp
                245                 250                 255

Val Trp Gly Gln Gly Thr Thr Val Thr Val Ser Ser Lys Pro Thr Thr
            260                 265                 270

Thr Pro Ala Pro Arg Pro Pro Thr Pro Ala Pro Thr Ile Ala Ser Gln
        275                 280                 285

Pro Leu Ser Leu Arg Pro Glu Ala Cys Arg Pro Ala Ala Gly Gly Ala
    290                 295                 300

Val His Thr Arg Gly Leu Asp Phe Ala Cys Asp Ile Tyr Ile Trp Ala
305                 310                 315                 320

Pro Leu Ala Gly Thr Cys Gly Val Leu Leu Leu Ser Leu Val Ile Thr
                325                 330                 335

Leu Tyr Cys Asn His Arg Asn Lys Arg Gly Arg Lys Lys Leu Leu Tyr
            340                 345                 350

Ile Phe Lys Gln Pro Phe Met Arg Pro Val Gln Thr Thr Gln Glu Glu
        355                 360                 365

Asp Gly Cys Ser Cys Arg Phe Pro Glu Glu Glu Glu Gly Gly Cys Glu
    370                 375                 380

Leu Arg Val Lys Phe Ser Arg Ser Ala Asp Ala Pro Ala Tyr Gln Gln
385                 390                 395                 400

Gly Gln Asn Gln Leu Tyr Asn Glu Leu Asn Leu Gly Arg Arg Glu Glu
                405                 410                 415

Tyr Asp Val Leu Asp Lys Arg Arg Gly Arg Asp Pro Glu Met Gly Gly
            420                 425                 430

Lys Pro Arg Arg Lys Asn Pro Gln Glu Gly Leu Tyr Asn Glu Leu Gln
        435                 440                 445

Lys Asp Lys Met Ala Glu Ala Tyr Ser Glu Ile Gly Met Lys Gly Glu
    450                 455                 460

Arg Arg Arg Gly Lys Gly His Asp Gly Leu Tyr Gln Gly Leu Ser Thr
465                 470                 475                 480

Ala Thr Lys Asp Thr Tyr Asp Ala Leu His Met Gln Ala Leu Pro Pro
                485                 490                 495

Arg

<210>  27
<211>  1836
<212>  DNA
<213>  Artificial sequence

<220>
<223>  Synthetic CAR oligonucleotide

<400>  27

atggaaaccc tgctgggcct gctgatcctg tggctgcagc tccagtgggt gtcctccaag 60

caggaagtga cccagatccc agccgccctg tccgtgccag agggcgagaa cctggtgctg 120

aactgctcct tcaccgactc cgccatctac aacctgcagt ggttcaggca ggaccccggc 180

aagggcctga cctccctgct gctgatccag tcctcccaga gagagcagac ctccggcagg 240

ctgaacgcct ccctggacaa gtcctccggc agatccaccc tgtatatcgc cgcctcccag 300

cccggcgaca gcgccacata tctgtgtgcc gtgcgccccc tgtacggcgg ctcctacatc 360

ccaaccttcg gcaggggcac ctccctgatc gtgcacccct acatccagaa ccccgacccc 420

gccgtgtacc agctgaggga ctccaagtcc agcgacaagt ccgtgtgcct gttcaccgac 480

ttcgactccc agaccaacgt gtcccagtcc aaggactccg acgtgtacat caccgacaag 540

accgtgctgg acatgagatc catggacttc aagtccaact ccgccgtggc ctggtccaac 600

aagtccgact tcgcctgcgc caacgccttc aacaactcca tcatccccga ggacacattc 660

ttcccatccc ccgagtccag ctgcgacgtg aagctggtgg aaaagtcctt cgaaaccgac 720

accaacctga acttccagaa cctgtccgtg atcggcttca ggattctgct gctgaaggtg 780

gccggcttca acctgctgat gaccctgagg ctgtggtcct ccagagccaa gagatccggc 840

tccggcgcca ccaacttcag cctgctgaaa caggccggcg acgtggaaga gaacccaggc 900

cccatgtcca tcggcctgct gtgctgcgcc gctctgtctc tgctgtgggc cggaccagtg 960

aacgctggcg tgacccagac ccccaagttc caggtgctga aaaccggcca gtccatgacc 1020

ctgcagtgcg cccaggacat gaaccacgag tacatgtcct ggtacagaca ggaccctgga 1080

atgggactga ggctgatcca ctactccgtg gccatcggca tcaccgacca gggcgaggtg 1140

cccaacggct acaacgtgtc caggtccacc accgaggact tcccactgag gctgctgtct 1200

gccgccccat cccagacctc cgtgtacttc tgcgcctcct cctacgtggg caacaccggc 1260

gagctgttct tcggcgaggg ctccaggctg acagtgctgg aagatctgaa gaacgtgttc 1320

cccccagagg tggccgtgtt cgagccctcc gaggccgaga tctcccacac ccagaaagcc 1380

accctggtct gcctggccac cggcttctac cctgaccacg tggaactgtc ttggtgggtg 1440

aacggcaaag aggtgcactc cggcgtctcc accgaccccc agcccctgaa agagcagccc 1500

gccctgaacg actccagata ctgcctgtcc tccagactga gggtgtccgc caccttctgg 1560

cagaacccca gaaaccactt caggtgccag gtgcagttct acggcctgtc cgagaacgac 1620

gagtggaccc aggacagggc caagcccgtg acacagatcg tgtctgccga ggcctggggc 1680

agggccgact gcggattcac ctccgagtcc taccagcagg gcgtgctgag cgccaccatc 1740

ctgtacgaga tcctgctggg caaggccacc ctgtacgccg tgctggtgtc cgctctggtg 1800

ctgatggcca tggtgaaaag aaaggactcc aggggc 1836

<210>  28
<211>  612
<212>  PRT
<213>  Artificial sequence

<220>
<223>  Synthetic CAR polypeptide

<400>  28

Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Leu Tyr Gly Gly Ser Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro

210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255

Leu Leu Lys Val Ala Gly Phe Asn Leu Leu Met Thr Leu Arg Leu Trp
            260                 265                 270

Ser Ser Arg Ala Lys Arg Ser Gly Ser Gly Ala Thr Asn Phe Ser Leu
        275                 280                 285

Leu Lys Gln Ala Gly Asp Val Glu Glu Asn Pro Gly Pro Met Ser Ile
    290                 295                 300

Gly Leu Leu Cys Cys Ala Ala Leu Ser Leu Leu Trp Ala Gly Pro Val
305                 310                 315                 320

Asn Ala Gly Val Thr Gln Thr Pro Lys Phe Gln Val Leu Lys Thr Gly
                325                 330                 335

Gln Ser Met Thr Leu Gln Cys Ala Gln Asp Met Asn His Glu Tyr Met
            340                 345                 350

Ser Trp Tyr Arg Gln Asp Pro Gly Met Gly Leu Arg Leu Ile His Tyr
        355                 360                 365

Ser Val Ala Ile Gly Ile Thr Asp Gln Gly Glu Val Pro Asn Gly Tyr
    370                 375                 380

Asn Val Ser Arg Ser Thr Thr Glu Asp Phe Pro Leu Arg Leu Leu Ser
385                 390                 395                 400

Ala Ala Pro Ser Gln Thr Ser Val Tyr Phe Cys Ala Ser Ser Tyr Val
                405                 410                 415

Gly Asn Thr Gly Glu Leu Phe Phe Gly Glu Gly Ser Arg Leu Thr Val
            420                 425                 430

Leu Glu Asp Leu Lys Asn Val Phe Pro Pro Glu Val Ala Val Phe Glu
        435                 440                 445

Pro Ser Glu Ala Glu Ile Ser His Thr Gln Lys Ala Thr Leu Val Cys
    450                 455                 460

Leu Ala Thr Gly Phe Tyr Pro Asp His Val Glu Leu Ser Trp Trp Val
465                 470                 475                 480

Asn Gly Lys Glu Val His Ser Gly Val Ser Thr Asp Pro Gln Pro Leu
                485                 490                 495

Lys Glu Gln Pro Ala Leu Asn Asp Ser Arg Tyr Cys Leu Ser Ser Arg
            500                 505                 510

Leu Arg Val Ser Ala Thr Phe Trp Gln Asn Pro Arg Asn His Phe Arg
        515                 520                 525

Cys Gln Val Gln Phe Tyr Gly Leu Ser Glu Asn Asp Glu Trp Thr Gln
    530                 535                 540

Asp Arg Ala Lys Pro Val Thr Gln Ile Val Ser Ala Glu Ala Trp Gly
545                 550                 555                 560

Arg Ala Asp Cys Gly Phe Thr Ser Glu Ser Tyr Gln Gln Gly Val Leu
                565                 570                 575

Ser Ala Thr Ile Leu Tyr Glu Ile Leu Leu Gly Lys Ala Thr Leu Tyr
            580                 585                 590

Ala Val Leu Val Ser Ala Leu Val Leu Met Ala Met Val Lys Arg Lys
        595                 600                 605

Asp Ser Arg Gly
    610

## Description

This application claims the benefit of United States Provisional Patent Application No. 62/075,642, filed November 5, 2014; United States Provisional Patent Application No. 62/075,561, filed November 5, 2014; United States Provisional Patent Application No. 62/075,667, filed November 5, 2014; and United States Provisional Patent Application No. 62/169,979, filed June 2, 2015, the entirety of each is incorporated herein by reference.

### INCORPORATION OF SEQUENCE LISTING

The sequence listing that is contained in the file named "UTFCP1251WO_ST25.txt", which is 48 KB (as measured in Microsoft Windows®) and was created on November 5, 2015, is filed herewith by electronic submission and is incorporated by reference herein.

### BACKGROUND

### 1. Field of the Invention

Described herein are chimeric antigen receptors (CAR), CAR-expressing immune effector cells and methods of making and using CARs and CAR T cells. Also provided are immune effector cell compositions and antigen presenting cells having enhanced therapeutic properties.

### 2. Description of Related Art

The potency of clinical-grade T cells can be improved by combining gene therapy with immunotherapy to engineer a biologic product with the potential for superior (i) recognition of tumor-associated antigens (TAAs), (ii) persistence after infusion, (iii) potential for migration to tumor sites, and (iv) ability to recycle effector functions within the tumor microenvironment. Such genetic engineering of T cells can be used to redirect the specificity of the cells and to provide therapeutic compositions having antigen-targeted cytotoxic activity. These engineered T-cell composition have been shown to be effective for therapeutic intervention in, for example, in cancer patients (Jena *et al*., 2010; Till *et al*., 2008; Porter *et al*., 2011; Brentjens *et al*., 2011; Cooper and Bollard, 2012; Kalos *et al*., 2011; Kochenderfer *et al*., 2010; Kochenderfer *et al*., 2012; Brentjens *et al*., 2013). There remains a need however, for improved T-cell and CAR constructs that allow for control of *in vivo* persistence.

### SUMMARY OF THE INVENTION

In a first embodiment there is provided chimeric antigen receptor (CAR) polypeptide comprising an antigen binding domain; a hinge domain; a transmembrane domain and one or more intracellular signaling domain(s). In some aspects, the hinge domain comprises a sequence that binds to an Fc receptor, such as a FcγR2a or FcγR1a. For example, the hinge sequence may comprise an Fc domain from a human immunoglobulin (*e.g*., IgG1, IgG2, IgG3, IgG4, IgA1, IgA2, IgM, IgD or IgE) that binds to an Fc receptor. In certain aspects, the hinge domain (and/or the CAR) does not comprise a wild type human IgG4 CH2 and CH3 sequence.

In further aspects, a hinge domain of a CAR of the embodiments may comprise a sequence that exhibits reduced or essentially no binding to a Fc receptor (such as FcγR2a and/or FcγR1a Fc receptor). In some aspects, the hinge domain comprises (i) a human IgG4-Fc sequence having at least 8 consecutive amino acids identical to SEQ ID NO:1 (the mutant IgG4-Fc sequence), said sequence having reduced Fc-receptor binding relative to full-length wild type IgG4-Fc; or (ii) a human CD8a extracellular sequence.

In some aspects, the hinge domain may comprise a human IgG4-Fc sequence having at least 8 consecutive amino acids identical to SEQ ID NO:1 (the mutant IgG4-Fc sequence), said sequence having reduced Fc-receptor binding relative to full-length wild type IgG4-Fc. In some aspects, the hinge domain may comprise a sequence at least about 85%, 86%, 87%, 88%, 89%, 90%, 91%, 92%, 93%, 94%, 95%, 96%, 97%, 98% or 99% identical to SEQ ID NO: 3 (the 12-aa spacer). In one aspect, the hinge domain may comprise a sequence identical to SEQ ID NO: 3 (the 12-aa spacer). In still further aspects, the hinge domain may comprise a sequence having no more than 1, 2, or 3 amino acid deletions or substitutions relative to SEQ ID NO: 3.

In certain aspects, the hinge domain may comprise an IgG4-Fc sequence, said sequence comprising at least one mutation relative to wild type IgG4-Fc that reduces Fc-receptor binding. In some aspects, the IgG4-Fc sequence may be at least about 85%, 86%, 87%, 88%, 89%, 90%, 91%, 92%, 93%, 94%, 95%, 96%, 97%, 98% or 99% identical to SEQ ID NO: 1. In some aspects, the IgG4-Fc sequence may comprise a L235E or N297Q mutation relative to the wild type sequence. In certain aspects, the IgG4-Fc sequence may comprise a L235E and N297Q mutation relative to the wild type sequence. In one aspect, the IgG4-Fc sequence may be identical to SEQ ID NO: 1 or may comprise no more than 1, 2, or 3 amino acid deletions or substitutions relative to SEQ ID NO: 1. In another aspect, the IgG4-Fc sequence I identical to SEQ ID NO: 1.

In some aspects, the hinge domain may comprise a CD8a extracellular sequence. For example, the hinge domain may comprise a sequence at least about 85%, 86%, 87%, 88%, 89%, 90%, 91%, 92%, 93%, 94%, 95%, 96%, 97%, 98% or 99% identical to the extracellular portion of SEQ ID NO: 2. In a further aspect, the CAR polypeptide may comprise a sequence identical to the extracellular portion of SEQ ID NO: 2 or may comprise no more than 1, 2, or 3 amino acid deletions or substitutions relative to the extracellular portion of SEQ ID NO: 2. In still a further aspects, the hinge domain of a CAR polypeptide comprises a sequence at least about 85%, 86%, 87%, 88%, 89%, 90%, 91%, 92%, 93%, 94%, 95%, 96%, 97%, 98% or 99% identical to SEQ ID NO: 20. In a further aspect, the CAR polypeptide may comprise a sequence identical to SEQ ID NO: 20 or may comprise no more than 1, 2, or 3 amino acid deletions or substitutions relative to SEQ ID NO: 20.

In some aspects, the transmembrane domain may comprise a CD8a transmembrane domain. In certain aspects, the CAR polypeptide may comprise a sequence at least about 85%, 86%, 87%, 88%, 89%, 90%, 91%, 92%, 93%, 94%, 95%, 96%, 97%, 98% or 99% identical to the transmembrane portion of SEQ ID NO: 2. In one aspect, the CAR polypeptide may comprise a sequence identical to the transmembrane portion of SEQ ID NO: 2 or may comprise no more than 1, 2, or 3 amino acid deletions or substitutions relative to the transmembrane portion of SEQ ID NO: 2. In certain aspects, the transmembrane domain may comprise a transmembrane domain of CD28, CD8a or CD137. In still further aspects, the transmembrane domain may comprise a CD8a transmembrane domain 85%, 86%, 87%, 88%, 89%, 90%, 91%, 92%, 93%, 94%, 95%, 96%, 97%, 98% or 99% identical to SEQ ID NO: 19. In yet a further aspects, the CAR polypeptide may comprise a transmembrane sequence identical to SEQ ID NO: 19 or may comprise no more than 1, 2, or 3 amino acid deletions or substitutions relative to SEQ ID NO: 19.

In some aspects, the intracellular cell signaling domain may comprise a domain from CD3ζ. For example, a CAR polypeptide of the embodiments may comprise a CD3ζ sequence that is 85%, 86%, 87%, 88%, 89%, 90%, 91%, 92%, 93%, 94%, 95%, 96%, 97%, 98% or 99% identical to SEQ ID NO: 13. In yet a further aspects, the CAR polypeptide may comprise a comprise a CD3ζ sequence identical to SEQ ID NO: 13 or may comprise no more than 1, 2, or 3 amino acid deletions or substitutions relative to SEQ ID NO: 13. In still further aspects, the intracellular cell signaling domain may comprise an intracellular domain from CD28 or CD137 (4-1BB). For example, a CAR may comprise an intracellular cell signaling domain comprising a sequence that is 85%, 86%, 87%, 88%, 89%, 90%, 91%, 92%, 93%, 94%, 95%, 96%, 97%, 98% or 99% identical to SEQ ID NO: 15 or SEQ ID NO: 17. In yet a further aspects, the CAR polypeptide may comprise an intracellular cell signaling domain comprising a sequence that is identical to SEQ ID NO: 15 or SEQ ID NO: 17 or may comprise no more than 1, 2, or 3 amino acid deletions or substitutions relative to SEQ ID NO: 15 or 17.

In various aspects, the antigen binding domain of a CAR of the embodiments may comprise a heavy chain variable domain (VH) and a light chain variable domain (VL) of a scFv or an antibody. In further aspects, the antigen binding domain may comprise a first scFv domain and a second scFv domain, wherein one of the first or second scFv domains binds a first antigen and the other domain binds a second antigen. In some aspects, the VL domain may be positioned N-terminal relative to the VH domain. In certain aspects, the VH domain may be positioned N-terminal relative to the VL domain. In certain aspects, the CAR polypeptide may further comprise a linker sequence between the light chain variable domain and heavy chain variable domain and/or the first and the second scFv domains. In some aspects, the linker sequence may be a Whitlow linker (SEQ ID NO: 7).

In various aspects, the antigen binding domain may bind to an infectious disease antigen or a cancer-cell antigen. Examples of cancer cell antigens include CD19, CD20, ROR1, CD22carcinoembryonic antigen, alphafetoprotein, CA-125, 5T4, MUC-1, epithelial tumor antigen, prostate-specific antigen, melanoma-associated antigen, mutated p53, mutated ras, HER2/Neu, folate binding protein, HIV-1 envelope glycoprotein gp120, HIV-1 envelope glycoprotein gp41, GD2, CD123, CD33, CD138, CD23, CD30 , CD56, c-Met, mesothelin, GD3, HERV-K, IL-11Ralpha, kappa chain, lambda chain, CSPG4, ERBB2, EGFRvIII, VEGFR2, HER2-HER3 in combination, HER1-HER2 in combination or HER1-HER3 in combination. In some aspects, the cancer cell antigen may be CD19 and the CAR may be a CD19-targeted CAR.

In a further embodiment there are provided nucleic acid molecules encoding a CAR polypeptide in accordance with the embodiments. In some aspects, the sequence encoding the CAR is flanked by transposon repeats or viral LTRs.

In yet a further embodiment, there is provided an isolated immune effector cell (*e.g*., a T-cell, NK cell or NK T-cell) comprising a CAR polypeptide or nucleic acid in accordance with the embodiments. In some aspects the cell is a T-cell or a T-cell progenitor. In further aspects, the cell is a human cell. In certain aspects, the cell composition may comprise as low as a hundred or 1,000 cells. However, in some aspects, the cell composition comprises at least about 10⁴, 10⁵, 10⁶, 10⁷, 10⁸, 10⁹, 10¹⁰, 10¹¹ or more CAR-expressing immune effector cells. In some aspects, the cells may have essentially identical genetic material. In further aspects, the cells may be human cells derived from a single subject, who may be a donor or a patient. A further embodiment provides a pharmaceutical composition comprising a population of CAR-expressing cells in accordance with the embodiments in a pharmaceutically acceptable carrier.

In still a further embodiment, there is provided a method of treating a subject (*e.g*., a subject having a cancer or an infectious disease) comprising administering an effective amount of immune effector cells that expresses a CAR polypeptide in accordance with the embodiments. In certain aspects, the subject may have cancer. In certain aspects, the cancer may be a hematological or solid cancer, such as T-cell ALL, B-ALL, CML, colon cancer, ovarian cancer, neuroblastoma, brain tumor(s), or pancreatic cancer. In some aspects, the patient may have undergone a previous anti-cancer therapy. In one aspect, the patient may be in remission. In yet another aspect, the patient may be free of symptoms of the cancer but comprise detectable cancer cells. In another aspect, the subject may have a viral infection (*e.g*., cytomegalovirus (CMV), Epstein-Barr virus (EBV), or human immunodeficiency virus (HIV)). In yet another aspect, the subject may have a bacterial infection. In one aspect, the disease may be sepsis.

In certain aspects, a method of treating a subject comprises administering immune effector cells (*e.g*., T-cells) expressing a CAR that comprises a hinge domain having polypeptide sequence that binds to a human Fc receptor. In some aspects, such immune effector cells are administered locally, *e.g*., to a site of diseased tissue. In certain aspects, the site of local delivery has a reduced number of (or essentially no) cells expressing Fc receptors. For example, the site of administration can be in the central nervous system (e.g., the brain), the eye or testicle of a subject.

In some aspects, the CAR polypeptide may comprise an antigen binding domain that binds to a cancer cell antigen. In some aspects, the cancer may be a breast cancer, ovarian cancer, melanoma, non-small cell lung cancer, gastric cancer, colorectal cancer or pancreatic cancer. In further aspects, the cancer expresses the cancer cell antigen that is bound by the antigen binding domain of the CAR. In still further aspects, the subject has been identified as having a cancer that expresses the cancer cell antigen to which the CAR polypeptide binds. In still further aspects, the immune effector cells (*e.g*., T-cells) were previously isolated from the subject.

In some further aspects, the immune effector cells (*e.g*., T cells) may be allogeneic to the patient. In various aspects, allogeneic cells may or may not share HLA with the patient. In another aspect, the immune effector cells may be autologous to the patient.

In yet a further embodiment, there is provided a method comprising obtaining a sample of cells comprising immune effector cells or progenitors thereof (*e.g*., T-cells or T-cell progenitors), transfecting the cells with a DNA encoding a CAR polypeptide in accordance with the embodiments, to provide a population of transgenic CAR-expressing cells, and culturing the population of transgenic CAR cells *ex vivo* in a medium that selectively enhances proliferation of CAR-expressing immune effector cells (*e.g*., CAR-expressing T-cells). In certain aspects, the method further comprises transfecting the cells with a transposon-flanked CAR and a transposase effective to integrate the DNA encoding the CAR into the genome of the cells. In further aspects, a method comprises purifying or enriching T-cells in the sample prior to transfection. In certain cases the immune effector cells (*e.g*., T-cells or T-cell progenitors) are derived from induced pluripotent stem cells or embryonic stem cells. In further aspects, enriching cells in the sample comprises collecting a mononuclear cell fraction. The sample of cells may be from umbilical cord blood, a lymphoid organ or a peripheral blood sample from the subject in some cases. The sample of cells may be obtained by apheresis or venipuncture in some cases. In still further aspects, the sample of cells is a subpopulation of immune cells, such as a subpopulation of T-cells. In some aspects, transgenic CAR cells are inactivated for expression of an endogenous T-cell receptor and/or endogenous HLA. In further aspects, obtaining the sample of cells comprises obtaining the cells from a 3rd party.

In some aspects, the method of transfection (*e.g*., of a CAR construct) comprises electroporating DNA encoding a CAR into a cell (*e.g*., a T-cell). In further aspects, a CAR construct may be transduced into a cell using a viral vector. In some aspects, the transfection may not involve infecting or transducing the cells with virus. In still further aspects, the cells are additionally transfected with a nucleic acid encoding a membrane-bound Cγ cytokine. The membrane-bound Cγ cytokine may be a membrane bound IL-7, IL-15 or IL-21 in some instances. In a specific aspect, the membrane-bound Cγ cytokine is IL-15-IL-15Rα fusion protein (*e.g*., such as the mIL-15 polypeptide of SEQ ID NO: 4).

In still further aspects, the DNA encoding the CAR is a plasmid. In a further aspect, the CAR may be encoded by an RNA that is introduced into the cells. The transposase may be provided as a DNA expression vector, an mRNA, a polypeptide, or an expressible RNA in some aspects. In a specific aspect, the transposase is salmonid-type Tc1-like transposase (SB). In a further specific aspect, the transposase is the SB11 or SB100x transposase.

In yet a further aspect of the embodiments, culturing the transgenic CAR cells in accordance with the method detailed herein comprises culturing the transgenic CAR cells in the presence of dendritic cells or activating and propagating cells (AaPCs) that stimulate expansion of the CAR-expressing immune effector cells. In still further aspects, the AaPCs comprise a CAR-binding antibody or fragment thereof expressed on the surface of the AaPCs. The AaPCs may comprise additional molecules that activate or co-stimulate T-cells in some cases. The additional molecules may, in some cases, comprise membrane-bound Cγ cytokines. In yet still further aspects, the AaPCs are inactivated or irradiated, or have been tested for and confirmed to be free of infectious material. In still further aspects, culturing the transgenic CAR cells in the presence of AaPCs comprises culturing the transgenic CAR cells in a medium comprising soluble cytokines, such as IL-21 and/or IL-2. The cells may be cultured at a ratio of about 10:1 to about 1:10; about 3:1 to about 1:5; about 1:1 to about 1:3 (immune effector cells to AaPCs); or any range derivable therein. For example, the co-culture of T cells and aAPCs can be at a ratio of about 1:1, about 1:2 or about 1:3.

In a further aspect, the population of transgenic CAR cells is cultured for no more than 7, 14, 21, 28, 35 or 42 days. In some instances, the transgenic cells are not cultured *ex vivo* in the presence of AaPCs. In some specific instances, the method of the embodiment further comprises enriching the cell population for CAR-expressing immune effector cells (*e.g*., T-cells) after the transfection and/or culturing step. The enriching may comprise fluorescence-activated cell sorting (FACS) and sorting for CAR-expressing cells. In a further aspect, the sorting for CAR-expressing cells comprises use of a CAR-binding antibody. The enriching may also comprise depletion of CD56+ cells. In yet still a further aspect of the embodiment, the method further comprises cryopreserving a sample of the population of transgenic CAR cells.

In a further embodiment, there is provided a CAR-expressing immune effector cell (*e.g*., T-cell) population made by a method of any one of the embodiments.

In a further embodiment, a method of treating a disease in a patient is provided comprising producing a cell composition according to the methods of the present embodiments and administering an effective amount of said cell composition to a patient in need thereof. In some aspects, methods are provided for treating an individual with a medical condition comprising the step of providing an effective amount of cells from the population of cells described herein, including more than once in some aspects, such as at least 1, 2, 3, 4, 5, 6, 7, 8, 9, 10, 11, 12, 13, 14, or more days apart.

In yet a further embodiment there is provided an engineered antigen presenting cell (APC) comprising a first transgene encoding a target antigen and a second transgene encoding a human leukocyte antigen (HLA), said HLA being expressed on the surface of the APC in complex with an epitope of the target antigen. The engineered APC may or may not be immortalized. In some aspects, the engineered APC is a primary cell or a T-cell or T-cell progenitor. In some further aspects, the engineered APC is a T-cell expressing a αβTCR or a γδTCR. In certain specific aspects, the HLA expressed in the engineered APC is HLA-A2. In still further aspects, the engineered APC has been tested for and confirmed to be free of infectious material.

In some aspects, an engineered APC of the embodiments may further comprise at least a third transgene encoding co-stimulatory molecule. The co-stimulatory molecule may be a co-stimulatory cytokine that may be a membrane-bound Cγ cytokine. In certain aspects, the co-stimulatory cytokine is IL-15 that may or may not be membrane-bound.

In further aspects, an engineered APC of the embodiments is inactivated or irradiated. In some aspects, the engineered APC may comprise an edited gene to reduce or eliminate expression of an inhibitory gene. In specific aspects, the inhibitory gene may be PD-1, LIM-3, CTLA-4 or a TCR.

In still further aspects, the target antigen expressed in an engineered APC of the embodiments can be an infectious disease antigen or a tumor-associated antigen (TAA). Target antigens may be intracellular or cell surface antigens. For example, in some aspects, the target antigen is a TAA such as a TAA derived from a subcellular compartment of the tumor cell. The TAA may be membrane-bound, cytoplasmic, nuclear-localized, or even secreted by the tumor cells. Preferably, the TAA is differentially expressed compared to the corresponding normal tissue thereby allowing for a preferential recognition of tumor cells by immune effector cells. Tumor antigens can be loosely categorized as oncofetal (typically only expressed in fetal tissues and in cancerous somatic cells), oncoviral (encoded by tumorigenic transforming viruses), overexpressed/accumulated (expressed by both normal and neoplastic tissue, with the level of expression highly elevated in neoplasia), cancer-testis (expressed only by cancer cells and adult reproductive tissues such as testis and placenta), lineage-restricted (expressed largely by a single cancer histotype), mutated (only expressed by cancer as a result of genetic mutation or alteration in transcription), posttranslationally altered (tumor-associated alterations in glycosylation, etc.), or idiotypic (highly polymorphic genes where a tumor cell expresses a specific "clonotype", *e.g*., as in B cell, T cell lymphoma/leukemia resulting from clonal aberrancies), any such TAA may serve as a target antigen in accordance with the embodiments. Specific examples of target antigens include, without limitation, WT1, MUC1, LMP2, HPV E6 E7, EGFRvIII, HER-2/neu, Idiotype, MAGE A3, p53 nonmutant, NY-ESO-1, PSMA, GD2, CEA, MelanA/MART1, Ras mutant, gp100, p53 mutant, Proteinase3 (PR1), bcr-abl, Tyrosinase, Survivin, PSA, hTERT, Sarcoma translocation breakpoints, EphA2, PAP, ML-IAP, AFP, EpCAM, ERG (TMPRSS2 ETS fusion gene), NA17, PAX3, ALK, Androgen receptor, Cyclin B1, Polysialic acid, MCN, RhoC, TRP-2, GD3, Fucosyl GM1, Mesothelin, PSCA, MAGE A1, sLe(a), CYP1B1, PLAC1, GM3, BORIS, Tn, GloboH, ETV6-AML, NY-BR-1, RGS5, SART3, STn, Carbonic anhydrase IX, PAX5, OT-TES1, Sperm protein 17, LCK, HMWMAA, AKAP-4, SSX2, XAGE 1, B7H3, Legumain, Tie 2, Page4, VEGFR2, MAD-CT-1, FAP, PDGFR-β, MAD-CT-2, and Fos-related antigen 1. In other aspects, the target antigen is an infectious disease antigen.

In yet still further aspects, an engineered APC of the embodiments is a human cell. In some aspects, the first and/or second transgene of the engineered APC may be integrated into the genome of the cell. Further, in certain aspects, the first and/or second transgene may be flanked by a transposon repeat or a viral LTR. In further aspects, the first and/or second transgene is encoded by a recombinant mRNA.

In a further embodiment there is provided a cell culture comprising a population of engineered APCs in accordance with the embodiments and a population of immune effector cells (*e.g*., T-cells) with specificity to an epitope of the target antigen in complex with the HLA expressed in the APCs. In certain aspects, the immune effector cells of a culture have the same genetic background as the engineered APCs. In specific aspects, the immune effector cells express a T-cell receptor (TCR) that binds to an epitope of the target antigen in complex with the HLA expressed in the engineered APCs. In particular aspects, the TCR is an αβTCR. In certain aspects, the target antigen is NY-ESO-1. In some specific aspects, the TCR comprises a sequence at least 80%, 85%, 86%, 87%, 88%, 89%, 90%, 91%, 92%, 93%, 94%, 95%, 96%, 97%, 98% or 99% identical or identical to the anti-NY-ESO-1-αβTCR (SEQ ID NO: 28).

In further aspects, the immune effector cells of the cell culture (comprising an engineered APC of the embodiments) express a chimeric T-cell receptor (CAR) that binds to an epitope of the target antigen in complex with the HLA expressed in the engineered APCs. The CAR may comprise antibody variable domains that bind to an epitope of the target antigen in complex with the HLA expressed in the APCs. In some specific aspects, the target antigen is NY-ESO-1. In certain aspects, the CAR comprises a sequence at least 80%, 85%, 86%, 87%, 88%, 89%, 90%, 91%, 92%, 93%, 94%, 95%, 96%, 97%, 98% or 99% identical or identical to the NY-ESO-1R-CD28Tm-CD137-Z CAR (SEQ ID NO: 22); NY-ESO-1R-CD8a-CD28-Z CAR (SEQ ID NO: 24); or NY-ESO-1R-CD8a-CD137-Z CAR (SEQ ID NO: 26).

In some aspects, the cell culture of engineered APCs of the embodiments may comprise a medium that stimulates immune effector cell (*e.g*., T-cell) expansion. The medium may comprise IL-21 and/or IL-2. In specific aspects, the culture comprises a ratio of about 10:1 to about 1:10 (immune effector cells to engineered APCs).

In yet a further embodiment, there is provided a pharmaceutical composition comprising a population of engineered APCs in accordance with the embodiments described herein in a pharmaceutically acceptable carrier.

In still a further embodiment, there is provided a method of treating a subject having a disease comprising administering an effective amount of engineered APCs in accordance with the embodiments to the subject, wherein said APCs express a target antigen associated with the disease. In some aspects, the subject has a cancer that expresses the target antigen encoded by the engineered APCs. The cancer may be a myeloma or a synovial sarcoma. In certain aspects, the cancer is a NY-ESO-1 positive cancer and the target antigen is NY-ESO-1.

In some aspects of the embodiment, the engineered APCs were previously isolated from the subject. In further aspects, the method additionally comprises administering a population of antigen-specific immune effector cells to the subject, said immune effector cells having specificity to an epitope of the target antigen in complex with the HLA expressed in the engineered APCs. The immune effector cells may have been previously isolated from the subject. In further aspects, the immune effector cells have been engineered to express a T-cell receptor (TCR) that binds to an epitope of the target antigen in complex with the HLA expressed in the engineered APCs. In specific aspects, the TCR is an αβTCR. In some particular aspects, the target antigen is NY-ESO-1. In certain aspects, the TCR comprises a sequence at least 80%, 85%, 86%, 87%, 88%, 89%, 90%, 91%, 92%, 93%, 94%, 95%, 96%, 97%, 98% or 99% identical or identical to the anti-NY-ESO-1-αβTCR (SEQ ID NO:28).

In further aspects of the embodiment, the immune effector cells express a chimeric T-cell receptor (CAR) that binds to an epitope of the target antigen in complex with the HLA expressed in the engineered APCs. The CAR may comprise antibody variable domains that bind to an epitope of the target antigen in complex with the HLA expressed in the engineered APCs. In some specific aspects, the target antigen is NY-ESO-1. In further aspects, the CAR comprises a sequence at least 80%, 85%, 86%, 87%, 88%, 89%, 90%, 91%, 92%, 93%, 94%, 95%, 96%, 97%, 98% or 99% identical or identical to the NY-ESO-1R-CD28Tm-CD137-Z CAR (SEQ ID NO: 22); NY-ESO-1R-CD8a-CD28-Z CAR (SEQ ID NO: 24); or NY-ESO-1R-CD8a-CD137-Z CAR (SEQ ID NO: 26).

In a further embodiment, there is provided a recombinant CAR polypeptide comprising a sequence at least 80%, 85%, 86%, 87%, 88%, 89%, 90%, 91%, 92%, 93%, 94%, 95%, 96%, 97%, 98% or 99% identical or identical to the NY-ESO-1R-CD28Tm-CD137-Z CAR (SEQ ID NO: 22); NY-ESO-1R-CD8a-CD28-Z CAR (SEQ ID NO: 24); or NY-ESO-1R-CD8a-CD137-Z CAR (SEQ ID NO: 26). In still a further embodiment there is provided an immune effector cell (*e.g*., a T-cell) comprising a CAR polypeptide at least 80%, 85%, 86%, 87%, 88%, 89%, 90%, 91%, 92%, 93%, 94%, 95%, 96%, 97%, 98% or 99% identical or identical to the NY-ESO-1R-CD28Tm-CD137-Z CAR (SEQ ID NO: 22); NY-ESO-1R-CD8a-CD28-Z CAR (SEQ ID NO: 24); or NY-ESO-1R-CD8a-CD137-Z CAR (SEQ ID NO: 26).

In still further aspects, a method of the embodiments additionally comprises enriching the cell population for antigen-expressing engineered APCs. In some aspects, the enriching comprises fluorescence-activated cell sorting (FACS). In a specific aspect, the enriching comprises sorting for antigen expressing.

A further embodiment there is provided a method of making engineered APCs of the embodiments comprising obtaining a sample of cells comprising APCs and transfecting the cells with nucleic acid comprising a transgene encoding a target antigen and a transgene encoding a human leukocyte antigen (HLA), such that said HLA is expressed on the surface of the APC in complex with an epitope of the target antigen to produce a population of engineered APCs. In certain aspects, the APCs are T-cells or T-cell progenitors. In the further aspects, the method additionally comprises purifying or enriching APCs in the sample prior to transfection. In specific aspects, the T-cells or T-cell progenitors are derived from induced pluripotent stem cells, embryonic stems cells or hematopoietic stem cells. In further aspects, enriching T-cells in the sample comprises collecting a mononuclear cell fraction. In some aspects, the sample of cells may be from umbilical cord blood, a lymphoid organ or a peripheral blood sample from the subject. In certain aspects, the sample of cells may be obtained by apheresis or venipuncture. In still further aspects, the sample of cells is a subpopulation of T-cells. In some specific aspects, the transgenic CAR cells are inactivated for expression of an endogenous T-cell receptor and/or endogenous HLA. In particular aspects, obtaining the sample of cells comprises obtaining the cells from a 3rd party.

In some aspects, the transfection of APCs comprises electroporating DNA encoding transgenes into the APCs to generate engineered APCs. In further aspects, APCs can be transduced using a viral vector. In some aspects, the transfection may or may not involve infecting or transducing the cells with virus. In further aspects, the cells are additionally transfected with a nucleic acid encoding a membrane-bound Cγ cytokine. In certain aspects, the membrane-bound Cγ cytokine may be a membrane bound IL-7, IL-15 or IL-21. In a specific aspect, the membrane-bound Cγ cytokine is IL-15-IL-15Rα fusion protein.

In a further aspect, the transfection of APCs additionally comprises introducing a transposase into the cells and wherein at least one of the transgenes is flanked by a transposon repeat. In some aspects, the transposase may be provided as a DNA expression vector, an mRNA, a polypeptide, or an expressible RNA. In a specific aspect, the transposase is salmonid-type Tc1-like transposase (SB). In a further specific aspect, the transposase is the SB11 or SB100x transposase.

In yet a further aspect of the embodiments, a method of producing engineered APCs comprises an additional step of culturing the population of engineered APCs cells *ex vivo* in a medium that enhances proliferation of the APCs. In some aspects, culturing the engineered APCs may comprise culturing the cells in the presence of antigen-specific immune effector cells, such as T-cells, said cells having specificity to an epitope of the target antigen in complex with the HLA expressed in the APCs. In certain aspects, immune effector cells may be obtained from the same subject as the APCs. In further aspects, the immune effector cells have been engineered to express a T-cell receptor (TCR) that binds to an epitope of the target antigen in complex with the HLA expressed in the APCs. In a specific aspect, the TCR is an αβTCR. In still further aspects, the immune effector cells have been engineered to express a chimeric antigen receptor (CAR) that binds to an epitope of the target antigen in complex with the HLA expressed in the APCs. In some aspects, the CAR may comprise antibody variable domains that bind to an epitope of the target antigen in complex with the HLA expressed in the APCs.

In a further aspect, the engineered APCs of the embodiments have been tested for and confirmed to be free of infectious material. In some aspects, culturing the engineered APCs in the presence of immune effector cells may comprise culturing the cells in a medium comprising IL-21 and/or IL-2. In further aspects, culturing the engineered APCs in the presence of immune effector cells may comprise culturing the cells at a ratio of about 10:1 to about 1:10 (immune effector cells to APCs). In certain aspects, culturing of engineered APCs is for no more than 7, 14, 21, 28, 35 or 42 days.

Still a further embodiment there is provided an engineered APC population made by a method of any embodiments described herein.

In further embodiments, a cell composition is provided comprising engineered APCs and/or antigen specific immune effector cells. The cell composition may comprise as low as a hundred or 1,000 cells. However, in some aspects, the cell composition comprises at least about 10⁴, 10⁵, 10⁶, 10⁷, 10⁸, 10⁹, 10¹⁰, 10¹¹ or more engineered APCs and/or antigen specific immune effector cells. In one aspect, the cells may have essentially identical genetic material. In some aspects, the cells may be human derived from a single subject, who may be a donor or a patient.

In still a further embodiment, a pharmaceutical composition is provided comprising a cell composition comprising engineered APCs of the present embodiments and a pharmaceutically acceptable carrier.

In yet a further embodiment, a method of treating a disease in a patient is provided comprising administering an effective amount of a cell population comprising engineered APCs or pharmaceutical composition comprising engineered APCs. In one aspect, the patient may be a human patient. In certain aspects, the disease may be cancer. In further aspects, the cancer may be a hematological or solid cancer, such as T-cell ALL, B-ALL, CML, colon cancer, ovarian cancer, neuroblastoma, brain tumor(s), or pancreatic cancer. In some aspects, the patient may have undergone a previous anti-cancer therapy. In one aspect, the patient may be in remission. In yet another aspect, the patient may be free of symptoms of the cancer but comprise detectable cancer cells. In another aspect, the disease may be a viral infection (*e.g*., cytomegalovirus (CMV), Epstein-Barr virus (EBV), or human immunodeficiency virus (HIV)). In yet another aspect, the disease may be a bacterial infection. In one aspect, the disease may be sepsis.

In some further aspects, the cell composition comprising engineered APCs may be allogeneic to the patient. In various aspects, an allogeneic cell composition may or may not share HLA with the patient. In another aspect, the cell composition comprising engineered APCs may be autologous to the patient.

In a further embodiment, a method of treating a disease in a patient is provided comprising producing a cell composition comprising engineered APCs of the present embodiments and administering an effective amount of said cell composition to a patient in need thereof.

In some aspects, methods are provided for treating an individual with a medical condition comprising the step of providing an effective amount of engineered APCs of the embodiments, including more than once in some aspects, such as at least 1, 2, 3, 4, 5, 6, 7, 8, 9, 10, 11, 12, 13, 14, or more days apart.

In yet a further embodiment provided herein is a method to identify and select high-energy immune effector cells (*e.g*., CAR+ T cells) after gene modification for therapeutic purposes. Accordingly, in one embodiment, a method is provided for selecting CAR-modified immune effector cells with mitochondrial spare respiratory capacity. In some aspects, the method comprises detecting an expression level of a mitochondrial reporter gene and selecting immune effector cells with an elevated expression level of the mitochondrial reporter gene, thereby selecting immune effector with mitochondrial spare respiratory capacity. In some aspects, the immune effector cells may be human cells, such as CAR-modified human T cells.

In some aspects, the mitochondrial reporter gene for use according to the embodiments may be an endogenous gene. In some aspects, the mitochondrial reporter gene may be an exogenous gene, such as a gene encoding a fluorescent reporter protein. In some aspects, the fluorescent reporter protein may comprise a mitochondrial localization sequence. In certain aspects, a method for selecting immune effector cells having SRC may comprise flow cytometry or FACS.

In certain aspects, expression of the reporter gene for identifying immune effector cells with SRC may be under the control of a nuclear promoter (*e.g*., hEF1a). In certain aspects, expression of the reporter gene may be under the control of a mitochondrial promoter. In certain aspects, the expressed reporter protein may comprise a mitochondrial localization sequence. In certain aspects, the expressed reporter protein may be directed to the cell surface. In certain aspects, expression of the reporter gene may be under the control of a mitochondrial promoter and the expressed reporter protein may be directed to the cell surface. In some aspects, an exogenous reporter gene may be flanked by a transposon repeat or a viral LTR. In some aspects, an exogenous reporter gene may be comprised in an extrachromosomal nucleic acid, such as an mRNA or an episomal vector.

In some aspects, immune effector cells selected for SRC (*e.g*., CAR-modified T cells) may be expanded *ex vivo* for a period of time, which, in some aspects, may comprise culturing the cells with feeder cells, such as Activating and Propagating Cells (AaPC) containing a CAR ligand or an antibody that binds to a CAR (*e.g*. 2D3 scFv). In one aspect, the AaPCs may be transgenic K562 cells. In one aspect, the AaPCs may express CD137L. In other aspects, the AaPCs may further express CD19, CD64, CD86, or mIL15. In certain aspects, the AaPCs may expression at least one anti-CD3 antibody clone, such as, for example, OKT3 and/or UCHT1. In one aspect, the AaPCs may be inactivated (*e.g*., irradiated). In one aspect, the AaPCs may have been tested for and confirmed to be free of infectious material. Methods for producing such AaPCs are known in the art. In one aspect, culturing the CAR-modified T cell population with AaPCs may comprise culturing the cells at a ratio of about 10:1 to about 1:10; about 3:1 to about 1:5; about 1:1 to about 1:3 (T cells to AaPCs); or any range derivable therein. For example, the co-culture of T cells and AaPCs can be at a ratio of about 1:1, about 1:2 or about 1:3. In one aspect, the culturing step may further comprise culturing with an aminobisphosphonate (*e.g*., zoledronic acid).

In a further embodiment, a population of immune effector cells (*e.g*., CAR-modified T cells) is provided with mitochondrial spare respiratory capacity and a pharmaceutically acceptable carrier. The population of cells may be selected according to a method of the present embodiment. In some aspects, the cells may be therapeutically viable. In some aspects, the population of cells may comprise at least 10, 1 x 10², 1 x 10³, 1 x 10⁴, 1 x 10⁵, or 1 x 10⁶ cells, or any number of cells derivable therein. In various aspects, the cells may be NK-cells, natural killer T cells, αβ T cells, or γδ T cells. In one aspect, the immune effector cells may have essentially identical genetic material. In some aspects, the immune effector cells may be human CAR-modified T cells. In some aspects, the cells may be derived from a single subject, who may be a donor or a patient.

In still a further embodiment, a method of treating a disease in a patient in need thereof is provided, the method comprising administering an effective amount of a population of immune effector cells (*e.g*., CAR-modified T cells) with or selected for mitochondrial spare respiratory capacity. In some aspects, the method may comprise administering from about 100 to about 1 x 10⁶ cells, or any number of cells derivable therein, to the patient. In some aspects, the patient may be a human patient. In certain aspects, the population of immune effector cells with SRC may be allogeneic to the patient. In various aspects, an allogeneic cell composition may or may not share HLA with the patient. In certain aspects, the population of immune effector cells with SRC may be autologous to the patient. In various aspects, the method may further comprise administering a second therapy to the patient.

In some aspects, the disease for treatment with immune effector cells with (or selected for) SRC may be cancer. In certain aspects, the cancer may be a hematological or solid cancer, such as T-cell ALL, B-ALL, CML, colon cancer, ovarian cancer, neuroblastoma, brain tumor(s), or pancreatic cancer. In some aspects, the patient may have undergone a previous anti-cancer therapy. In one aspect, the patient may be in remission. In yet another aspect, the patient may be free of symptoms of the cancer but comprise detectable cancer cells. In another aspect, the disease for treatment with immune effector cells with SRC may be a viral infection (*e.g*., cytomegalovirus (CMV), Epstein-Barr virus (EBV), or human immunodeficiency virus (HIV)). In yet another aspect, the disease may be a bacterial infection. In one aspect, the disease may be sepsis.

In one embodiment, a method of treating a disease in a patient is provided comprising producing a cell composition comprising immune effector cells with SRC according to the embodiments and administering an effective amount of said cell composition to a patient in need thereof. In some aspects, methods are provided for treating an individual with a medical condition comprising the step of providing an effective amount of immune effector cells with SRC, including more than once in some aspects, such as at least 1, 2, 3, 4, 5, 6, 7, 8, 9, 10, 11, 12, 13, 14, or more days apart.

In one embodiment, a composition is provided comprising a cell population of the present embodiments, for use in the treatment of a disease in a patient.

Embodiments discussed in the context of methods and/or compositions of the embodiments may be employed with respect to any other method or composition described herein. Thus, and embodiment pertaining to one method or composition may be applied to other methods and compositions of the embodiments as well.

Other objects, features and advantages of the embodiments will become apparent from the following detailed description. It should be understood, however, that the detailed description and the specific examples, are given by way of illustration only, since various changes and modifications within the spirit and scope of the embodiments will become apparent to those skilled in the art from this detailed description.

### BRIEF DESCRIPTION OF THE DRAWINGS

**FIG. 1****:** An example schematic of chimeric antigen receptor targeted to the CD19 receptor. The CAR construct (CD19RCD28) includes a CD19 binding domain (VL/VH); a hinge domain (IgG4-Fc); a transmembrane domain (CD28 TM) and an intracellular signaling domain (CD28- CD3ζ).
**FIG. 2A****-B:** A, Figure shows scatter plots obtained by flow cytometry showing expression of the CD19RCD28 CAR on T cells, 1 day after electroporation and after 28 days of co-culture with K562 aAPC. B, Figure is scatter plots obtained by flow cytometry showing *in vitro* binding of Fc receptors (CD32 (FcγR2a), CD64 (FcγR1a)) by CD19RCD28 CAR T-cells.
**FIG. 3A****-B:** Limited *in vivo* persistence due to FcR binding may reduce efficacy. NSG mice were injected with hRLuc⁺ NALM6 tumor followed by a single injection of CD19RCD28 T cells (or control injection). A, Bioluminescence associated with tumor was measured via imaging over time. B, Graph shows the tumor burden in treated or control animals over time based on the measure luminescence levels.

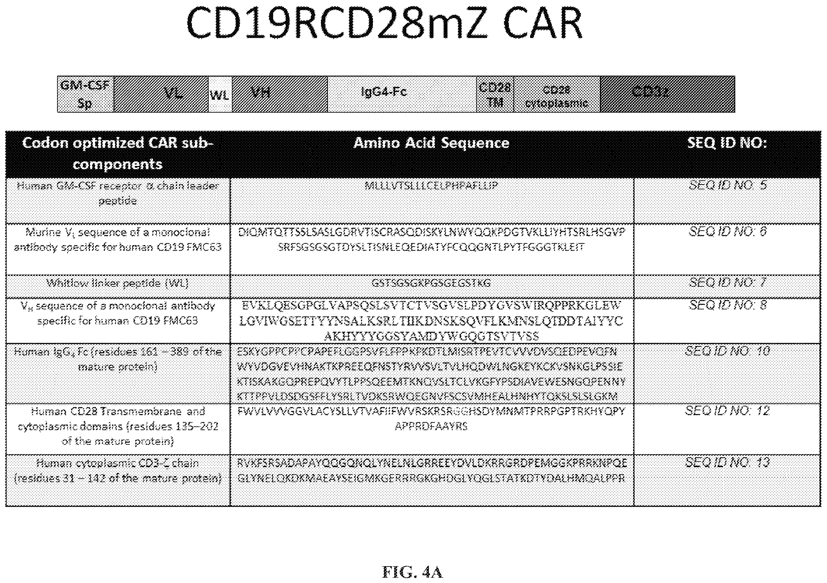

**FIG. 4A****-H:** The figures show schematics of various CARs that were constructed and tested. Sequences of selected CAR domains are also provided. For the human IgG4 hinge region, the hinge was mutated from amino acids CPSC (full-length sequence provided as SEQ ID NO: 9) to CPPC (full-length sequence provided as SEQ ID NO: 10) to enhance stability of the dimerized IgG4 heavy chain. The human IgG4 Fc (EQ mutant, having amino acid changes L235E, and N297Q) is provided as SEQ ID NO: 1. The human CD8a chain hinge and transmembrane domain is provided as SEQ ID NO: 3. The 12 amino acid spacer from human IgG4 Fc was mutated from amino acids CPSC (provided as SEQ ID NO: 18) and CPPC (provided as SEQ ID NO: 2) to enhance stability of the dimerized IgG4 heavy chain provided as SEQ ID NO: 2. For the human CD28 transmembrane cytoplasmic domain, the human CD28 transmembrane and endodomain was modified from RLLH (full-length sequence provided as SEQ ID NO: 11) to RGGH (full-length sequence provided as SEQ ID NO: 12) to improve expression of the CAR. For the human CD28 cytoplasmic domain, the human CD28 endodomain was modified from RLLH (full-length sequence provided as SEQ ID NO: 16) to RGGH (full-length sequence provided as SEQ ID NO: 17) to improve expression of the CAR.
**FIG. 5****:** The figure shows scatter plots obtained by flow cytometry showing expression of various CARs of CAR on T cells, 1 day after electroporation and after 28 days of co-culture with K562 aAPCs.
**FIG. 6****:** The figure shows scatter plots obtained by flow cytometry showing reduced *in vitro* binding of Fc receptors (CD32 (FcγR2a), CD64 (FcγR1a)) by CD19R*CD28 CAR T cells.
**FIG.** 7: The figure shows a graph depicting the expansion kinetics of various CAR T cells over 28 days of co-culture with K562 aAPCs.
**FIG. 8****:** The figure shows a graph depicting the CD19-specific cytotoxicity shown by various CAR T cells relative to CD19EL-4 and NALM-6 target cells.
**FIG. 9****:** The figure shows a graph depicting CD19-specific IFN-gamma production by various CAR T cells when contacted with different target cells.
**FIG. 10****:** The figure shows luminescence imaging of NSG mice that were injected (i.v.) with tumor (hRLuc+ NALM-6) followed by injection (i.c.) with ffLuc+ CAR+ T cells the next day. Tumor and T cell imaging was performed after injection of EnduRen or Luciferin respectively using Xenogen IVIS 100 series system. T cells were imaged immediately after to confirm intracardiac injection (top row). Shaded images represent photon flux from tumor-derived hRLuc activity over time is shown.
**FIG. 11****:** Improved *in vivo* efficacy of CAR+ T cells to control disease. The figure shows a graph representing tumor burden based on the bioluminescence measurements in FIG. 10. As detailed above, NSG mice were injected with hRLuc+ NALM-6 tumor followed by a single injection of CAR T cells. Tumor associated bioluminescence was measured over time and is shown. (*) represents at least p<0.05 significance over tumor only group.
**FIG. 12****:** The figure shows a graph depicting survival curves for NSG mice treated with various CAR T cells. The significance (p value) is calculated when compared to tumor only (no treatment) group.

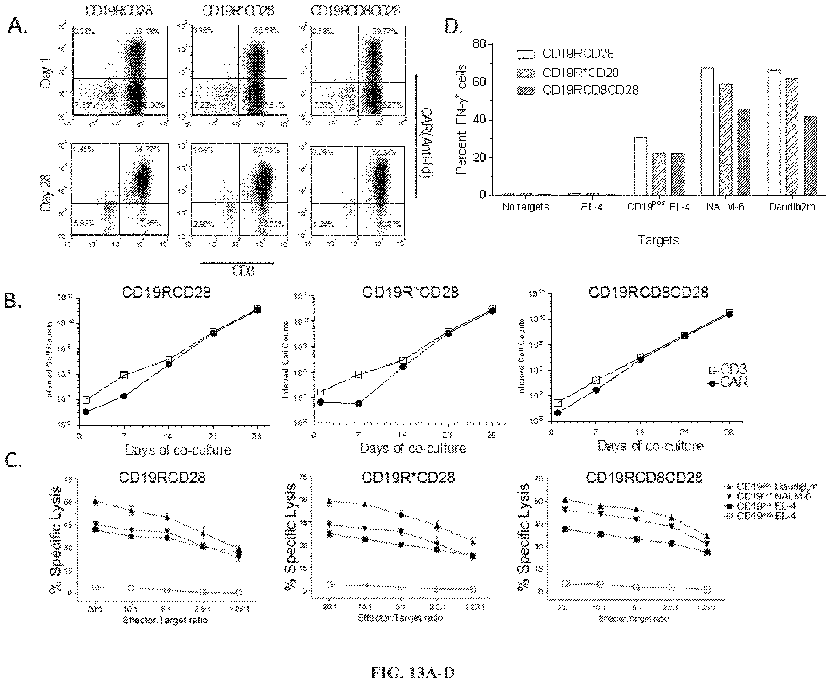

**FIG. 13A****-D:** Characterization of CAR⁺ T cells. PBMCs were electroporated with CD19R^{∗}CD28 or CD19RCD8CD28 transposon & SB11 transposase and co-cultured on irradiated K562 antigen presenting cells along with IL-2 and IL-21 for 28 days in a 7-day stimulation cycle. Cells were enumerated and phenotyped (CD3, CAR) at the end of each stimulation cycle. (A) Expression of CAR on CD3⁺ T cells day after electroporation (day 1) and after 28 days of co-culture. (B) Inferred cell counts for CD3 and CAR+ T cells showing expansion kinetics. (C) CD19-specific cytotoxicity was measured using standard chromium release assay against CD19^{pos} targets (CD19⁺ EL-4, NALM-6, Daudiβ₂m). Background lysis is depicted by lysis of CD19^{neg} EL-4. (D) Intra-cellular production of IFN-γ by CAR⁺ T cells when stimulated with various effector targets expressing CD19.

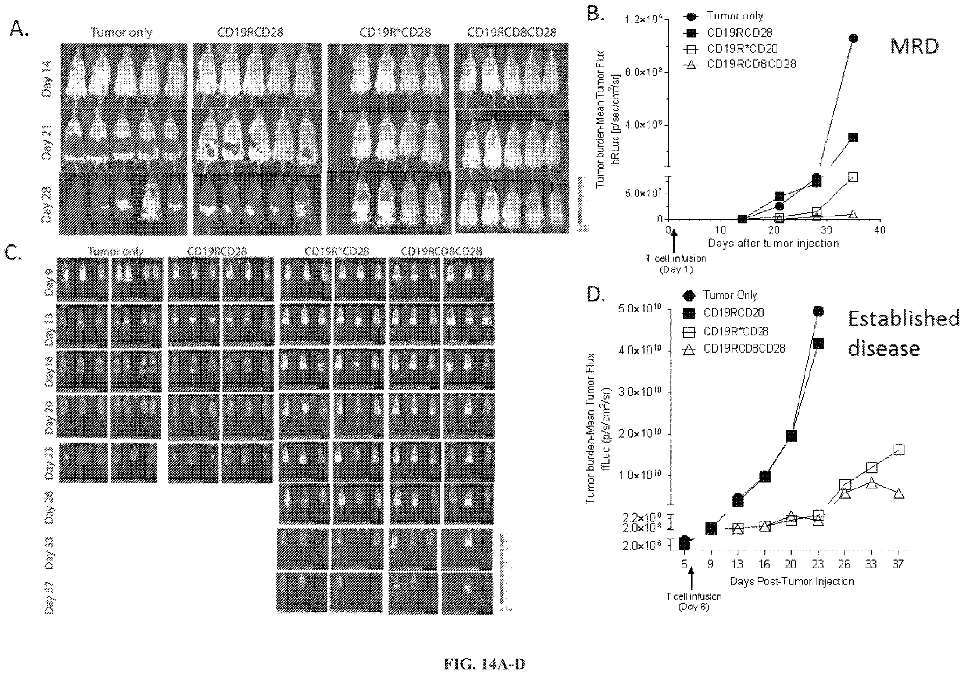

**FIG. 14A****-D:** *In vivo* efficacy of CAR⁺ T cells against CD19⁺ NALM-6 cell line in NSG (NOD*.*Cg*-Prkdd^{scid} Il2rg^{tm1Wjl}*/SzJ*,* NOD *scid* gamma) mice. (A-B) In a minimal residual disease model, NSG mice (n=5/group) were injected with 10⁴ cells of hRluc⁺mKate⁺NALM-6 on day 0, followed by injection of T-cells (10⁷/mouse) intra-cardiacally on day 1. Tumor burden was evaluated by bioluminescent imaging derived from bRluc⁺NALM-6 using EnduRen Live Cell Substrate over time. (C-D) For an established tumor model, NSG mice (n=6/group) were injected with 1.5x10⁴ ffLuc⁺EGFP⁺NALM-6 on day 0 and tumor was allowed to engraft for 5 days, tumor burden was evaluated and T cells (10⁷/mouse) injected intracardiacally on day 6. Tumor flux was calculated using bioluminescent imaging from ffLuc⁺NALM-6 using D-Luciferin substrate over time. False-color images representing photon flux and tumor-flux over time is shown.
**FIG. 15****:** The figure shows additional signaling molecules for CAR T-cells. Upper panel shows a membrane-bound IL-15/IL-15 receptor (mIL15) construct (the polypeptide sequence for the construct is provided as SEQ ID NO: 4). Lower panels are schematics of signaling provided by the membrane-bound IL-15/IL-15 receptor (left panel) and by CAR construct (right panel).

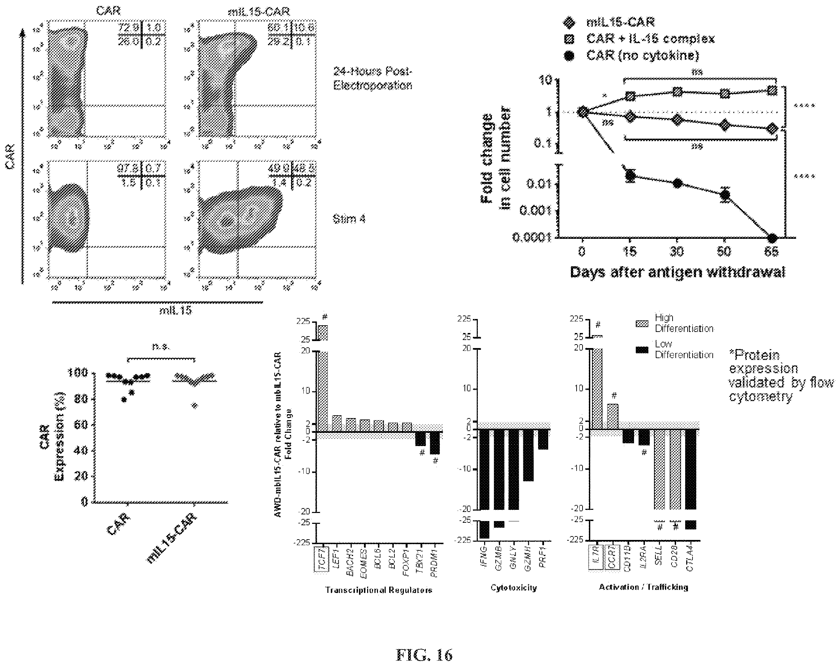

**FIG. 16****:** Stable co-expression of mIL15 and CAR. Upper left panel shows a representative flow plot of mIL15+CAR+ T cells 24-hours post-electroporation or from stimulation 4 (Stim 4). Lower left panel is a graph showing the percentage of electroporated cells that express CAR or CAR and mIL15. Upper right panel, graph shows the change in CAR T-cell number in culture following antigen withdrawal. Studies were completed without IL-15, with added IL-15 complex or with cells co-expressing mIL-15 and CAR. Lower right panel shows a graph of expression levels for various transcriptional regulators in CAR T-cells expressing mIL-15.
**FIG. 17A****-B:** A, The figure shows luminescence imaging of mice that were injected with ffLuc+ T cells either expressing CAR alone or in combination with mIL-15. T cell imaging was performed after injection of Luciferin respectively using Xenogen IVIS 100 series system. Shaded images represent photon flux from ffLuc+ T cells activity over time. B, Left panel, graph shows expression levels of factors in culture mIL-15-CAR T-cells in the presence of antigen or after antigen withdrawal (WD). Right panel shows a histogram of CAR T cells and mIL-15-CAR T-cells with and without stimulation.

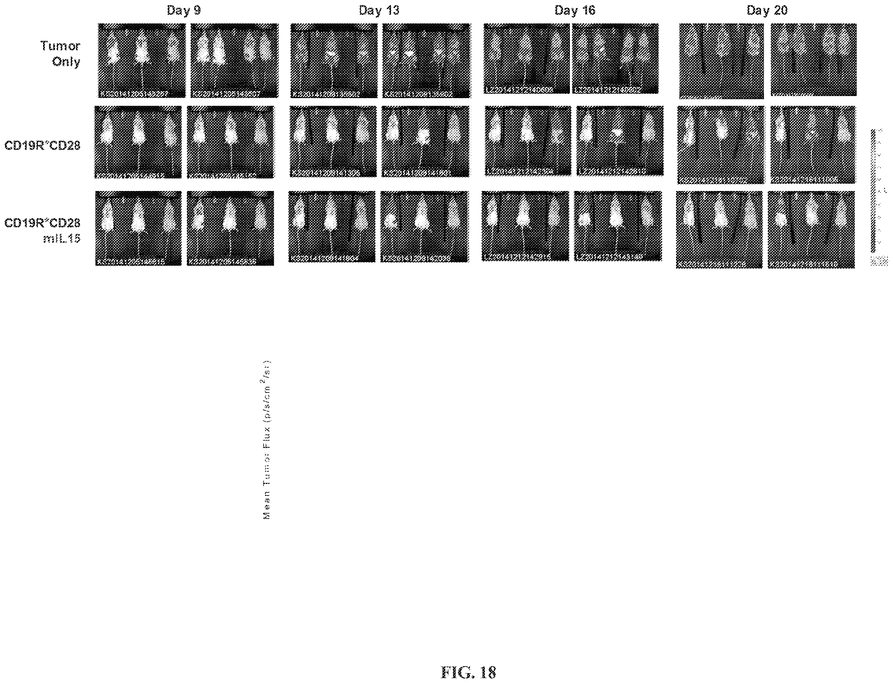

**FIG. 18****:** *In vivo* control of tumor growth by CAR T-cells. The figure shows luminescence imaging (upper panels) and quantitation (lower panel, graph) of mice that were injected with hRLuc+ tumor cells. As indicated, certain mice were also injected with T cells expressing CAR or CAR and mIL-15.

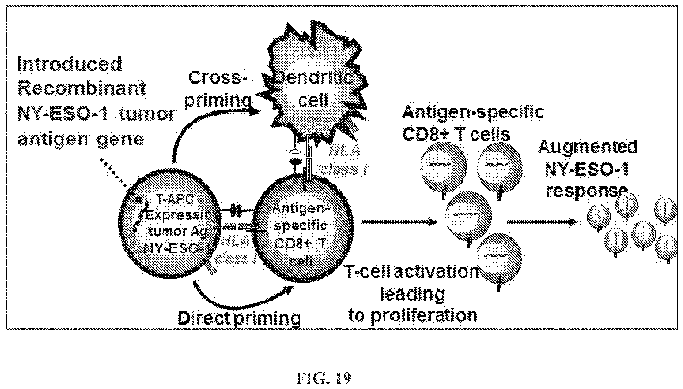

**FIG. 19****:** Schematic drawing illustrating T cells functioning as antigen-presenting cells (T-APC). T cells post autologous transplant as well as improve endogenous long-term immunity through both direct and cross-priming.
**FIG. 20****:** Graphic representation of the Sleeping Beauty plasmids used to genetically modify human T-cells to express the tumor antigen NY-ESO-1. Constitutive expression is driven by the hEF1-alpha promoter and selection is achieved by propagating the T-cells under 0.2 mg/mL Hygromycin.
**FIG. 21****:** Expression of T cell costimulatory molecules on K562 for use as aAPC.
**FIG. 22****:** Flow cytometry of human T-APC on Day 28 of co-culture on OKT3-aAPC after electro-transfer of SB plasmid co-expressing FLAG-tagged NY-ESO-1 and HyTK.
**FIG. 23**: PBMC expanded on NY-ESO-1⁺T-APC (upper) vs K562 aAPC (lower).
**FIG. 24**: NY-ESO-1⁺ CAR T cells expanded on NY-ESO-1⁺T-APC vs K562 aAPC.
**FIG. 25**: Inhibitory molecules on NY-ESO-1⁺T-APC after 3 stimulations.
**FIG. 26**: hTY-ESO-1⁺mIL-15⁺HLA-A2⁺T-APC propagated in the presence of aAPC.
**FIG. 27****:** T-APC expansion. The T-APC were successfully able to expand the CAR T cells as well as the positive control, the K562-derived aAPC.
**FIG. 28****:** Chromium release assay showing killing of NY-ESO-1⁺ U266 human multiple myeloma cell line by NY-ESO-1 specific TCR⁺ and CAR⁺ T cells. The T-cells were expanded on artificial activating and propagating cells (AaPC) derived from K562 and expresses HLA-AO2 and NY-ESO-1⁺. Data represents two independent experiments where the NY-ESO-1 specific CAR T-cells of 9-2-15 where propagated for 3 stims with HLA-A2/NY-ESO-1 T-APC and 2 stims with AaPC K562 derived. The 9-2-15 CAR contains the CD8a stalk while the previous one contained the IgG4 stalk.

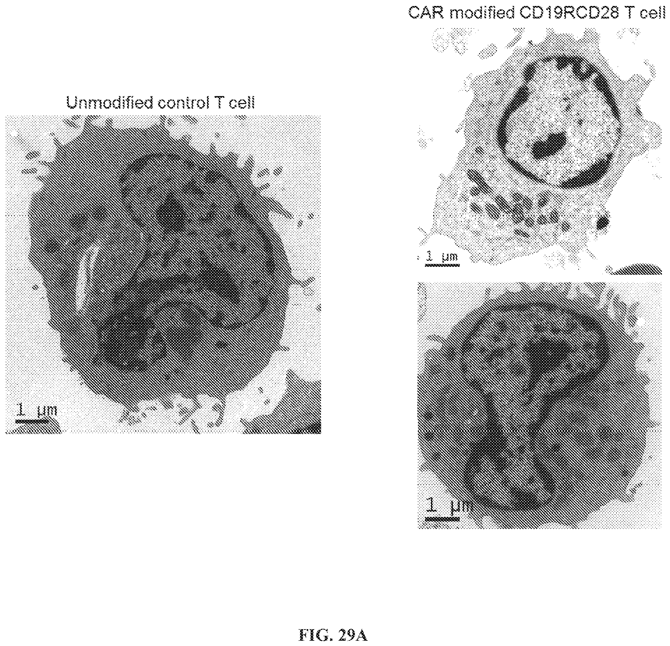

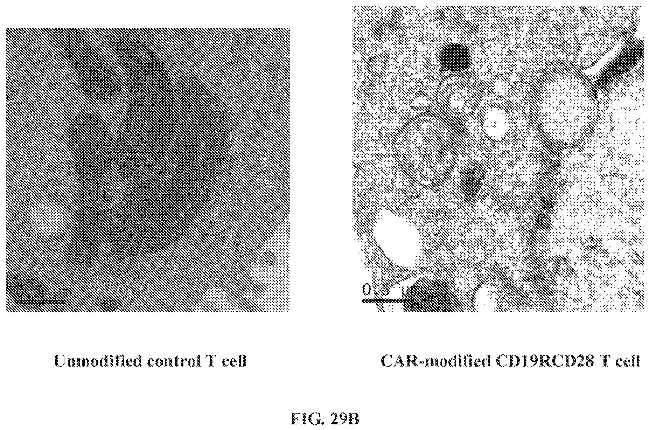

**FIGS. 29A-29B****:** FIG. 29A. TEM (transmission electron microscopy) images of gene modified T cells along with control T cells grown *ex vivo* in the presence of irradiated K562 AaPC. An increase in number of mitochondria is seen in CAR modified cells (right panel) as compared to the unmodified control cells (left panel) activated under similar conditions. FIG. 29B. Transmission electron microscopy (TEM) images (scale 500 nm, 50,000 X magnification) showing regular orthodox structure of mitochondria in unmodified control T cells as compared to condensed state of mitochondria in CAR-modified T cells propagating in culture at Day 35.

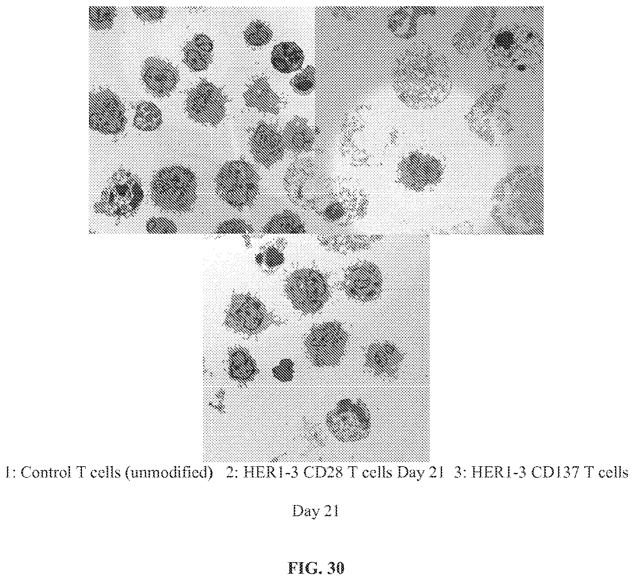

**FIG. 30****:** *Ex vivo* expansion of HER1-3 bi-specific CAR-T cells without co-stimulation. Cells were either unmodified or CAR modified with CD28 and/ or CD137 T-cell signaling endodomain. Cells with the CD137 T-cell signaling endodomain survived better in culture as opposed to CAR T cells containing CD28 signaling domain when no co-stimulation provided in culture. OKT3 stimulation for unmodified control cells worked better may be because of higher signaling strength induced through CD3z. TEM images of CAR-T cells targeting tumor antigen HER1-3 propagated *ex vivo* in the presence of K562 AaPC are shown. This was also tested for other tumor antigen targeted CARs, such as ROR1, CD123, and C19. Viable absolute T-cell numbers were counted by a Nexcelom Cellometer® using Trypan blue live/dead exclusion method.
**FIG. 31****:** SB plasmid constructs for fluorescent protein reporter. GRX2 is the mitochondrial localization sequence derived from GLRX2 (Pubmed ID# NP_932066.1). NLS is the nuclear localization sequence (Pubmed ID# NM_017921).
**FIG. 32****:** Flow cytometry analysis of endogenous expression of EYFP in CAR modified cells. Emergence of CAR+ T cells with high EYFP in CD137-CAR T cells grown without co-stimulation on Day 35 of culture. CD28-T cells showed fewer or dimmer EYFP-positive T cells. Flow cytometry was based on Fc staining for CAR expression and endogenous EYFP for mitochondrial strength.

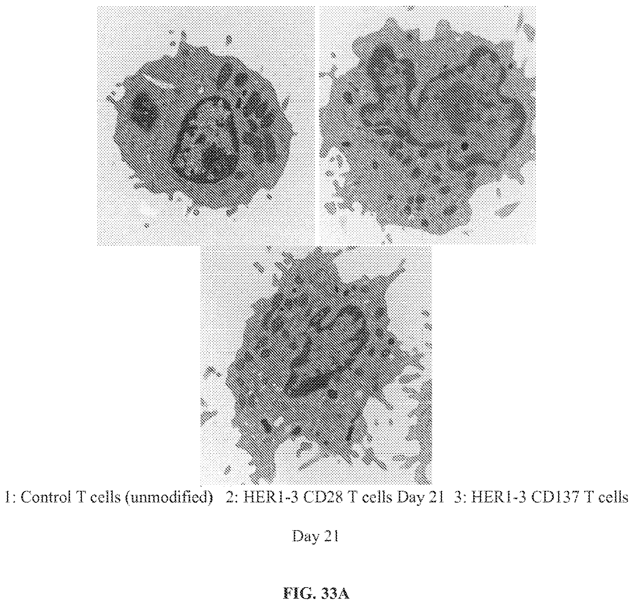

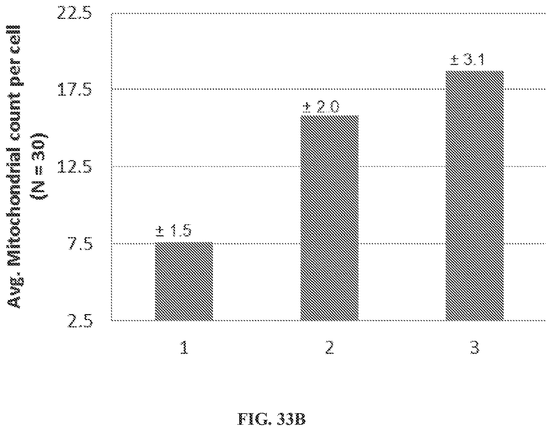

**FIGS. 33A-33B****:** Mitochondrial biomass of HER1-3 Bi-specific CAR-T cells expanded with irradiated feeder cells (K562 AaPC) without T-cell co-stimulation. FIG. 33A. Transmission electron microscopy (TEM) images (scale 500 nm, 15,000X magnification) showing the distribution of mitochondria in unmodified T cells and T cells modified to express a prototypical CAR targeting HER1-HER3 (consisting of either a CD28-CD3z or a CD137-CD3z T cell signaling endodomain). FIG. 33B. Average numbers of mitochondria per cell are represented in the bar graph. Overall CAR-T cells with the CD137 T cell signaling domain showed increase in biomass after Day 21 of culture as compared to CAR-T cells containing the CD28 signaling domain.
**FIG. 34****:** *In vitro* cytotoxicity of high energy T cells (eT). High-energy T cells sorted for high mitochondrial mass were found to retain cytotoxic ability as evidenced by specific killing of tumor antigen positive breast cancer cells. The data are from a chromium release assay performed on Cr-labeled HER1-3⁺ T cells against breast tumor lines positive for specific antigens, which were lysed by CAR T cells with high EYFP/mitochondrial strength.

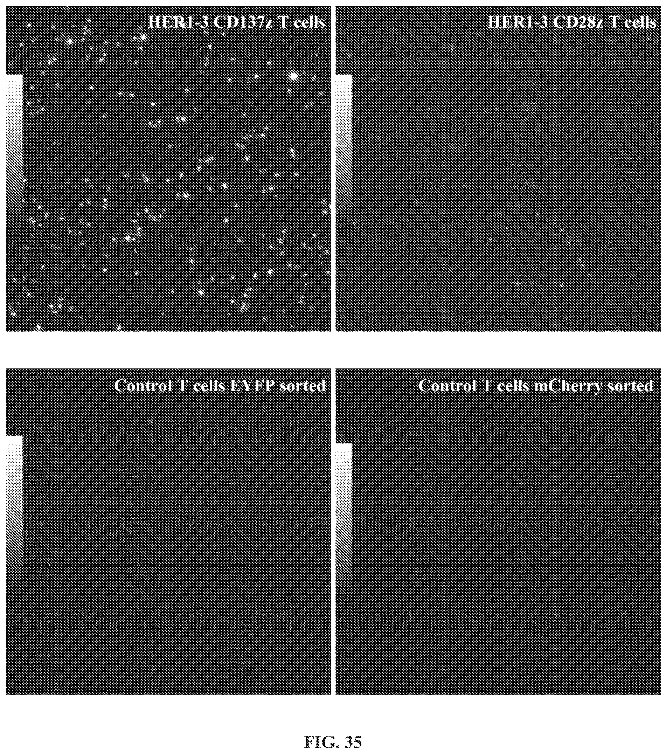

**FIG. 35****:** Fluorescence microscopy images of unmodified and CAR-modified T cells expanded *ex vivo* in the presence of irradiated feeder cells (K562 AaPC) with minimal activation and complete absence of co-stimulation. CAR-T cells containing CD137 T cell signaling endodomain showed higher distribution of mitochondrial mass as observed from fluorescence signal intensity emanating endogenously from the reporter protein (EYFP-GRX2). The number of metabolically active T cells was significantly higher in CAR-T cells containing the CD137 T cell signaling endodomain as compared to CAR-T cells containing the CD28 signaling domain. This was consistent, irrespective of donor or CAR types.
**FIG. 36****:** Pattern of mitochondrial distribution in *ex vivo* expanded CAR-modified T cells grown in the presence of irradiated feeder cells (K562 AaPC).
**FIGS. 37A-37B****:** Integrated morphometry analysis of fluorescence microscopy images show a heterogeneous population of CAR-modified T cells based on mitochondrial strength within gene modified T-cell groups representing a particular signaling endodomain (HER1-HER3 CD137-CD3z T cells). FIG. 37A. Population variation of EYFP-Mito among HER1-3 CD137 CAR⁺ T cells. FIG. 37B. Average intensity of EYFP-Mito among HER1-3 CD137 CAR⁺ T cells.
**FIG. 38****:** Transmission electron microscopy (TEM) images of CAR+T cells grown on universal K562-AaPC. Images are from day 14 of the study described in Example 8.
**FIG. 39****:** Growth kinetics of ROR1-CAR T cells on universal K562-AaPC. CAR-T cells containing CD28 signaling rapidly underwent apoptosis (left chart), whereas CAR-T cells containing CD137z signaling persisted longer (right chart).

### DETAILED DESCRIPTION

Clinical trials have demonstrated anti-tumor effects in patients that have received genetically modified T-cells. For example, CAR-T cell therapy has been shown to deliver meaningful remission in human patients afflicted with advanced B-cell malignancies (Maude *et al*., 2014). CAR-T cells have the requisite specificity and cytotoxic ability to seek and destroy tumor cells in the body and can persist long enough to prevent relapse. This classic situation of serial killing (one T-cell killing multiple tumor cells without undergoing anergy) has been shown recently in several patients treated under various Phase I trials (Corrigan-Curay *et al*., 2014). However, a uniform and unambiguous clinically relevant outcome is yet to emerge across CAR design, CAR T-cell endodomain, spacer length usage, affinity of the scFv, etc (Jena *et al*., 2014). This is compounded again by variable tumor burden and tumor types among patient population seeking treatment through CAR-T cell infusion. It is a major challenge to design a therapeutically effective CAR construct, and ultimately a gene-modified T-cell population, that will remit clinically relevant results (without significant toxicity) across the entire spectra of tumors types, tumor stages, and patient population.

Studies detailed herein identify key elements that play a role in CAR T-cell persistence, which can affect the efficacy of the T-cells and potential toxicity (*e.g*., from off-target cytotoxic activity). In particular, it is shown that the ability of the CAR polypeptides to be bound by Fc receptors modulates the persistence and anti-tumor efficacy of the T-cells. Thus, CAR T-cell efficacy (and *in vivo* persistence) can be altered by changing Fc receptor binding properties of the CAR polypeptides expressed on the T-cells. For example, Fc receptor binding sequences, such as from human immunoglobulin constant regions, can be included in a CAR to reduce persistence and control potential toxicity of the T-cells. Conversely, CAR polypeptides may be modified to remove Fc receptor-binding elements to increase *in vivo* persistence and enhance efficacy of CAR T-cells. Thus, the CAR polypeptides, CAR T-cells and methods detailed herein allow for the fine-tuning of CAR T-cell persistence (by adjusting Fc receptor binding) to control efficacy and toxicity of CAR T-cell therapies.

In a further embodiment, engineered antigen presenting cells (APCs) are provided that comprise a transgene encoding a target antigen and a transgene encoding a human leukocyte antigen (HLA). In some aspects, the engineered APCs may additionally comprise one or more transgenes encoding a co-stimulating factor (such as IL-15). Such engineered APCs may be used for example in *ex vivo* methods for expansion of T-cells (*e.g*., CAR T-cells) or may be administered to a subject to stimulate growth of antigen-specific T-cells *in vivo.* Thus, cells provided herein may be used to stimulate growth of antigen-specific T-cells to for treatment of a disease (*e.g*., cancer).

Further embodiments disclosed herein provide immune effector cells with or selected for SRC, which may provide enhanced therapeutic properties. Cells with a high mitochondrial biomass are referred to herein, for example, as "energy T cells" or "eT cells." Since increased persistence of gene-modified immune effector cells positively correlates with improved therapeutic potential, immune cells such as energy T cells are predicted to perform better than bulk gene-modified T cells. In addition, the influence of a multitude of variables on CAR design, and thus CAR efficacy, could be harnessed if long-term surviving CAR-T cells could be identified pre-infusion based on mitochondrial strength (*i.e*., spare respiratory capacity or SRC). Thus, mitochondrial strength may help with selecting/sorting the best serial T-cell killers irrespective of CAR endodomain use, CAR construct design, spacer length, and affinity of the scFv, thereby reducing the translational hurdle to optimize a CAR design that is suited for a particular tumor antigen and tumor type. Picking the best T cells (*e.g*., those with the greatest survival potential in a tumor microenvironment) pre-infusion may be advantageous in multiple situations. As such, a method is provided to measure the "fitness" of CAR+ T cells using a reporter fluorescent protein (*e.g*., EYFP-GRX2), which is quantifiable and correlates with mitochondrial SRC, which in turn indicates the metabolic ability of genetically-modified T cells to survive unfavorable conditions of low oxygen, absence of specific nutrients for glycolysis and activation induced cell death caused by high load tumor antigens.

Furthermore, selecting fewer, more potent CAR+ T cells for infusion may help reduce or even abrogate T cell-associated toxicity post-infusion. Limiting the number of cells needed for infusion will lessen the burden of growing large numbers of difficult-to-grow, patient-derived autologous T cells for genetic modification. For example, in methods of adoptive cell therapy, the use of low numbers of immune effector cells, such as T cells, to illicit high anti-tumor killing may be desirable. The use of low numbers of T cells may minimize infusion-related toxicity arising out of CAR-modified T cell infusion. Furthermore, it may be preferred if infused cells survive longer *in vivo* thereby preventing tumor relapse. In both of these situations, the present methods of identifying, selecting, and infusing high energy CAR-modified T cells will be beneficial.

There is heterogeneity in mitochondrial distribution as well as SRC among CAR-modified T cells for any particular batch. Thus, if selected carefully, fewer cells can achieve similar results *in vivo* as compared to a bulk population. This would be helpful for tumors where (i) the CAR cannot distinguish between normal versus tumor associated antigens and hence infusion of fewer cells is desirable, (ii) expansion of patient-derived autologous T cells is difficult due to an inherent deformity in original cell population, (iii) limited persistence of CAR-modified T cells *in vivo* after infusion is desired. Methods to determine SRC/mitochondrial strength can be based on labeling cells using a mito-tracker dye (Invitrogen) or similar post-modification method where retrieval of cells for clinical application is not possible. Other methods to determine mitochondrial strength, such as measuring oxygen consumption or extracellular acidification, measure the strength of bulk cells rather than the strength of individual cells, which also lack clinical application. As provided herein, a non-viral DNA electroporation method may be used to modify the effector cells, such as T cells simultaneously for CAR expression and SRC identification. Specifically, in order to select eT cells, expression of a fluorescent mitochondrial-directed reporter protein in cells along with a CAR molecule can be achieved by Sleeping Beauty-mediated transposition, a non-viral DNA based gene delivery system that has been adapted for clinical translation. Flow cytometry-based sorting of eT cells after gene modification and ligand-stimulated K562-based co-culture can be performed under GMP conditions, is amenable to translation, and can be readily adapted in the clinic. In summary, metabolically active T cells (eT cells) that can deliver desirable anti-tumor efficacy may be generated by combining clinically relevant approaches to genetically modify T cells, expanded to large numbers using irradiated K562-based co-culture with minimal activation, and identified based on expression of a fluorescent reporter protein.

### I. Definitions

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

As used herein, the term "antigen" is a molecule capable of being bound by an antibody or T-cell receptor. An antigen is additionally capable of inducing a humoral immune response and/or cellular immune response leading to the production of B and/or T lymphocytes.

As used herein the term "anti-tumor effective amount" refers to an effective amount of CAR-expressing immune effector cells to reduce cancer cell or tumor growth or to decrease tumor volume or number of tumor cells in a subject. "An anti-tumor effective amount" can also refer to an effective amount of CAR-expressing immune effector cells to increase life expectancy or to alleviate physiological effects associated with the tumor or cancer.

### II. Chimeric Antigen Receptors and Components

Chimeric antigen receptor molecules are recombinant fusion protein and are distinguished by their ability to both bind antigen (*e.g*., HER1/HER3) and transduce activation signals *via* immunoreceptor activation motifs (ITAM's) present in their cytoplasmic tails. Receptor constructs utilizing an antigen-binding moiety (for example, generated from single chain antibodies (scFv) afford the additional advantage of being "universal" in that they bind native antigen on the target cell surface in an HLA-independent fashion.

A chimeric antigen receptor according to the embodiments can be produced by any means known in the art, though preferably it is produced using recombinant DNA techniques. A nucleic acid sequence encoding the several regions of the chimeric antigen receptor can be prepared and assembled into a complete coding sequence by standard techniques of molecular cloning (genomic library screening, PCR, primer-assisted ligation, scFv libraries from yeast and bacteria, site-directed mutagenesis, etc.). The resulting coding region can be inserted into an expression vector and used to transform a suitable expression host allogeneic or autologous immune effector cells.

Embodiments of the CARs described herein include nucleic acids encoding an antigen-specific chimeric antigen receptor (CAR) polypeptide, including a comprising an intracellular signaling domain, a transmembrane domain, and an extracellular domain comprising one or more signaling motifs. In certain embodiments, the CAR may recognize an epitope comprised of the shared space between one or more antigens. In some embodiments, the chimeric antigen receptor comprises: a) an intracellular signaling domain, b) a transmembrane domain, and c) an extracellular domain comprising an antigen binding domain. Optionally, a CAR can comprise a hinge domain positioned between the transmembrane domain and the antigen binding domain. In certain aspects, a CAR of the embodiments further comprises a signal peptide that directs expression of the CAR to the cell surface. For example, in some aspects, a CAR can comprise a signal peptide from GM-CSF (see, *e.g*., SEQ ID NO: 5).

In certain embodiments, the CAR can also be co-expressed with a membrane-bound cytokine to improve persistence when there is a low amount of tumor-associated antigen. For example, CAR can be co-expressed with membrane-bound IL-15.

Depending on the arrangement of the domains of the CAR and the specific sequences used in the domains, immune effector cells expressing the CAR may have different levels activity against target cells. In some aspects, different CAR sequences may be introduced into immune effector cells to generate transgenic cells, the transgenic cells selected for elevated SRC and the selected cells tested for activity to identify the CAR constructs predicted to have the greatest therapeutic efficacy.

### A. Antigen binding domain

In certain embodiments, an antigen binding domain can comprise complementary determining regions of a monoclonal antibody, variable regions of a monoclonal antibody, and/or antigen binding fragments thereof. In another embodiment, that specificity is derived from a peptide (*e.g*., cytokine) that binds to a receptor. A "complementarity determining region (CDR)" is a short amino acid sequence found in the variable domains of antigen receptor (*e.g*., immunoglobulin and T-cell receptor) proteins that complements an antigen and therefore provides the receptor with its specificity for that particular antigen. Each polypeptide chain of an antigen receptor contains three CDRs (CDR1, CDR2, and CDR3). Since the antigen receptors are typically composed of two polypeptide chains, there are six CDRs for each antigen receptor that can come into contact with the antigen -- each heavy and light chain contains three CDRs. Because most sequence variation associated with immunoglobulins and T-cell receptors are found in the CDRs, these regions are sometimes referred to as hypervariable domains. Among these, CDR3 shows the greatest variability as it is encoded by a recombination of the VJ (VDJ in the case of heavy chain and TCR αβ chain) regions.

It is contemplated that the CAR nucleic acids, in particular the scFv sequences are human genes to enhance cellular immunotherapy for human patients. In a specific embodiment, there is provided a full length CAR cDNA or coding region. The antigen binding regions or domains can comprise a fragment of the VH and VL chains of a single-chain variable fragment (scFv) derived from a particular mouse, or human or humanized monoclonal antibody. The fragment can also be any number of different antigen binding domains of an antigen-specific antibody. In a more specific embodiment, the fragment is an antigen-specific scFv encoded by a sequence that is optimized for human codon usage for expression in human cells. In certain aspects, VH and VL domains of a CAR are separated by a linker sequence, such as a Whitlow linker (see, e.g., SEQ ID NO: 20). CAR constructs that may be modified or used according to the embodiments are also provided in International (PCT) Patent Publication No. WO/2015/123642, incorporated herein by reference.

In some specific examples the antigen binding domain of a CAR is specific for binding to HER2/Neu (Stancovski *et al*., 1993), ERBB2 (Moritz *et al*., 1994), folate binding protein (Hwu *et al*., 1995), a renal cell carcinoma (Weitjens *et al*., 1996), and HIV-1 envelope glycoproteins gp120 and gp41 (Roberts *et al*., 1994). Other cell-surface target antigens that could be bound by a CAR include, but are not limited to, CD20, carcinoembryonic antigen, mesothelin, ROR1, c-Met, CD56, GD2, GD3, αfetoprotein, CD23, CD30, CD123, IL-11Rα, κ, chain, λ chain, CD70, CA-125, MUC-1, EGFR and variants, epithelial tumor antigen, and so forth.

In certain embodiments of the chimeric antigen receptor, the antigen-specific portion of the receptor (which may be referred to as an extracellular domain comprising an antigen binding region) comprises a HER1/HER3 binding domain as detailed herein above. In some aspects, for example, the HER1/HER3 binding domain comprises one of the sequences provided in U.S. Patent Publication No. 2012/0121596, incorporated herein by reference.

### B. Hinge domain

In certain aspects, a CAR polypeptide of the embodiments can include a hinge domain positioned between the antigen binding domain and the transmembrane domain. In some cases, a hinge domain may be included in CAR polypeptides to provide adequate distance between the antigen binding domain and the cell surface or to alleviate possible steric hindrance that could adversely affect antigen binding or effector function of CAR-gene modified T cells.

In some cases the CAR hinge domain could be derived from human immunoglobulin (Ig) constant region or a portion thereof including the Ig hinge, or from human CD8 α transmembrane domain and CD8a-hinge region. In one aspect, the CAR hinge domain can comprise a hinge-CH₂-CH₃ region of antibody isotype IgG₄. In some aspects, point mutations could be introduced in antibody heavy chain CH₂ domain to reduce glycosylation and non-specific Fc gamma receptor binding of CAR-T cells or any other CAR-modified cells.

In certain aspects, a CAR hinge domain of the embodiments comprises an Ig Fc domain that comprises at least one mutation relative to wild type Ig Fc domain that reduces Fc-receptor binding. For example, the CAR hinge domain can comprise an IgG4-Fc domain that comprises at least one mutation relative to wild type IgG4-Fc domain that reduces Fc-receptor binding. In some aspects, a CAR hinge domain comprises an IgG4-Fc domain having a mutation (such as an amino acid deletion or substitution) at a position corresponding to L235 and/or N297 relative to the wild type IgG4-Fc sequence. For example, a CAR hinge domain can comprise an IgG4-Fc domain having a L235E and/or a N297Q mutation relative to the wild type IgG4-Fc sequence. In further aspects, a CAR hinge domain can comprise an IgG4-Fc domain having an amino acid substitution at position L235 for an amino acid that is hydrophilic, such as R, H, K, D, E, S, T, N or Q or that has similar properties to an "E" such as D. In certain aspects, a CAR hinge domain can comprise an IgG4-Fc domain having an amino acid substitution at position N297 for an amino acid that has similar properties to a "Q" such as S or T. In a further aspect, a CAR hinge domain comprises a mutation at a position corresponding to L235 and/or N297 relative to the wild type IgG4-Fc sequence and is about 90% identical to SEQ ID NO: 1 or SEQ ID NO: 10.

In certain specific aspects, the hinge domain comprises a sequence that is about 85%, 90%, 91%, 92%, 93%, 94%, 95%, 96%, 97%, 98%, 99% or 100% identical to: an IgG4-Fc domain of SEQ ID NO: 1 or SEQ ID NO: 10, a CD8a extracellular domain of SEQ ID NO: 20 or a synthetic hinge sequence of SEQ ID NO: 3.

### C. Transmembrane domain

The antigen-specific extracellular domain and the intracellular signaling-domain may be linked by a transmembrane domain. Polypeptide sequences that can be used as part of transmemebrane domain include, without limitation, the human CD4 transmembrane domain, the human CD28 transmembrane domain, the transmembrane human CD3ζ domain, or a cysteine mutated human CD3ζ domain, or other transmembrane domains from other human transmembrane signaling proteins, such as CD16 and CD8 and erythropoietin receptor. In some aspects, for example, the transmembrane domain comprises one of the sequences provided in U.S. Patent Publication No. 2014/0274909 or U.S. Patent No. 8,906,682, both incorporated herein by reference. In certain specific aspects, the transmembrane domain can be 85%, 90%, 91%, 92%, 93%, 94%, 95%, 96%, 97%, 98%, 99% or 100% identical to a CD8 α transmembrane domain of SEQ ID NO: 19 or a CD28 transmembrane domain of SEQ ID NO: 12.

### D. Intracellular signaling domain

The intracellular signaling domain of the chimeric antigen receptor of the embodiments is responsible for activation of at least one of the normal effector functions of the immune cell engineered to express a chimeric antigen receptor. The term "effector function" refers to a specialized function of a differentiated cell. Effector function of a T cell, for example, may be cytolytic activity or helper activity including the secretion of cytokines. Effector function in a naive, memory, or memory-type T cell includes antigen-dependent proliferation. Thus the term "intracellular signaling domain" refers to the portion of a protein that transduces the effector function signal and directs the cell to perform a specialized function. In some aspects, the intracellular signaling domain is derived from the intracellular signaling domain of a native receptor. Examples of such native receptors include the zeta chain of the T-cell receptor or any of its homologs (*e.g*., eta, delta, gamma, or epsilon), MB1 chain, B29, Fc RIII, Fc RI, and combinations of signaling molecules, such as CD3ζ and CD28, CD27, 4-1BB, DAP-10, OX40, and combinations thereof, as well as other similar molecules and fragments. Intracellular signaling portions of other members of the families of activating proteins can be used, such as FcγRIII and FcεRI. See Gross *et al.* (1992), Stancovski *et al.* (1993), Moritz *et al.* (1994), Hwu *et al.* (1995), Weijtens *et al.* (1996), and Hekele *et al.* (1996) for disclosures of T-cell receptors using these alternative transmembrane and intracellular domains. While usually the entire intracellular signaling domain will be employed, in many cases it will not be necessary to use the entire intracellular polypeptide. To the extent that a truncated portion of the intracellular signaling domain may find use, such truncated portion may be used in place of the intact chain as long as it still transduces the effector function signal. The term "intracellular signaling domain" is thus meant to include a truncated portion of the intracellular signaling domain sufficient to transduce the effector function signal, upon CAR binding to a target. In a preferred embodiment, the human CD3ζ intracellular domain is used as the intracellular signaling domain for a CAR of the embodiments.

In specific embodiments, intracellular receptor signaling domains in the CAR include those of the T cell antigen receptor complex, such as the ζ chain of CD3, also Fcγ RIII costimulatory signaling domains, CD28, CD27, DAP10, CD137, OX40, CD2, alone or in a series with CD3ζ, for example. In specific embodiments, the intracellular domain (which may be referred to as the cytoplasmic domain) comprises part or all of one or more of TCRζ chain, CD28, CD27, OX40/CD134, 4-1BB/CD137, FcεRIγ, ICOS/CD278, IL-2Rβ/CD122, IL-2Rα/CD132, DAP10, DAP12, and CD40. In some embodiments, one employs any part of the endogenous T cell receptor complex in the intracellular domain. One or multiple cytoplasmic domains may be employed, as so-called third generation CARs have at least two or three signaling domains fused together for additive or synergistic effect, for example.

In some embodiments, the CAR comprises additional other costimulatory domains. Other costimulatory domains can include, but are not limited to one or more of CD28, CD27, OX-40 (CD134), DAP10, and 4-1BB (CD137). In addition to a primary signal initiated by CD3ζ, an additional signal provided by a human costimulatory receptor inserted in a human CAR is important for full activation of T cells and could help improve *in vivo* persistence and the therapeutic success of the adoptive immunotherapy.

In certain specific aspects, the intracellular signaling domain comprises a sequence 85%, 90%, 91%, 92%, 93%, 94%, 95%, 96%, 97%, 98%, 99% or 100% identical to a CD3ζ intracellular domain of SEQ ID NO: 13, a CD28 intracellular domain of SEQ ID NO: 17 or a CD137 intracellular domain of SEQ ID NO: 15.

### III. Vectors and Cell engineering

In particular embodiments, isolated nucleic acid segments and expression cassettes incorporating DNA sequences that encode transgenes are provided. For example, the transgene can encode a CAR polypeptide. In further aspects, a transgene encodes a target antigen (or an epitope thereof) and/or a HLA polypeptide. In further aspects, a transgene encodes mitochondrial reporter transgene, such a reporter polypeptide (*e.g*., a fluorescent reporter) comprising a mitochondria localization signal..

As will be appreciated by one of skill in the art that, in some instances, the coding sequence for a few amino acids at the ends of an encoded transgene may be deleted. For example, in the case of a transgene encoding a CAR, the coding sequence for a few amino acids of the antigen binding domain in the CAR can be deleted without affecting either specificity or effector binding affinity of the molecules, usually not more than 10, more usually not more than 5 residues, for example. Also, it may be desirable to introduce a small number of amino acids at the borders of a transgene coding sequence, usually not more than 10, more usually not more than 5 residues. The deletion or insertion of amino acids may be as a result of the needs of the construction, providing for convenient restriction sites, ease of manipulation, improvement in levels of expression, or the like. In addition, the substitute of one or more amino acids with a different amino acid can occur for similar reasons, usually not substituting more than about 5 amino acids in a transgene coding sequence (e.g., in any domain of a CAR).

The transgenic constructs according to the embodiments can be prepared in conventional ways. Because, for the most part, natural sequences may be employed, the natural genes may be isolated and manipulated, as appropriate, so as to allow for the proper joining of the various components. For example, in the case of a CAR, the nucleic acid sequences encoding for the N-terminal and C-terminal protein components of the chimeric antigen receptor can be isolated by employing the polymerase chain reaction (PCR), using appropriate primers that result in deletion of the undesired portions of the gene. Alternatively, restriction digests of cloned genes can be used to generate the chimeric construct. In either case, the sequences can be selected to provide for restriction sites that are blunt-ended, or have complementary overlaps.

The various manipulations for preparing the transgenic construct can be carried out *in vitro* and in particular embodiments the chimeric construct is introduced into vectors for cloning and expression in an appropriate host using standard transformation or transfection methods. Thus, after each manipulation, the resulting construct from joining of the DNA sequences is cloned, the vector isolated, and the sequence screened to ensure that the sequence encodes the desired transgene (*e.g*., a chimeric antigen receptor) and expression control sequences. The sequence can be screened by restriction analysis, sequencing, or the like.

Vectors of the embodiments designed, primarily, to deliver desired genes to immune cells, preferably immune effector cells (e.g., T cells) or APCs, under the control of regulated eukaryotic promoters. Promoters that can be used according to the embodiments include, for example, MNDU3 promoter, CMV promoter, EF1α promoter, or Ubiquitin promoter. Also, the vectors may contain a selectable marker, if for no other reason, to facilitate their manipulation *in vitro.* In other embodiments, the transgene (e.g., a CAR) can be expressed from mRNA *in vitro* transcribed from a DNA template.

In an exemplary nucleic acid construct (polynucleotide) employed according to the embodiments, the promoter is operably linked to the nucleic acid sequence encoding a transgene of the embodiments, *i.e.*, they are positioned so as to promote transcription of the messenger RNA from the DNA encoding the transgene. The promoter can be of genomic origin or synthetically generated. A variety of promoters for use in T cells are well-known in the art (*e.g*., the CD4 promoter disclosed by Marodon *et al.* (2003)). The promoter can be constitutive or inducible, where induction is associated with the specific cell type or a specific level of maturation, for example. Alternatively, a number of well-known viral promoters are also suitable. Promoters of interest include the β-actin promoter, SV40 early and late promoters, immunoglobulin promoter, human cytomegalovirus promoter, retrovirus promoter, and the Friend spleen focus-forming virus promoter. The promoters may or may not be associated with enhancers, wherein the enhancers may be naturally associated with the particular promoter or associated with a different promoter.

The sequence of the open reading frame encoding the transgene can be obtained from a genomic DNA source, a cDNA source, or can be synthesized (*e.g*., *via* PCR), or combinations thereof. Depending upon the size of the genomic DNA and the number of introns, it may be desirable to use cDNA or a combination thereof as it is found that introns stabilize the mRNA or provide T cell-specific expression (Barthel and Goldfeld, 2003). Also, it may be further advantageous to use endogenous or exogenous non-coding regions to stabilize the mRNA.

For expression of a transgene of the embodiments, the naturally occurring or endogenous transcriptional initiation region of the nucleic acid sequence encoding transgene can be used to generate the chimeric antigen receptor in the target host. Alternatively, an exogenous transcriptional initiation region can be used that allows for constitutive or inducible expression (*e.g*., a tet-on or tet-off promoter system), wherein expression can be controlled depending upon the target host, the level of expression desired, the nature of the target host, and the like.

Likewise, in some cases, a signal sequence directing the polypeptide encoded by the transgene to the cell surface may be used. In some cases, the signal sequence is the signal sequence present in the native version of a transgene. Optionally, in some instances, it may be desirable to exchange this sequence for a different signal sequence. However, the signal sequence selected should be compatible with the secretory pathway of the cell used for expression of the transgene (*e.g*., T cells) so that the transgene is presented on the surface of the cell.

Similarly, a termination region may be provided by the naturally occurring or endogenous transcriptional termination region for the native version of the transgene. Alternatively, the termination region may be derived from a different source. For the most part, the source of the termination region is generally not considered to be critical to the expression of a recombinant protein and a wide variety of termination regions can be employed without adversely affecting expression.

It is contemplated that transgenes constructs, such as CAR expression constructs, can be introduced into the subject's own cells (or into cells from a different donor subject) as naked DNA or in a suitable vector. Methods of stably transfecting cells, such as T cells, by electroporation using naked DNA are known in the art. See, for example, U.S. Pat. No. 6,410,319, incorporated herein by reference. Naked DNA generally refers to the DNA encoding a transgene (e.g., chimeric antigen receptor) of the present embodiments contained in a plasmid expression vector in proper orientation for expression. Advantageously, the use of naked DNA can reduce the time required to produce cells expressing the transgene of the embodiments.

In further aspects, transgene constructs can be introduced into cells using a transposon-based system to mediate integration of the transgene (e.g., CAR) construct into genomic DNA of the cells. Generally, such methods will involve introducing into cells (i) a first vector encoding the transposase (or a transposase polypeptide) and (ii) a second vector encoding a desired transgene expression element that is flanked by transposon repeats. Transposons or transposable elements include a (short) nucleic acid sequence with terminal repeat sequences upstream and downstream thereof and encode enzymes that facilitate the excision and insertion of the nucleic acid into target DNA sequences. Several transposon/transposase systems have been adapted for genetic insertions of heterologous DNA sequences, including *Sleeping Beauty (SB)*, a Tc1/mariner-like element from fish that exhibits transpositional activity in a variety of vertebrate cultured cell lines, embryonic stem cells and *in vivo* (Ivics *et al*., 1997). Additional transposases and transposon systems are provided in U.S. Patent Nos. 6,489,458; 7,148,203; 8,227,432; U.S. Patent Publn. No. 2011/0117072; Mates et al., 2009 and in Ivics *et al*., 1997, each of which are incorporated herein by reference in their entirety.

Alternatively, a viral vector (*e.g*., a retroviral vector, adenoviral vector, adeno-associated viral vector, or lentiviral vector) can be used to introduce the transgenes into cells. Suitable vectors for use in accordance with the method of the embodiments are non-replicating in the subject's cells. A large number of vectors are known that are based on viruses, where the copy number of the virus maintained in the cell is low enough to maintain the viability of the cell. Illustrative vectors include the pFB-neo vectors (STRATAGENE®) disclosed herein as well as vectors based on HIV, SV40, EBV, HSV, or BPV.

### IV. Immune Effector Cells

In certain aspects, the embodiments described herein include methods of making and/or expanding the antigen-specific redirected immune effector cells (*e.g*., T-cells, NK-cell or NK T-cells) that comprises transfecting the cells with an expression vector containing a DNA (or RNA) construct encoding the CAR, then, optionally, stimulating the cells with feeder cells, recombinant antigen, or an antibody to the receptor to cause the cells to proliferate. In certain aspects, the cell (or cell population) engineered to express a CAR is a stem cell, iPS cell, immune cell or a precursor of these cells. Methods described below address the specific example of T-cells (or other immune cell) engineering for CAR expression.

Sources of immune effector cells include both allogeneic and autologous sources. In some cases immune effector cells may be differentiated from stem cells or induced pluripotent stem cells (iPSCs). Thus, cell for engineering according to the embodiments can be isolated from umbilical cord blood, peripheral blood, human embryonic stem cells, or iPSCs. For example, allogeneic T cells can be modified to include a chimeric antigen receptor (and optionally, to lack functional TCR). In some aspects, the immune effector cells are primary human T cells, such as T cells derived from human peripheral blood mononuclear cells (PBMC), PBMC collected after stimulation with G-CSF, bone marrow, or umbilical cord blood. Following transfection or transduction (*e.g*., with a CAR expression construct), the cells may be immediately infused or may be stored. In certain aspects, following transfection, the cells may be propagated for days, weeks, or months *ex vivo* as a bulk population within about 1, 2, 3, 4, 5 days or more following gene transfer into cells. In a further aspect, following transfection, the transfectants are cloned and a clone demonstrating presence of a single integrated or episomally maintained expression cassette or plasmid, and expression of the chimeric antigen receptor is expanded *ex vivo.* The clone selected for expansion demonstrates the capacity to specifically recognize and lyse antigen-expressing target cells. The recombinant T cells may be expanded by stimulation with IL-2, or other cytokines that bind the common gamma-chain (*e.g*., IL-7, IL-12, IL-15, IL-21, and others). The recombinant T cells may be expanded by stimulation with artificial antigen presenting cells. The recombinant T cells may be expanded on artificial antigen presenting cell or with an antibody, such as OKT3, which cross links CD3 on the T cell surface. Subsets of the recombinant T cells may be deleted on artificial antigen presenting cell or with an antibody, such as Campath, which binds CD52 on the T cell surface. In a further aspect, the genetically modified cells may be cryopreserved.

In further aspects, immune effector cells of the embodiment have been selected for high mitochondrial spare respiratory capacity (SRC). As used herein an "immune effector cell having high mitochondrial SRC" refers to an immune effector cell (*e.g*., a T-cell) having higher mitochondria activity or mitochondria number than a corresponding average immune effector cell (*e.g*., a T-cell). Thus, in some aspects, a cell composition of the embodiments comprises a population of immune effector cells having high mitochondrial SRC, for example a population of CAR-expressing T-cell having high mitochondrial SRC.

Immune effector cells, such as CD8⁺ T cells, with high mitochondrial SRC may exhibit enhanced survival relative to cells with lower SRC during stress conditions, such as high tumor burden, hypoxia, lack of nutrients for glycolysis, or a suppressive cytokine milieu. Moreover, immune effector cells selected for high mitochondrial SRC may retain cytotoxic activity, even under stress conditions. Accordingly, by selecting immune effector cells with high mitochondrial SRC improved cell composition for both therapy and for testing of CAR constructs can be produced.

In one aspects, transgenic immune effector cells are provided that comprise a reporter that can be used to determine the mitochondrial SRC of the transgenic effector cells. For example, transgenic cells may comprise a reporter polypeptide that is linked to a mitochondria localization signal. For example, the reporter can be a fluorescent polypeptide such an enhanced Yellow Fluorescence Protein (YFP) or an enhanced Green Fluorescence Protein (EGFP) and the mitochondria localization signal can be from glutaredoxin (Grx2). In this context the fluorescence reporter identifies CAR+ T cells with high mitochondrial SRC. For example, the transgenic cells expressing the reporter can be sorted based on intensity fluorescence and infused for tumor killing in vivo. Likewise, the transgenic cells could be tested for ex vivo killing of target cells to determine, for example, the therapeutic effectiveness of a candidate CAR polypeptide.

In some aspects, the mitochondrial reporter gene for use according to the embodiments may be an endogenous gene. In further aspects, the mitochondrial reporter gene may be an exogenous gene, such as a gene encoding a fluorescent reporter protein. In some aspects, the fluorescent reporter protein may comprise a mitochondrial localization sequence. In certain aspects, a method for selecting immune effector cells having high SRC may comprise flow cytometry or FACS.

In certain aspects, expression of the reporter gene for identifying immune effector cells with SRC may be under the control of a nuclear promoter (*e.g*., hEF1a). In certain aspects, expression of the reporter gene may be under the control of a mitochondrial promoter. In certain aspects, the expressed reporter protein may comprise a mitochondrial localization sequence. In certain aspects, the expressed reporter protein may be directed to the cell surface. In certain aspects, expression of the reporter gene may be under the control of a mitochondrial promoter and the expressed reporter protein may be directed to the cell surface. In some aspects, an exogenous reporter gene may be flanked by a transposon repeat or a viral LTR. In some aspects, an exogenous reporter gene may be comprised in an extrachromosomal nucleic acid, such as an mRNA or an episomal vector.

### V. Method for Propagating Immune Effector Cells

In some cases, immune effector cells of the embodiments (*e.g*., T-cells) are co-cultured with activating and propagating cells (AaPCs), to aid in cell expansion. For example, antigen presenting cells (APCs) are useful in preparing therapeutic compositions and cell therapy products of the embodiments. For general guidance regarding the preparation and use of antigen-presenting systems, see, *e.g*., U.S. Pat. Nos. 6,225,042, 6,355,479, 6,362,001 and 6,790,662; U.S. Patent Application Publication Nos. 2009/0017000 and 2009/0004142; and International Publication No. WO2007/103009, each of which is incorporated by reference.

In some cases, AaPCs are incubated with a peptide of an optimal length that allows for direct binding of the peptide to the MHC molecule without additional processing. Alternatively, the cells can express an antigen of interest (*i.e*., in the case of MHC-independent antigen recognition). Furthermore, in some cases, APCs can express an antibody that binds to either a specific CAR polypeptide or to CAR polypeptides in general (*e.g*., a universal activating and propagating cell (uAPC). Such methods are disclosed in International (PCT) Patent Pub. no. WO/2014/190273, which is incorporated herein by reference. In addition to peptide-MHC molecules or antigens of interest, the AaPC systems may also comprise at least one exogenous assisting molecule. Any suitable number and combination of assisting molecules may be employed. The assisting molecule may be selected from assisting molecules such as co-stimulatory molecules and adhesion molecules. Exemplary co-stimulatory molecules include CD70 and B7.1 (B7.1 was previously known as B7 and also known as CD80), which among other things, bind to CD28 and/or CTLA-4 molecules on the surface of T cells, thereby affecting, for example, T-cell expansion, Th1 differentiation, short-term T-cell survival, and cytokine secretion such as interleukin (IL)-2 (see Kim *et al*., 2004). Adhesion molecules may include carbohydrate-binding glycoproteins such as selectins, transmembrane binding glycoproteins such as integrins, calcium-dependent proteins such as cadherins, and single-pass transmembrane immunoglobulin (Ig) superfamily proteins, such as intercellular adhesion molecules (ICAMs), that promote, for example, cell-to-cell or cell-to-matrix contact. Exemplary adhesion molecules include LFA-3 and ICAMs, such as ICAM-1. Techniques, methods, and reagents useful for selection, cloning, preparation, and expression of exemplary assisting molecules, including co-stimulatory molecules and adhesion molecules, are exemplified in, *e.g*., U.S. Pat. Nos. 6,225,042, 6,355,479, and 6,362,001, incorporated herein by reference.

Cells selected to become AaPCs, preferably have deficiencies in intracellular antigen-processing, intracellular peptide trafficking, and/or intracellular MHC Class I or Class II molecule-peptide loading, or are poikilothermic (*i.e*., less sensitive to temperature challenge than mammalian cell lines), or possess both deficiencies and poikilothermic properties. Preferably, cells selected to become AaPCs also lack the ability to express at least one endogenous counterpart (*e.g*., endogenous MHC Class I or Class II molecule and/or endogenous assisting molecules as described above) to the exogenous MHC Class I or Class II molecule and assisting molecule components that are introduced into the cells. Furthermore, AaPCs preferably retain the deficiencies and poikilothermic properties that were possessed by the cells prior to their modification to generate the AaPCs. Exemplary AaPCs either constitute or are derived from a transporter associated with antigen processing (TAP)-deficient cell line, such as an insect cell line. An exemplary poikilothermic insect cells line is a Drosophila cell line, such as a Schneider 2 cell line (see, *e.g*., Schneider 1972 Illustrative methods for the preparation, growth, and culture of Schneider 2 cells, are provided in U.S. Pat. Nos. 6,225,042, 6,355,479, and 6,362,001.

In one embodiment, AaPCs are also subjected to a freeze-thaw cycle. In an exemplary freeze-thaw cycle, the AaPCs may be frozen by contacting a suitable receptacle containing the AaPCs with an appropriate amount of liquid nitrogen, solid carbon dioxide (*i.e*., dry ice), or similar low-temperature material, such that freezing occurs rapidly. The frozen APCs are then thawed, either by removal of the AaPCs from the low-temperature material and exposure to ambient room temperature conditions, or by a facilitated thawing process in which a lukewarm water bath or warm hand is employed to facilitate a shorter thawing time. Additionally, AaPCs may be frozen and stored for an extended period of time prior to thawing. Frozen AaPCs may also be thawed and then lyophilized before further use. Preferably, preservatives that might detrimentally impact the freeze-thaw procedures, such as dimethyl sulfoxide (DMSO), polyethylene glycols (PEGs), and other preservatives, are absent from media containing AaPCs that undergo the freeze-thaw cycle, or are essentially removed, such as by transfer of AaPCs to media that is essentially devoid of such preservatives.

In further embodiments, xenogenic nucleic acid and nucleic acid endogenous to the AaPCs, may be inactivated by crosslinking, so that essentially no cell growth, replication or expression of nucleic acid occurs after the inactivation. In one embodiment, AaPCs are inactivated at a point subsequent to the expression of exogenous MHC and assisting molecules, presentation of such molecules on the surface of the AaPCs, and loading of presented MHC molecules with selected peptide or peptides. Accordingly, such inactivated and selected peptide loaded AaPCs, while rendered essentially incapable of proliferating or replicating, retain selected peptide presentation function. Preferably, the crosslinking also yields AaPCs that are essentially free of contaminating microorganisms, such as bacteria and viruses, without substantially decreasing the antigen-presenting cell function of the AaPCs. Thus crosslinking maintains the important AaPC functions of while helping to alleviate concerns about safety of a cell therapy product developed using the AaPCs. For methods related to crosslinking and AaPCs, see for example, U.S. Patent Application Publication No. 20090017000, which is incorporated herein by reference.

In certain cases, CAR modified cells can be sorted based on their mitochondrial strength (or total mitochondria content of the cells) by employing a fluorescent reporter protein using FACS prior to use as a therapeutic.

### VI. Engineered Antigen Presenting Cells

In certain embodiments there are further provided an engineered antigen presenting cell (APC). Such cells may be used, for example, as described above, to propagate immune effector cells ex vivo. In further aspects, engineered ACPs may, themselves be administered to a patient and thereby stimulate expansion of immune effector cells *in vivo.* Engineered APCs of the embodiments may, themselves, be used as a therapeutic agent. In some embodiments, the engineered APCs can be used as a therapeutic agent that can stimulate activation of endogenous immune effector cells specific for a target antigen and/or to increase the activity or persistence of adoptively transferred immune effector cells specific to a target antigen.

As used herein the term "engineered APC" refers to a cell(s) that comprises at least a first transgene encoding a human leukocyte antigen (HLA). Suvch engineered APCs may further comprise a second transgene for expression of an antigen, such that the antigen is presented at the surface on the APC in complex with the HLA. In some aspects, the engineered APC can be a cell type that presented antigens (e.g., a dendritic cell). In further aspects, engineered APC can be produced from a cell type that does not normally present antigens, such a T-cell or T-cell progenitor (referred to as "T-APC"). Thus, in some aspects, an engineered APC of the embodiments comprises a first transgene encoding a target antigen and a second transgene encoding a HLA, such that the HLA is expressed on the surface of the engineered APC in complex with an epitope of the target antigen. In certain specific aspects, the HLA expressed in the engineered APC is a HLA-A, HLA-B, HLA-C or HLA-DRB1. In further aspects, the HLA expressed in the engineered APC is HLA-A2.

In some aspects, an engineered APC of the embodiments may further comprise at least a third transgene encoding co-stimulatory molecule. The co-stimulatory molecule may be a co-stimulatory cytokine that may be a membrane-bound Cγ cytokine. In certain aspects, the co-stimulatory cytokine is IL-15, such as membrane-bound IL-15. In some further aspects, an engineered APC may comprise an edited (or deleted) gene. For example, an inhibitory gene, such as PD-1, LIM-3, CTLA-4 or a TCR, can be edited to reduce or eliminate expression of the gene.

An engineered APC of the embodiments may comprise a transgene encoding any target antigen of interest. For example, the target antigen can be an infectious disease antigen or a tumor-associated antigen (TAA). Target antigens may be intracellular or cell surface antigens. For example, in some aspects, the target antigen is a TAA such as a TAA derived from a subcellular compartment of the tumor cell. The TAA may be membrane-bound, cytoplasmic, nuclear-localized, or even secreted by the tumor cells. In some aspects, the TAA is differentially expressed compared to the corresponding normal tissue thereby allowing for a preferential recognition of tumor cells by immune effector cells. Specific examples of target antigens include, without limitation, WT1, MUC1, LMP2, HPV E6 E7, EGFRvIII, HER-2/neu, Idiotype, MAGE A3, p53 nonmutant, NY-ESO-1, PSMA, GD2, CEA, MelanA/MART1, Ras mutant, gp100, p53 mutant, Proteinases (PR1), bcr-abl, Tyrosinase, Survivin, PSA, hTERT, Sarcoma translocation breakpoints, EphA2, PAP, ML-IAP, AFP, EpCAM, ERG (TMPRSS2 ETS fusion gene), NA17, PAX3, ALK, Androgen receptor, Cyclin B1, Polysialic acid, MCN, RhoC, TRP-2, GD3, Fucosyl GM1, Mesothelin, PSCA, MAGE A1, sLe(a), CYP1B1, PLAC1, GM3, BORIS, Tn, GloboH, ETV6-AML, NY-BR-1, RGS5, SART3, STn, Carbonic anhydrase IX, PAX5, OT-TES1, Sperm protein 17, LCK, HMWMAA, AKAP-4, SSX2, XAGE 1, B7H3, Legumain, Tie 2, Page4, VEGFR2, MAD-CT-1, FAP, PDGFR-β, MAD-CT-2, and Fos-related antigen 1.

In some specific aspects, an engineered APC of the embodiments is a T cell that has been engineered to function as antigen presenting cells (referred to as a "T - APC"). In particular, a T-APC of the embodiments comprises a first transgene encoding a target antigen and a second transgene encoding a HLA. Thus, the T-APC can present the encoded antigen, such as a TAA. For example, T-APCs exemplified herein comprise a transgene encoding the NY -ES0-1 antigen and HLA-A2. Thus, these cells may be used to propagate NY-ESO-1-specific immune effector cells either *ex vivo* or *in vivo* (after being administered to a patient). Moreover, T-APCs exemplified herein were further engineered to express an additional of co-stimulatory molecule, specifically membrane-bound IL-15 (miL-15). The additional co-stimulatory molecule further improves the generation of target antigen specific immune effector cells and increases the persistence of these cells.

### VII. Therapeutic application

In some aspects, the chimeric antigen receptor constructs and cells of the embodiments find application in subjects having or suspected of having cancer by reducing the size of a tumor or preventing the growth or re-growth of a tumor in these subjects. Accordingly, embodiments of provided herein further relate to a method for reducing growth or preventing tumor formation in a subject by introducing a chimeric antigen receptor construct of the present embodiments into an isolated T cell of the subject and reintroducing into the subject the transformed T cell, thereby effecting anti-tumor responses to reduce or eliminate tumors in the subject. Suitable T cells that can be used include cytotoxic lymphocytes (CTL) or any cell having a T cell receptor in need of disruption. As is well-known to one of skill in the art, various methods are readily available for isolating these cells from a subject. For example, using cell surface marker expression or using commercially available kits (*e.g*., ISOCELL™ from Pierce, Rockford, Ill.).

Once it is established that the transfected or transduced immune effector cell (*e.g*., T cell) is capable of expressing the chimeric antigen receptor as a surface membrane protein with the desired regulation and at a desired level, it can be determined whether the chimeric antigen receptor is functional in the host cell to provide for the desired signal induction. Subsequently, the transduced immune effector cells are reintroduced or administered to the subject to activate anti-tumor responses in the subject. To facilitate administration, the transduced T cells according to the embodiments can be made into a pharmaceutical composition or made into an implant appropriate for administration *in vivo,* with appropriate carriers or diluents, which further can be pharmaceutically acceptable. The means of making such a composition or an implant have been described in the art (see, for instance, Remington's Pharmaceutical Sciences, 16th Ed., Mack, ed. (1980)). Where appropriate, the transduced T cells can be formulated into a preparation in semisolid or liquid form, such as a capsule, solution, injection, inhalant, or aerosol, in the usual ways for their respective route of administration. Means known in the art can be utilized to prevent or minimize release and absorption of the composition until it reaches the target tissue or organ, or to ensure timed-release of the composition. Desirably, however, a pharmaceutically acceptable form is employed that does not ineffectuate the cells expressing the chimeric antigen receptor. Thus, desirably the transduced T cells can be made into a pharmaceutical composition containing a balanced salt solution, preferably Hanks' balanced salt solution, or normal saline.

In certain embodiments, CAR-expressing cells of the embodiments are delivered to an individual in need thereof, such as an individual that has cancer or an infection. The cells then enhance the individual's immune system to attack the respective cancer or pathogen-infected cells. In some cases, the individual is provided with one or more doses of the antigen-specific CAR cells. In cases where the individual is provided with two or more doses of the antigen-specific CAR cells, the duration between the administrations should be sufficient to allow time for propagation in the individual, and in specific embodiments the duration between doses is 1, 2, 3, 4, 5, 6, 7, or more days. Suitable doses for a therapeutic effect would be at least 10⁵ or between about 10⁵ and about 10¹⁰ cells per dose, for example, preferably in a series of dosing cycles. An exemplary dosing regimen consists of four one-week dosing cycles of escalating doses, starting at least at about 10⁵ cells on Day 0, for example increasing incrementally up to a target dose of about 10¹⁰ cells within several weeks of initiating an intra-patient dose escalation scheme. Suitable modes of administration include intravenous, subcutaneous, intracavitary (for example by reservoir-access device), intraperitoneal, and direct injection into a tumor mass.

A pharmaceutical composition of the embodiments (*e.g*., comprising CAR-expressing T-cells) can be used alone or in combination with other well-established agents useful for treating cancer. Whether delivered alone or in combination with other agents, the pharmaceutical composition of the embodiments can be delivered *via* various routes and to various sites in a mammalian, particularly human, body to achieve a particular effect. One skilled in the art will recognize that, although more than one route can be used for administration, a particular route can provide a more immediate and more effective reaction than another route. For example, intradermal delivery may be used for the treatment of melanoma. Local or systemic delivery can be accomplished by administration comprising application or instillation of the formulation into body cavities, inhalation or insufflation of an aerosol, or by parenteral introduction, comprising intramuscular, intravenous, intraportal, intrahepatic, peritoneal, subcutaneous, or intradermal administration.

A composition of the embodiments can be provided in unit dosage form wherein each dosage unit, *e.g*., an injection, contains a predetermined amount of the composition, alone or in appropriate combination with other active agents. The term unit dosage form as used herein refers to physically discrete units suitable as unitary dosages for human and animal subjects, each unit containing a predetermined quantity of the composition of the embodiments, alone or in combination with other active agents, calculated in an amount sufficient to produce the desired effect, in association with a pharmaceutically acceptable diluent, carrier, or vehicle, where appropriate. The specifications for the novel unit dosage forms of the embodiments depend on the particular pharmacodynamics associated with the pharmaceutical composition in the particular subject.

Desirably an effective amount or sufficient number of the isolated transduced T cells is present in the composition and introduced into the subject such that long-term, specific, anti-tumor responses are established to reduce the size of a tumor or eliminate tumor growth or regrowth than would otherwise result in the absence of such treatment. Desirably, the amount of transduced T cells reintroduced into the subject causes a 10%, 20%, 30%, 40%, 50%, 60%, 70%, 80%, 90%, 95%, 98%, or 100% decrease in tumor size when compared to otherwise same conditions wherein the transduced T cells are not present. As used herein the term "anti-tumor effective amount" refers to an effective amount of CAR-expressing immune effector cells to reduce cancer cell or tumor growth in a subject.

Accordingly, the amount of transduced immune effector cells (*e.g*., T cells) administered should take into account the route of administration and should be such that a sufficient number of the transduced immune effector cells will be introduced so as to achieve the desired therapeutic response. Furthermore, the amounts of each active agent included in the compositions described herein (*e.g*., the amount per each cell to be contacted or the amount per certain body weight) can vary in different applications. In general, the concentration of transduced T cells desirably should be sufficient to provide in the subject being treated at least from about 1 × 10⁶ to about 1 × 10⁹ transduced T cells, even more desirably, from about 1 × 10⁷ to about 5 × 10⁸ transduced T cells, although any suitable amount can be utilized either above, *e.g*., greater than 5 × 10⁸ cells, or below, *e.g*., less than 1 × 10⁷ cells. The dosing schedule can be based on well-established cell-based therapies (see, *e.g*., Topalian and Rosenberg, 1987; U.S. Pat. No. 4,690,915), or an alternate continuous infusion strategy can be employed.

These values provide general guidance of the range of transduced T cells to be utilized by the practitioner upon optimizing the method of the embodiments. The recitation herein of such ranges by no means precludes the use of a higher or lower amount of a component, as might be warranted in a particular application. For example, the actual dose and schedule can vary depending on whether the compositions are administered in combination with other pharmaceutical compositions, or depending on interindividual differences in pharmacokinetics, drug disposition, and metabolism. One skilled in the art readily can make any necessary adjustments in accordance with the exigencies of the particular situation.

### VIL Kits of the Embodiments

Any of the compositions described herein may be comprised in a kit. In some embodiments, allogeneic CAR T-cells are provided in the kit, which also may include reagents suitable for expanding the cells, such as media, APCs, engineered APCs, growth factors, antibodies (*e.g*., for sorting or characterizing CAR T-cells) and/or plasmids encoding transgenes, such as a target antigen, HLA, mitochondrial reporter, CAR or transposase.

In a non-limiting example, a chimeric antigen receptor expression construct, one or more reagents to generate a chimeric antigen receptor expression construct, cells for transfection of the expression construct, and/or one or more instruments to obtain allogeneic cells for transfection of the expression construct (such an instrument may be a syringe, pipette, forceps, and/or any such medically approved apparatus).

In some embodiments, an expression construct for eliminating endogenous TCR α/β expression, one or more reagents to generate the construct, and/or CAR+ T cells are provided in the kit. In some embodiments, there includes expression constructs that encode zinc finger nuclease(s).

In some aspects, the kit comprises reagents or apparatuses for electroporation of cells.

The kits may comprise one or more suitably aliquoted compositions of the embodiments or reagents to generate compositions of the embodiments. The components of the kits may be packaged either in aqueous media or in lyophilized form. The container means of the kits may include at least one vial, test tube, flask, bottle, syringe, or other container means, into which a component may be placed, and preferably, suitably aliquoted. Where there is more than one component in the kit, the kit also will generally contain a second, third, or other additional container into which the additional components may be separately placed. However, various combinations of components may be comprised in a vial. The kits of the embodiments also will typically include a means for containing the chimeric antigen receptor construct and any other reagent containers in close confinement for commercial sale. Such containers may include injection or blow molded plastic containers into which the desired vials are retained, for example.

### VIII. Examples

The embodiments of the invention are further described in detail by reference to the following examples. These examples are provided for the purpose of illustration only, and are not intended to be limiting unless otherwise specified. Thus, the invention should in no way be construed as being limited to the following examples, but rather, should be construed to encompass any and all variations which become evident as a result of the teaching provided herein.

### Example 1 - Binding of CAR by Fc receptors is associated with rapid CAR T-cell clearance

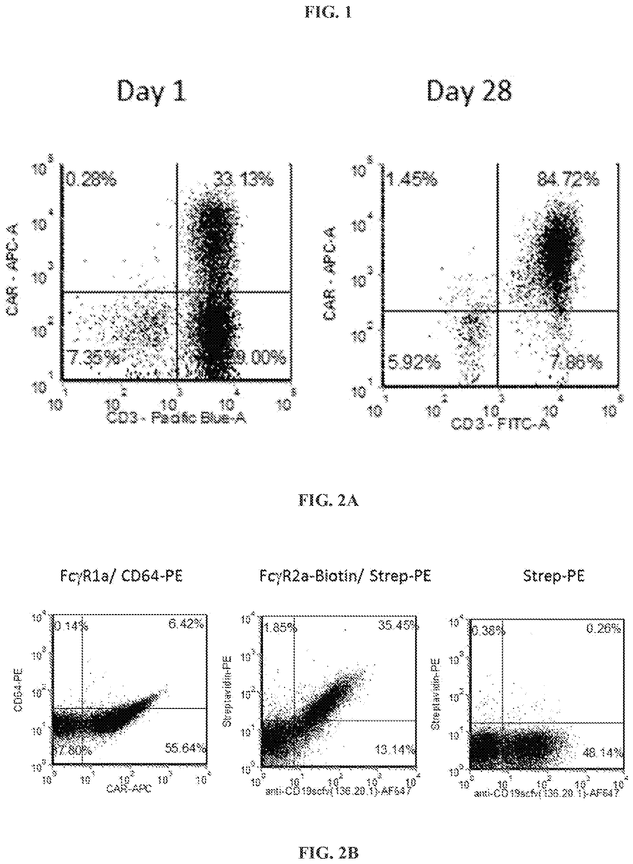

The role of Fc receptor binding to CAR polypeptides in CAR T-cell persistence was investigated. For the initial studies, CAR constructs targeted to the CD19 receptor were studied. The CAR T-cells expressed the CD19RCD28 construct, which includes a CD19 binding domain (VL/VH); a hinge domain (IgG4-Fc); a transmembrane domain (CD28 TM) and an intracellular signaling domain (CD28- CD3ζ) (see FIG. 1). CAR constructs were introduced into cells via electroporation, using a Sleeping Beauty-based transposon system to mediate genomic integration of the constructs (see, e.g., International (PCT) Application No. PCT/US14/38005, incorporated herein by reference). CAR T-cells produced were then analyzed by flow cytometry to assess CAR polypeptide expression. The CAR polypeptide was found to efficiently expressed post transfection and a high proportion of the resulting cell populations were positive for CAR expression following 28 days of co-culture with K562 aAPCs (FIG. 2A). Flow cytometry studies also showed that a proportion of the CAR T-cells were bound by FcγR2a *in vitro,* indicating that the binding of the Fc receptor likely effects CASR T-cells *in vivo* and could play a role in persistence CAR T-cells.

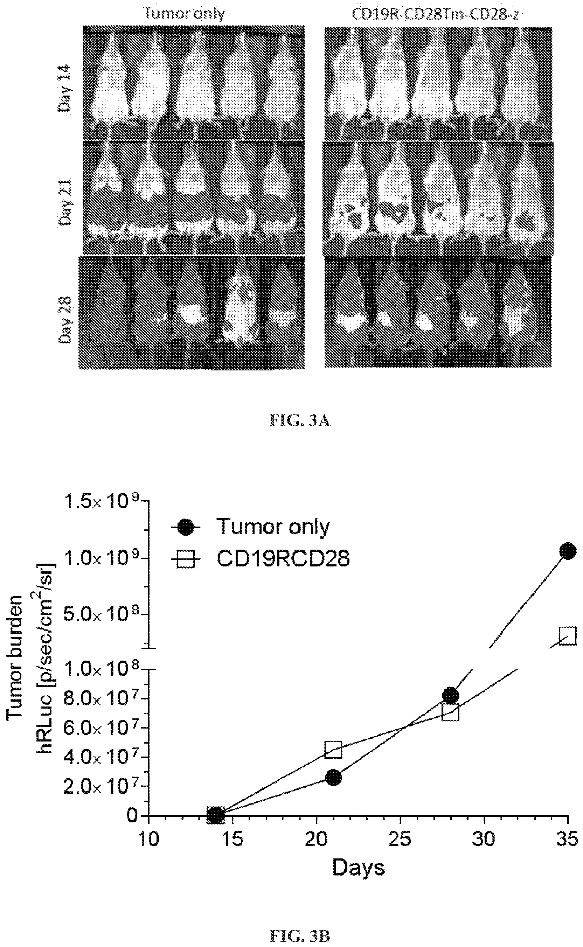

Studies were also completed in mice bearing CD19 positive tumors. Briefly, NSG mice were injected with *Renilla* Luciferase (hRLuc)⁺ NALM6 tumor cells followed by a single injection of CD19RCD28 T cells (or control injection). Bioluminescence associated with tumor cells was measured *via* imaging over time. As shown in FIG. 3A-B, while the CAR T-cells are initially able to control tumor growth (day 21 time point), by day 28 post injection tumor out-growth is clearly evident even in the treated animals. This effect may be associated with limited *in vivo* persistence of the T-cells *e.g*., due to FcR binding. Thus, by including a Fc receptor binding elements a CAR polypeptides *in vivo* persistence (and potential toxicity) could be limited.

### Example 2 -CAR polypeptides with reduced Fc receptor binding

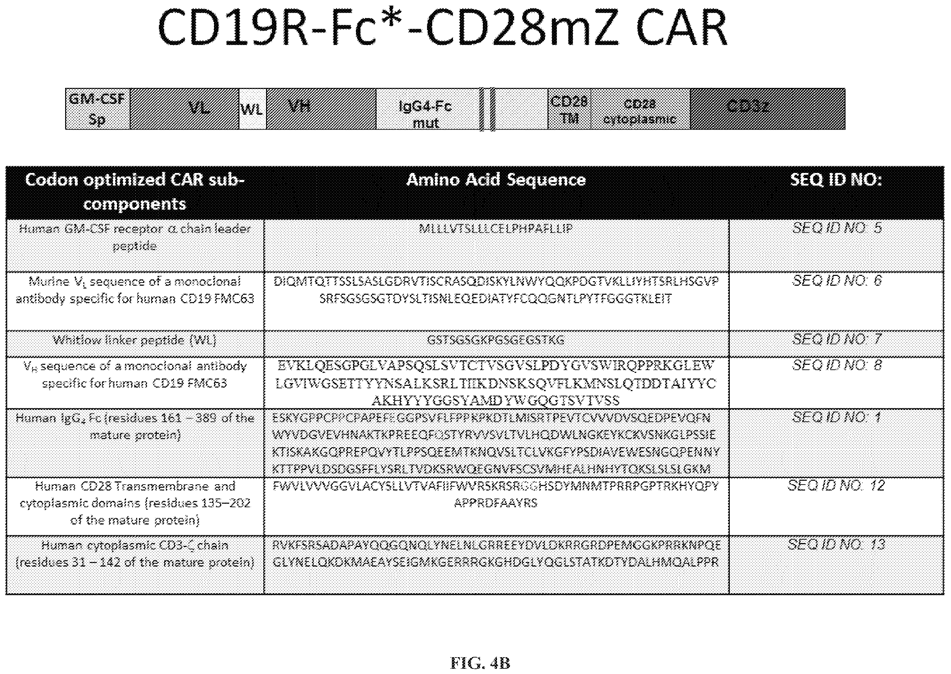

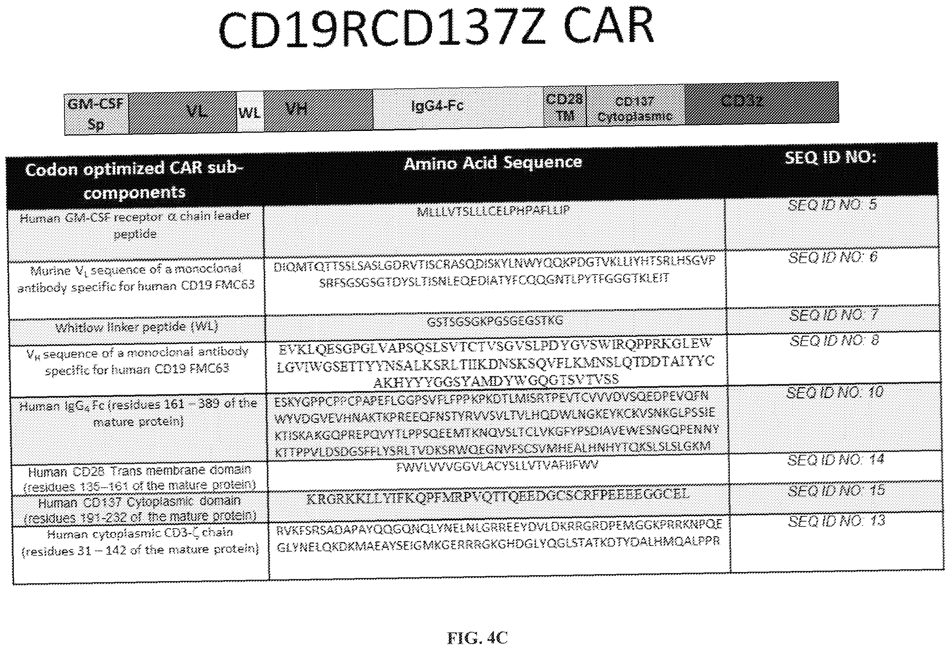

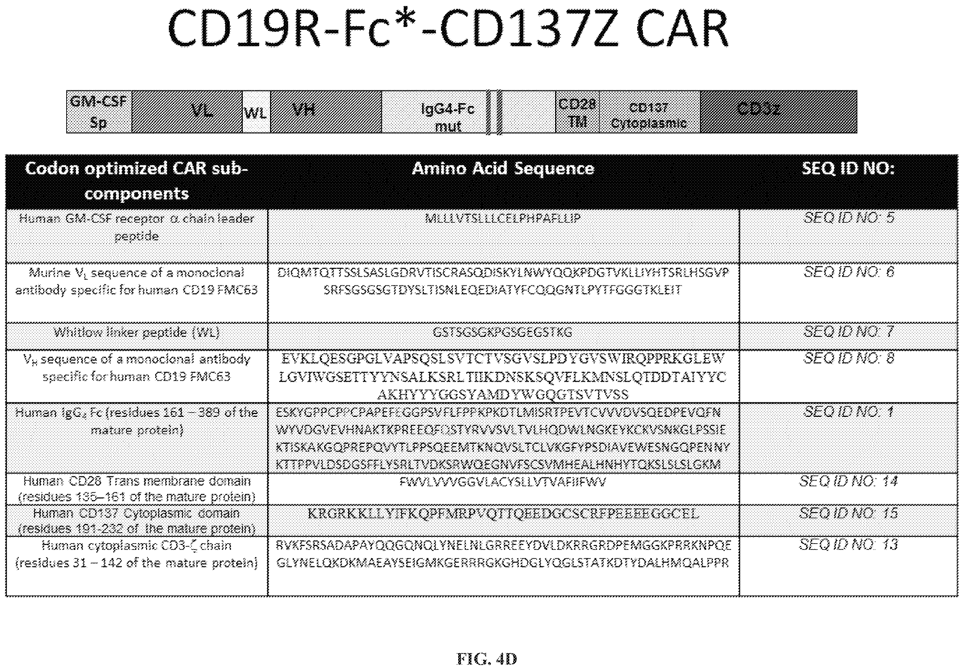

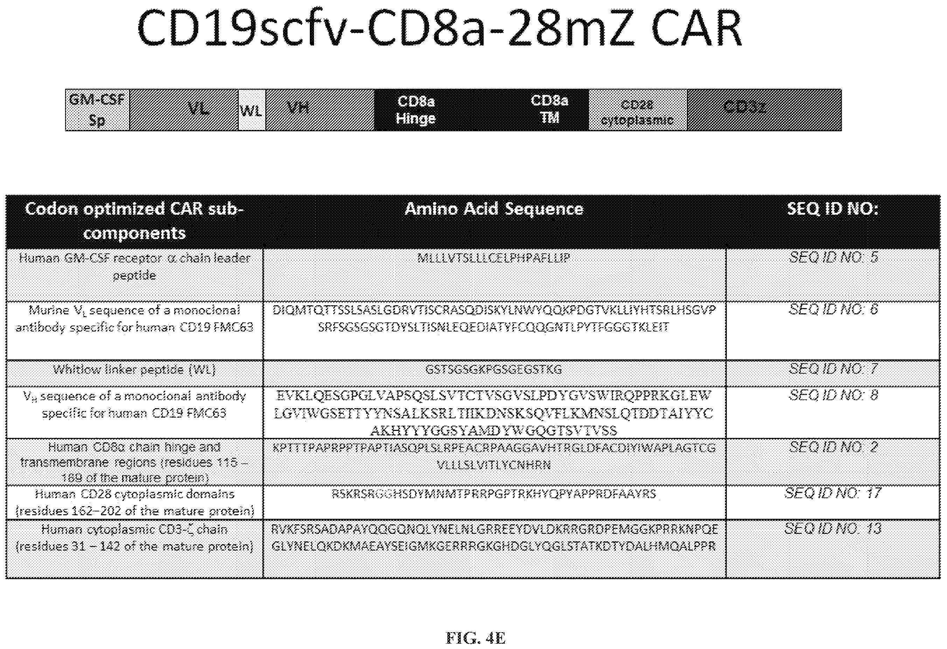

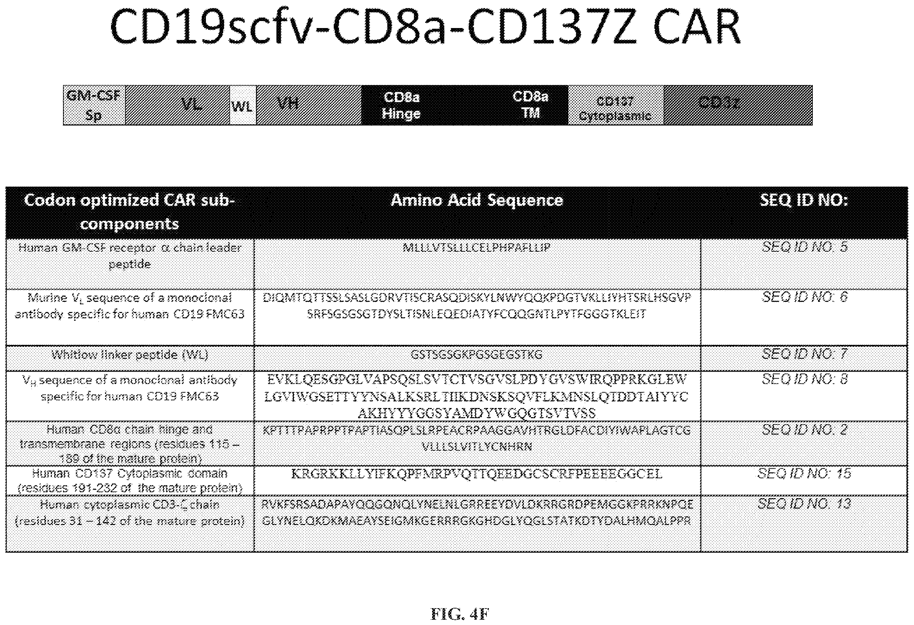

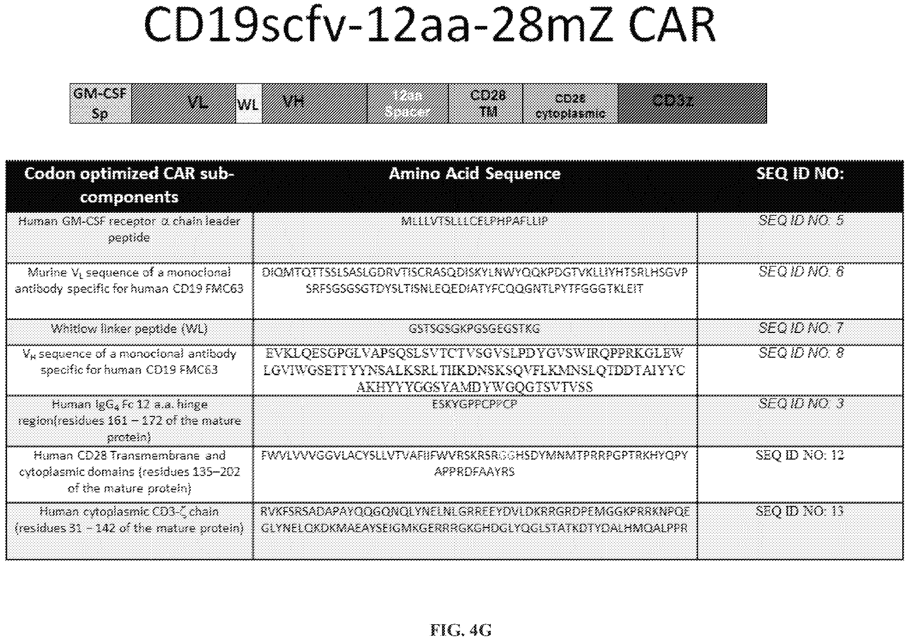

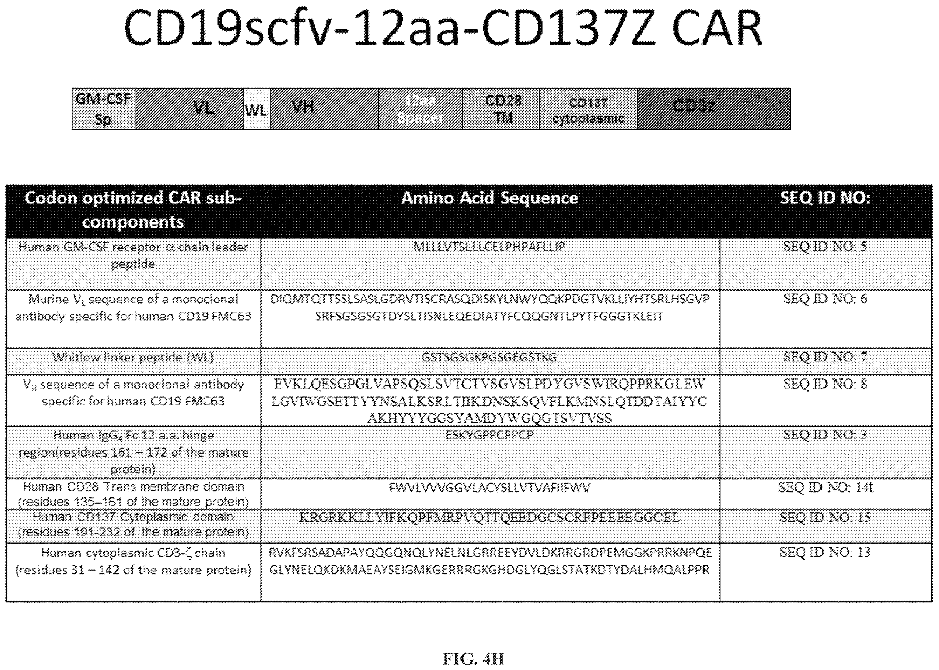

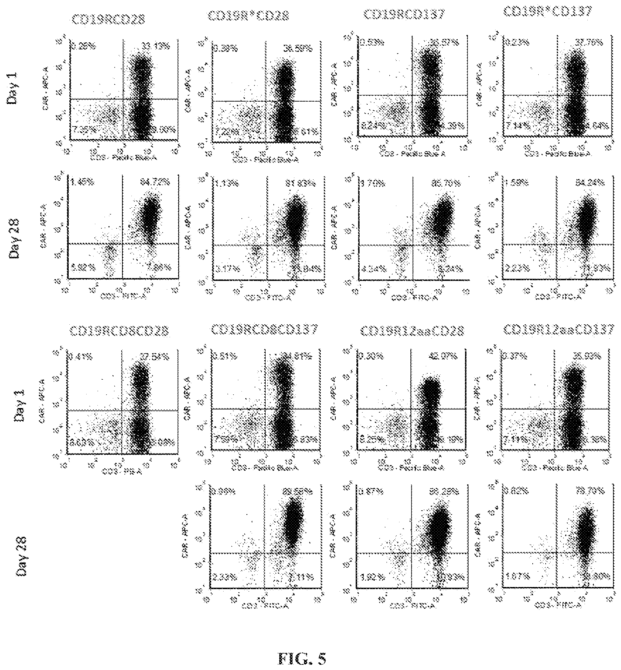

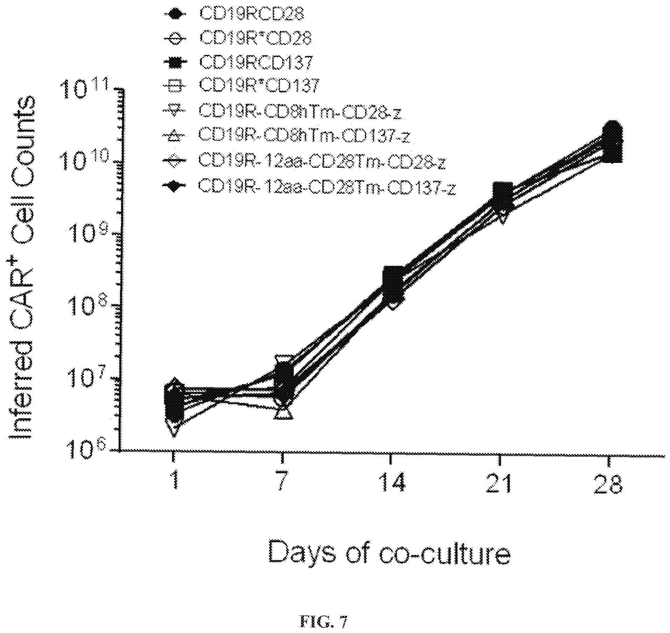

An array of different CAR T-cell constructs was produced to modulate Fc receptor binding to CAR T-cells. Constructs tested are shown in FIG. 4. In particular, different hinge sequences were tested in the context of a number of different transmembrane and intracellular/signaling domains. Flow cytometry studies presented in FIG. 5 show that all tested constructs were efficiently expressed in transfected T-cells (transfections were completed as above by electroporation using a Sleeping Beauty-based transposon system for integration of the CAR receptor constructs). Likewise, a high proportion of resulting T-cells were CAR receptor positive after 28 days of co-culture with K562 aAPCs (FIG. 5). The kinetics of CAR T-cell expansion in co-culture with K562 aAPCs is shown in FIG. 7 and demonstrates that all CAR T-cells expanded with similar efficiency.

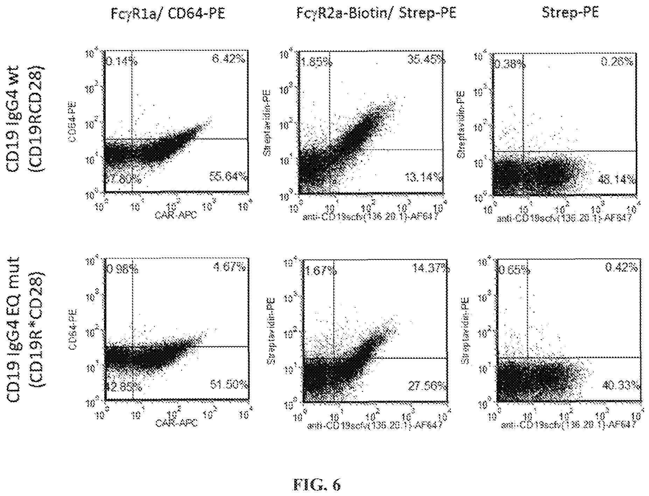

Next, CAR T-cells were tested for *in vitro* Fc receptor binding by flow cytometry. As shown in FIG. 6, while CAR T-cells including the human IgG4 hinge sequence were bound by Fc receptor (by FcγR2a in particular), cells expressing a mutant IgG4 hinge, having L235E and N297Q substitutions, showed significantly less binding to Fc receptors.

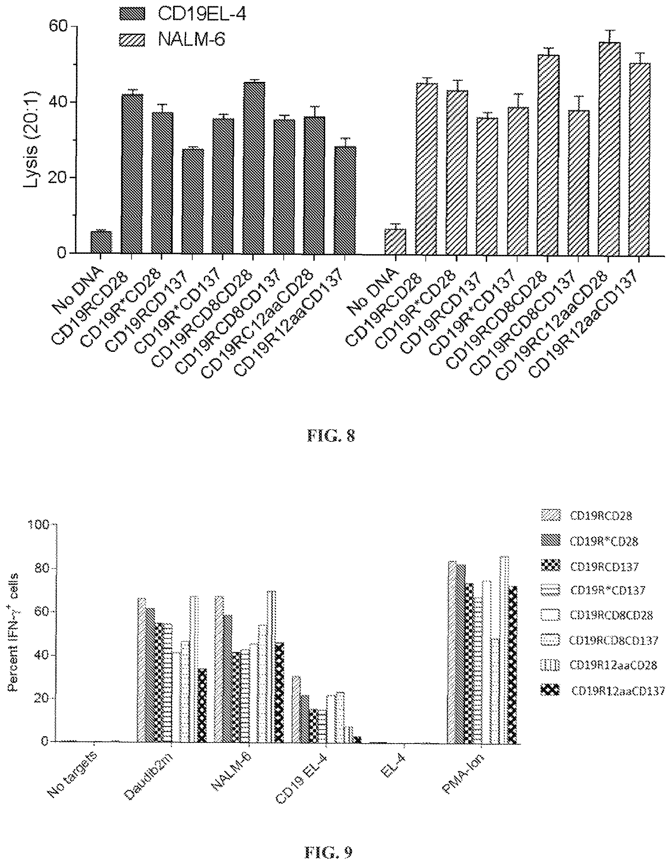

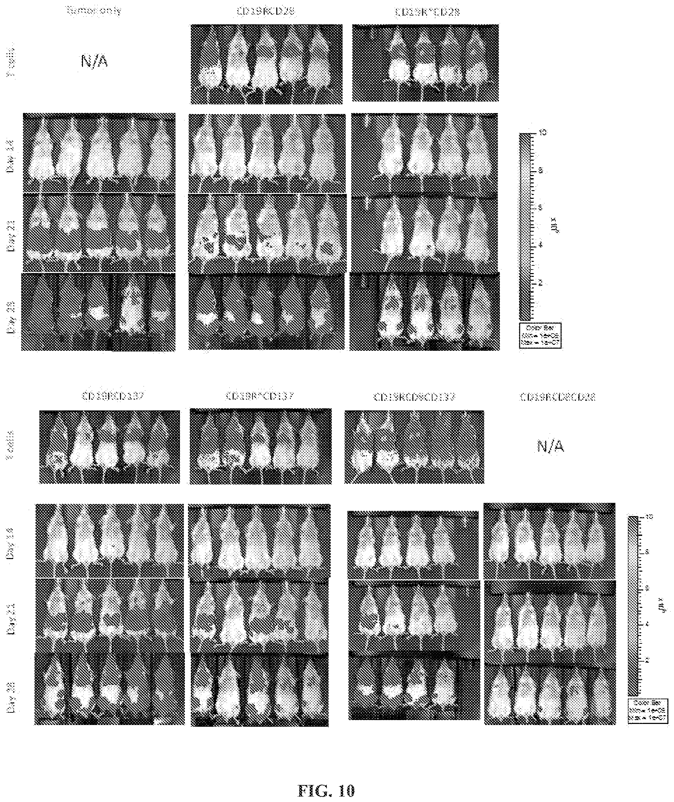

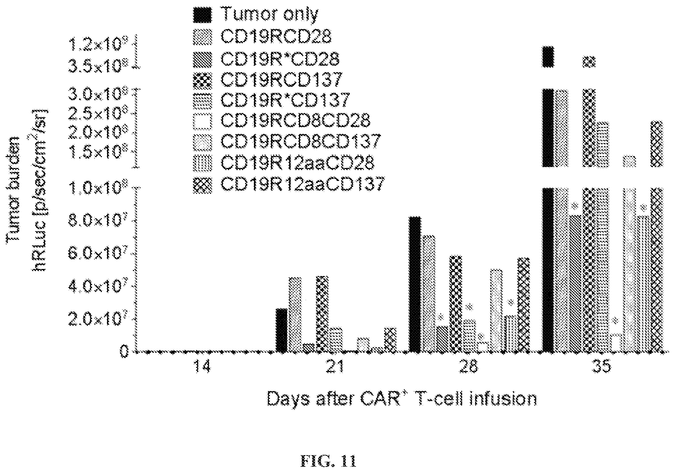

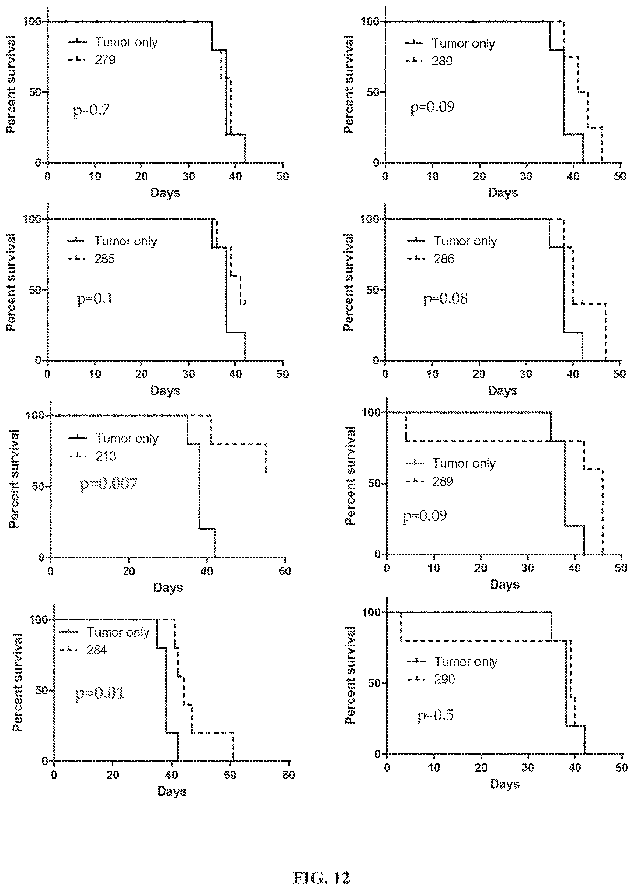

CAR T-cells expressing each of the tested CAR constructs were then assessed for the ability to target CD19 positive tumor cells. In studies presented in FIGs. 8-9, the ability of CAR T-cells to lyse target cells (FIG. 8) or produce IFNγ in response to target cells (FIG. 9) was tested in *ex vivo* experiments. These studies were followed by *in vivo* experiments to determine the efficacy of the various CAR T-cells to control explanted tumor out-growth in mice. Briefly, as above, NSG mice that were injected (i.v.) with tumor (hRLuc+ NALM-6) followed by injection (i.c.) with *P. pyralis* luciferase (ffLuc)+ CAR+ T-cells the next day. Tumor and T-cell imaging was performed after injection of EnduRen or Luciferin respectively using Xenogen® IVIS 100 series system. Imaging of the mice is shown in FIG. 10. In each case, T-cells were imaged immediately after administration to confirm intracardiac injection (top row). Shaded images represent photon flux from tumor-derived hRLuc activity over time. Luminescence data from these studies was also graphed and is shown in FIG. 11. Finally, FIG. 12 shows a graph depicting survival curves for NSG mice treated with various CAR T-cells. The results of these studies shown that each of the CAR T-cells expressing constructs that reduce Fc receptor binding (*i.e*., the EQ mutant IgG4 hinge, the CD8a hinge or the 12-aa spacer hinge) had greater efficacy as compared to CAR T-cells bearing a wild type IgG4 hinge sequence. In particular, these constructs showed both improved control of tumor cell growth in mice and lengthened survival time of treated animals. Thus, these studies indicate that, by reducing Fc receptor binding, *in vivo* persistence and anti-tumor efficacy of the CAR T-cells can be significantly enhanced.

### Example 3 - Additional CAR construct studies

Additional studies were conducted using CAR constructs targeted to the CD19 receptor. Similar to the initial studies, PBMCs were electroporated with CD19R^{∗}CD28 or CD19RCD8CD28 transposon & SB11 transposase and co-cultured on irradiated Clone 1 along with IL-2 and IL-21 for 28 days in a 7-day stimulation cycle. Cells were enumerated and phenotyped (CD3, CAR) at the end of each stimulation cycle (FIGS. 13A-D).

These studies were followed by *in vivo* experiments to determine the efficacy of the various CAR T-cells against CD19⁺ NALM-6 cell line in NSG (NOD.Cg-*Prkdc^{scid} Il2rg^{tm1Wjl}*/SzJ*,* NOD *scid* gamma) mice. Both a minimal residual disease (MRD) model and an established tumor model were studied. In the MRD model, NSG mice were injected with 10⁴ cells of hRluc⁺mKate⁴NALM-6 on day 0, followed by injection of T-cells (10⁷/mouse) intra-cardiacally on day 1. Tumor burden was evaluated by bioluminescent imaging derived from hRluc⁺NALM-6 using EnduRen Live Cell Substrate over time (FIG. 14A). In the established tumor model, NSG mice were injected with 1.5x10⁴ ffLuc⁺EGFP⁺NALM-6 on day 0 and tumor was allowed to engraft for 5 days, tumor burden was evaluated and T cells (10⁷/mouse) injected intracardiacally on day 6. Tumor flux was calculated using bioluminescent imaging from ffLuc⁺NALM-6 using D-Luciferin substrate over time. False-color images representing photon flux and tumor-flux over time is shown (FIG. 14C). Luminescence data from these studies was also graphed and is shown in FIGS. 14B and 14D, for each model respectively.

### Example 4 - The effect of mIL-15 co-expression CAR T-cell efficacy and persistence

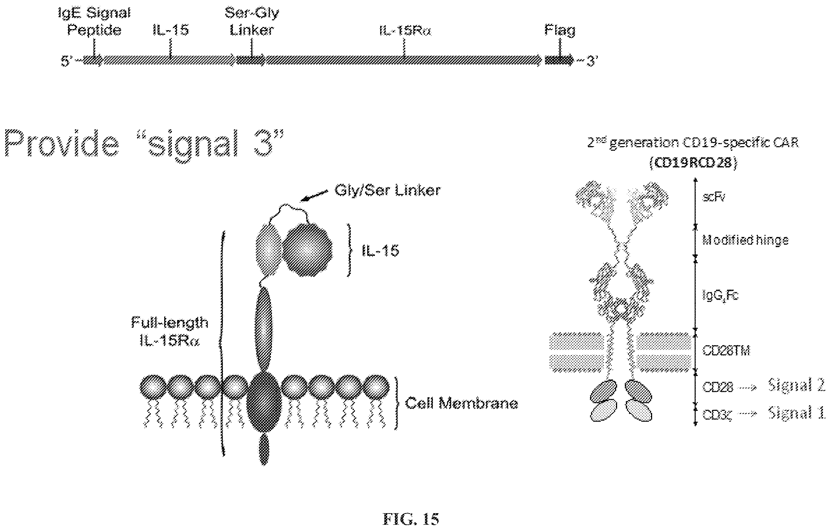

Following CAR T-cell infusions in either an autologous or allogenic mouse models, levels of cytokines IL-2, IL-4, IL-7, IL-9 and IL-15 were monitored. Additional signaling constructs were explored to improve persistence. FIG. 15 shows a membrane-bound IL-15/IL-15 receptor (mIL15) construct The mIL-15 construct was co-expressed with CAR in T-cells. Data in FIG. 16 (see, *e.g*., left panels) demonstrate co-expression of CAR and mIL15 on the vast majority of elecroporated cells. Further data presented in FIG. 16 shows mIL-15, when co-expressed with CAR was able to protect CAR T-cells from decline in culture following antigen withdrawal (see FIG. 16, upper right panel).

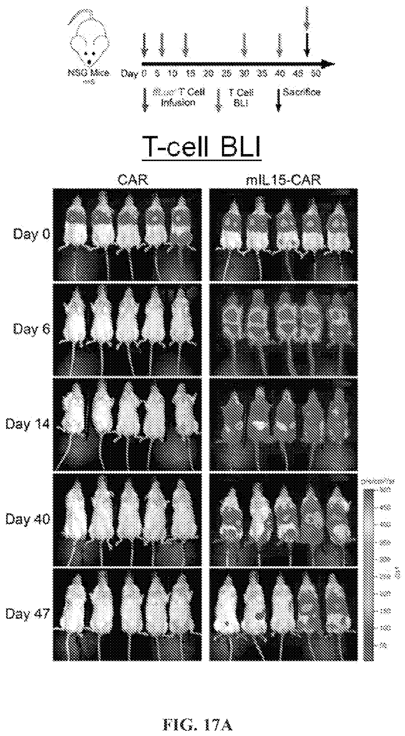

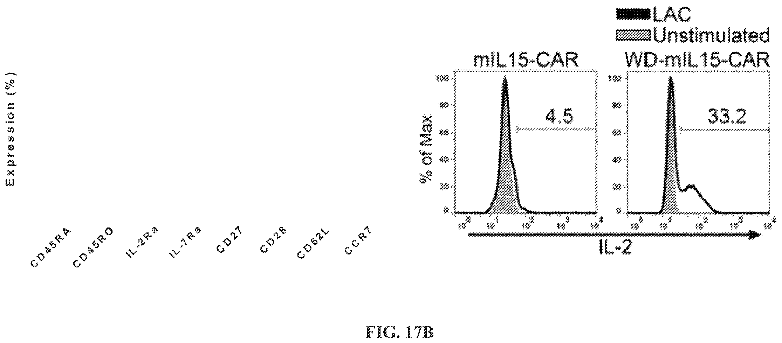

Cell culture results indicating enhanced persistence of CAR-mIL-15 T-cells were next confirmed *in vivo.* For these studies, mice were injected with ffLuc+ T cells either expressing CAR alone or CAR in combination with mIL-15. T cell imaging was performed after injection of Luciferin. As shown in FIG. 17B, mIL-15-CAR T-cells exhibited extended persistence *in vivo.* Further *in vivo* studies examined the effectiveness of CAR T-cells expressing mIL-15 in controlling tumor growth. These studies were completed using an established tumor model and genetically-modified T cells were injected (i.c.), as indicated, after 6 days of tumor engraftment (injected i.v.).

### Example 5 - Genetic modification of T cells

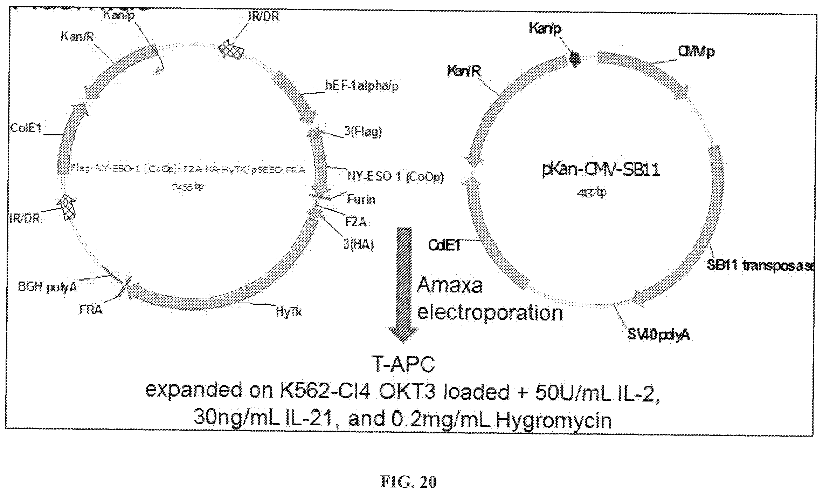

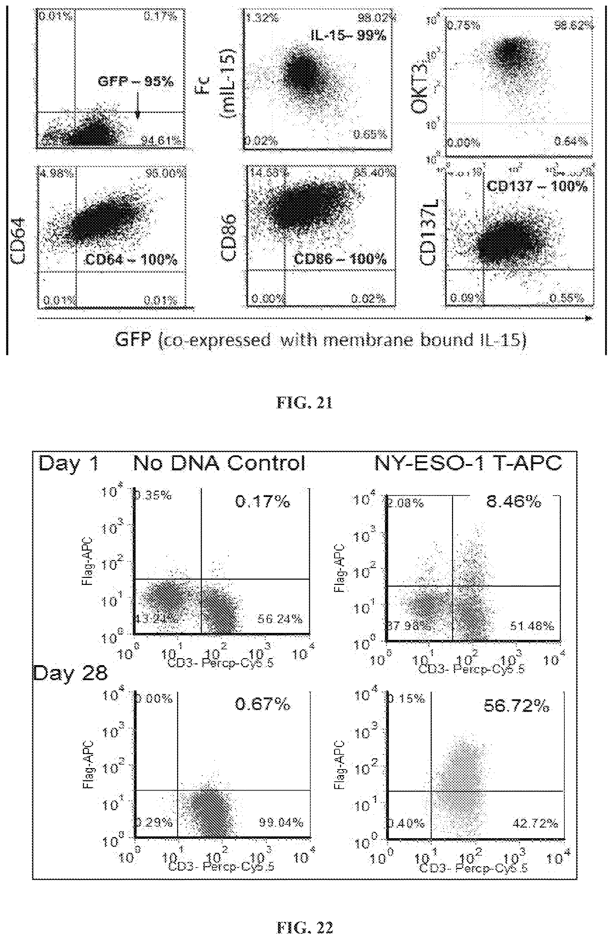

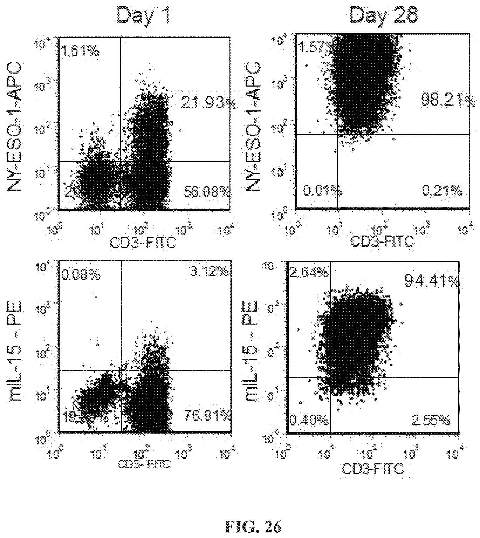

T cells were propagated *ex vivo* to function as antigen presenting cells (T-APC) to present the tumor associated antigen (TAA) NY-ESO-1. The *Sleeping Beauty* (SB) system. (Hackett *et al*., 2010) was adapted to genetically modify the T cells (FIG. 20) (Cooper *et al.,* 2005). This involved two steps: (i) the electro-transfer of DNA plasmids expressing a SB transposon [i.e., chimeric antigen receptor (CAR) to redirect T-cell specificity (Jin *et al*., 2011; Kebriaei *et al*., 2012)] and SB transposase and (ii) the propagation and expansion of T cells stably expressing integrants on designer artificial antigen-presenting cells (aAPC) derived from the K562 cell line that co-expresses CD64 (Fc receptor), CD86, CD137L and a membrane-bound mutein of interleukin (IL)-15 (mIL-15, FIG. 21). To enforce the expression of the NY-ESO-1 immunogen, the T-APC in culture were selected with hygromycin, as the plasmid SB expression cassette co-espresses a fusion of hygromycin phosphotransferase (Hy) with thymidine kinase (HyTK) (FIG. 20). The genetically modified T cells were propagated by serial stimulations with γ-irradiated OKT3-loaded aAPC in presence of a cytocidal concentration of hygromycin B (FIG. 20). T cells can function as T-APC *in vitro* and *in vivo,* using the viral antigen influenza A MP1 (Cooper *et al.,* 2005). In this example, T cells were engineered to express NY-ESO-1 (fused to FLAG-tagged HyTK) (FIG. 22) and NY-ESO-1⁺mIL-15⁺HLA-A2⁺T-APC propagated in the presence of artificial antigen presenting cells (aAPC) (FIG. 26) using the SB system (Hackett *et al.,* 2010).

### Example 6 - Evaluation of engineered T cells

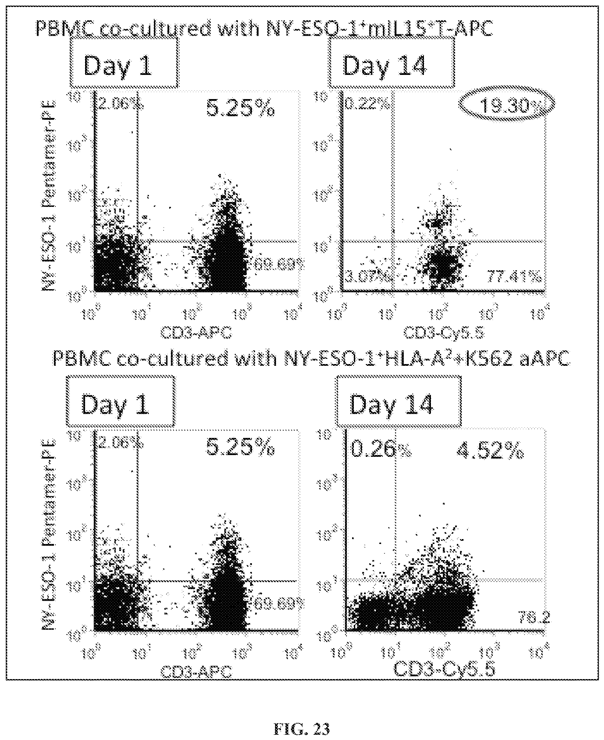

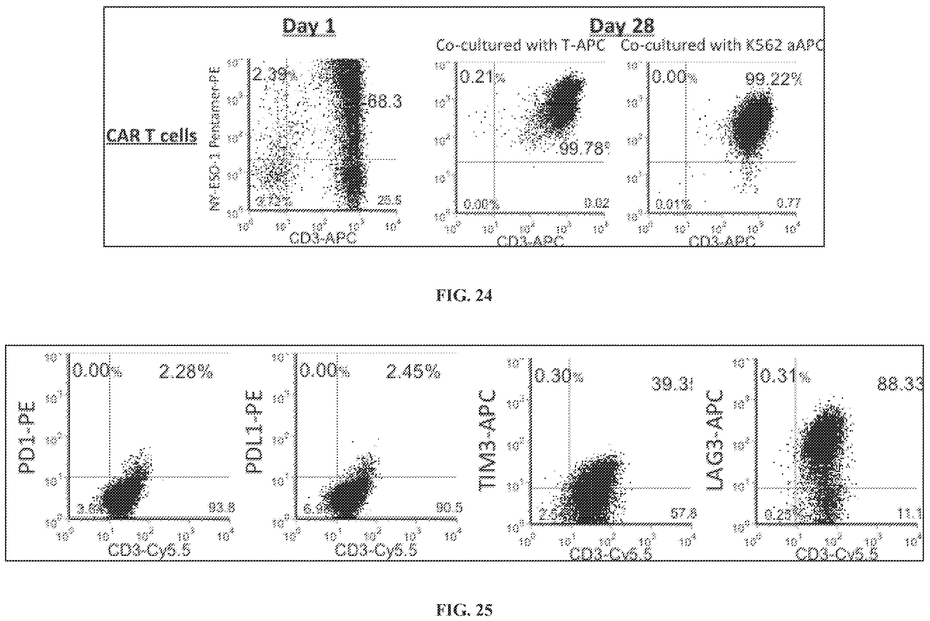

mIL15⁺NY-ESO-I⁺T-APC of the embodiments described herein are able to generate NY-ESO-1 pentamer positive T cells when co-cultured with PBMC and are able to expand NY-ESO-1⁺ CAR T cells which are capable of killing myeloma cells *in vitro* (FIGS. 23 and 24). However, the absence of CD137L activation from the T-APC as well as increased inhibition from LAG3 expression may have led to the diminished responses observed after 3 weeks in co-culture (FIG. 25). LAG3 expression increased with each stimulation of the T-APC and may be a surrogate for exhaustion.

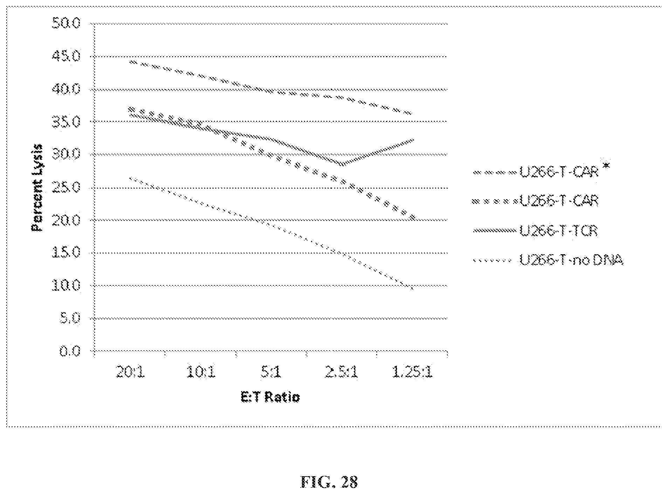

FIG. 28 further illustrates these results showing the killing of NY-ESO-1⁺ U266 human multiple myeloma cell line by NY-ESO-1 specific TCR⁺ and CAR⁺ T cells. The T-cells were expanded on artificial activating and propagating cells (AaPC) derived from K562 and expresses HLA-A02 and NY-ESO-1⁺. The data shown represent two independent experiments where the NY-ESO-1 specific CAR T-cells of 9-2-15 where propagated for 3 stimulations with HLA-A2/NY-ESO-1 T-APC and 2 stimulations with AaPC K562 derived. The 9-2-15 CAR contains the CD8α stalk while the previous one contained the IgG4 stalk. The increased stimulations resulted in increased lysis of myeloma cells.

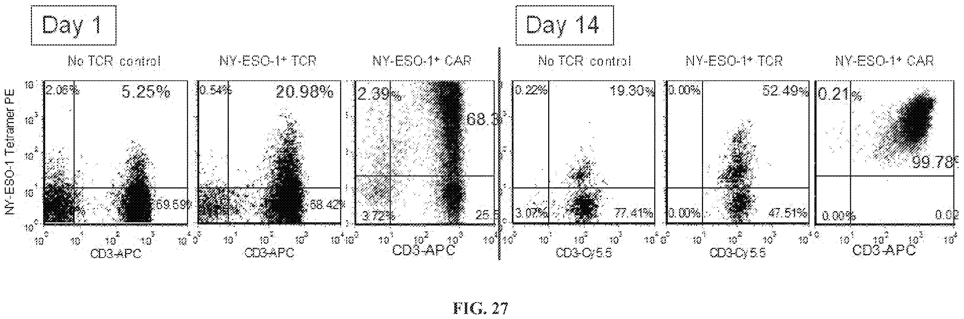

The NY-ESO-1⁺mIL-15⁺HLA-A2⁺T-APC were also evaluated for their ability to present the TAA. This was achieved by co-culture of PBMC with autologous T cells that had been *a priori* genetically modified to express an NY-ESO-1 specific αβTCR. The T-APCs were used to successfully numerically expand NY-ESO-1 pentamer⁺ T cells. Similarly, K562-derived aAPCs were also modified to present NY-ESO-1 and used to selectively activate and propagate NY-ESO-1-specific genetically modified T cells as a positive control. This positive control was unable to induce immunity in PBMC as the T-APC were able to do. Redirecting the specificity of T cells using a chimeric antigen receptor (CAR) that recognizes NY-ESO-1-derived peptide was also studied in the context of HLA. The T-APC were successfully able to expand the CAR T cells as well as the positive control, the K562-derived aAPC (FIG. 27).

### Example 7 - Determination of SRC/mitochondrial strength

All cells (both cancerous and primary) have the ability to switch their mitochondria from an orthodox configuration to a condensed state and *vice versa* in response to available metabolites (Krauss *et al*., 2001). The mitochondrial shape is different in CAR-modified T cells as compared to mock-electroporated control T cells grown in similar *ex vivo* culture conditions (FIGs. 29A and 29B). This observation that certain CAR-modified T cells have condensed state mitochondria became striking when comparing T cells modified with a CD28 signaling domain with cells modified with a CD137 T-cell signaling domain in the absence of co-stimulation (FIG. 30). Thus, metabolic demand and consumption patterns of unmodified and modified T cells grown *ex vivo* are different and become prominent when cells are propagated *ex vivo* in the absence of specific co-stimulation. Parallel to this, it was shown in a mouse model that maintaining mitochondrial function and SRC (Spare Respiratory Capacity) in CD8+ T cells is key to stable CD8⁺ memory T cell formation after infection (Pearce *et al.*, 2013; van der Windt *et al.*, 2012). SRC is described as the extra mitochondrial capacity available in a cell to produce energy under conditions of increased work or stress and is thought to be important for long-term cellular survival and function. A series of experiments was performed *in vitro* to determine whether T cells with high mitochondrial strength could survive better, and thereby be more efficacious *in vivo,* as compared to T cells with regular mitochondrial mass.

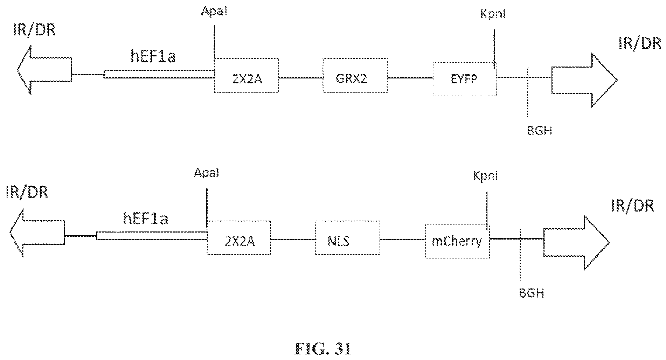

**Genetic modification of T cells to express a fluorescent reporter protein.** A fluorescent reporter protein EYFP-GRX2 (FIG. 31) was developed and expressed on T cells using Sleeping Beauty-mediated transposition as previously described (Rushworth *et al*., 2014). Plasmid EYFP-GRX2-SB encodes for a fusion protein EYFP-Mito expressed under the control of the hEFla promoter, which is all embedded on a Sleeping Beauty (SB) vector backbone. The SB vector IR/DR sequence helps in transposition of the transgene encoding the fluorescent protein with a mitochondrial localization sequence (GRX2) guided by enzyme SB11 (transposase) expressed from a second plasmid pCMV-Kan-SB11. Along with the fluorescent protein reporter and SB11, two more plasmids are expressed, one that encodes for a chimeric antigen receptor (CAR) specific to a tumor-associated antigens (such as, HER1-3, ROR1, CD19, or CD123) and one that contains a second fluorescent protein, mCherry, fused with a nuclear localization sequence (NLS) that guides the fluorescent protein to the cell nucleus (*i.e*., mCherry-NLS-SB). All four plasmids combined were delivered to the cell by electroporation using a human T-cell nucleofector kit (Amaxa/Lonza) and electroporation device (Lonza). The schematic of vector diagram to express the fluorescent protein plasmid are depicted in FIG. 31.

**Generation of CAR⁺ T cells.** Chimeric antigen receptor-positive T cells targeting specific tumor-associated antigens were generated by similar methods of SB-mediated transposition and DNA transfer by electroporation to PBMC-derived T cells. CAR+ T cells with specificity targeting to CD19 and/or ROR1 on B-cell malignancies, CD123 on leukemic stem cells (Acute Myeloid Leukemia), or HER1-3 on EGFR-positive breast cancer were designed and expressed on T cells by similar methods of electroporation. Each CAR was designed to contain either T-cell co-stimulatory endodomain CD28 or 4-1BB (CD137) along with CD3z. Each CAR contains a modified IgG4-Fc stalk, which serves as a scaffold to protrude the scFv on the surface of the cell, fused to the T-cell signaling endodomain. Expression of the CAR on the T-cell surface was confirmed using an Fc-stalk specific antibody.

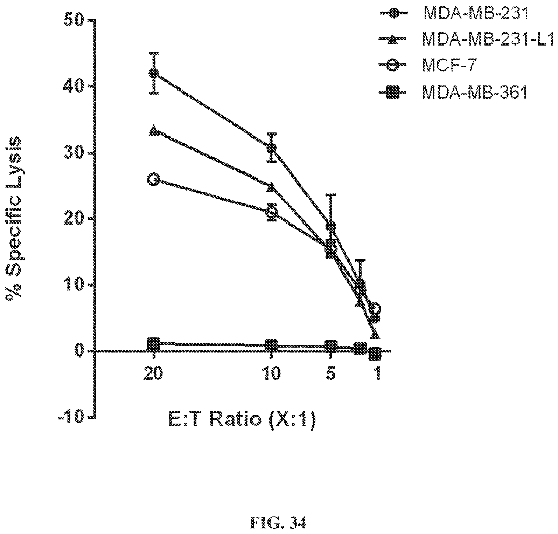

Methods to design and generate CAR species (see, *e.g*., International (PCT) Patent Publication No. WO/2015/123642, which is incorporated herein by reference) are known, but predicting which *in vitro* or animal test(s) will identify a CAR design with superior therapeutic potential when infused in humans remains a challenge. Currently, investigators typically rely on an array of the following tests to advance CAR structures to the clinic: resistance to activation-induced cell death (AICD) and CAR-mediated proliferation, killing, and cytokine production. Taught herein is a single test to identify CAR design that may have improved therapeutic potential. CD8⁺ memory T cells (but not CD8⁺ effector T cells) possess substantial mitochondrial spare respiratory capacity (SRC). Memory cells, unlike effector cells, have unique sets of metabolic demand. In the case of increased stress during high tumor burden, mitochondria of genetically modified T cells convert from an orthodox structure to a condensed form (FIGS. 29B and 33A-33B). However, only some cells retain the flexibility to switch back to the original orthodox mitochondrial structure, a characteristic feature of T effector cells. T cells with high mitochondrial SRC survive adverse conditions associated with high tumor burden, such as hypoxia, lack of nutrients for glycolysis, and suppressive cytokine milieu, but still could retain their cytotoxic ability (FIG. 34). Such cytotoxic T cells are defined herein as "high energy cells" which could be the best serial killers of tumors *in vivo.*

***Ex vivo* expansion of CAR⁺ T cells.** CAR-T cells were co-cultured along with irradiated K562 HLA ^{Cneg} cells expressing a CAR-specific ligand (2D3 scFv, see, *e.g*., PCT Application No. PCT/US2014/039365) and exogenous addition of IL2 but absence of co-stimulation (Rushworth *et al.,* 2014). After genetic modification of CAR-T cells, irradiated, activated, and propagated (AaPC) feeder cells were added at a ratio of 1:2 (1 CAR-T cell: 2 AaPC) (FIG. 30).

Metabolically active high-energy CAR⁺ T cells that can survive long-term *in vivo* in human trials were identified and numerically expanded. This was achieved by employing the *Sleeping Beauty* (SB) system to mediate gene transfer. T cells that had undergone electro-transfer of two DNA plasmids from the SB system (coding for CAR and SB transposase) were co-cultured on irradiated "universal" activating and propagating cells (K562-uAPC). To identify the genetically-modified T cells with the ability to participate in "high energy" metabolism, a third SB DNA species, designated EYFP-GRX2-SB, was also electroporated in parallel. This SB transposon expresses the fusion protein EYFP-2A-GRX2 and contains a mitochondrial-localization sequence. The fluorescence reporter identifies CAR⁺ T cells with extra mitochondrial spare respiratory capacity (SRC). These cells could be sorted based on the intensity of EYFP fluorescence and infused for tumor killing *in vivo.*

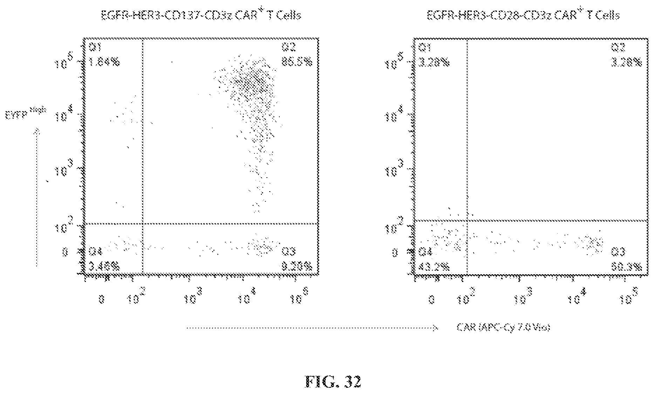

**Flow cytometry.** CAR-T cells were sorted for high mitochondrial strength based on endogenous expression of EYFP on a LSR-Fortessa Flow cytometer (BD) without any staining marker as per standard sorting procedure (FIG. 32).

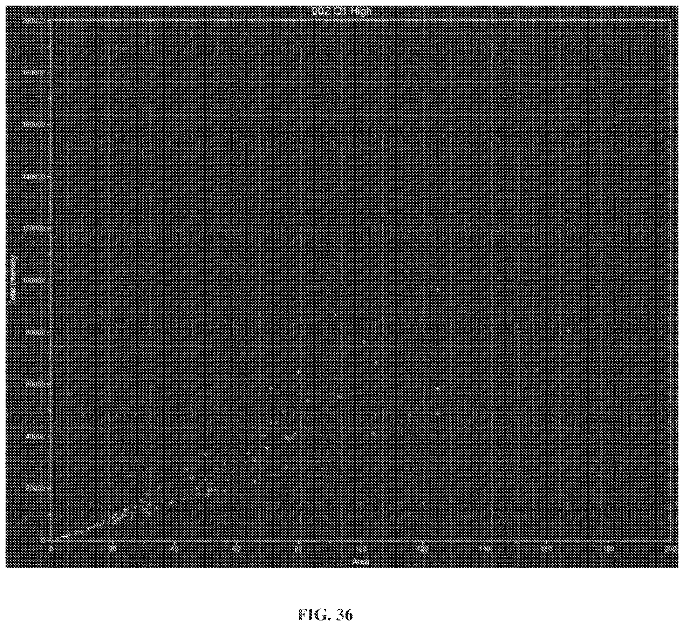

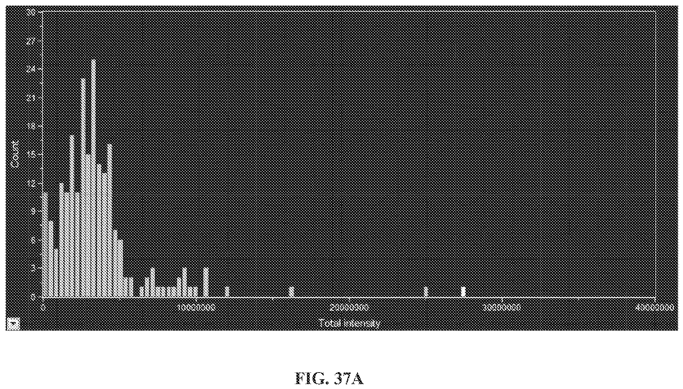

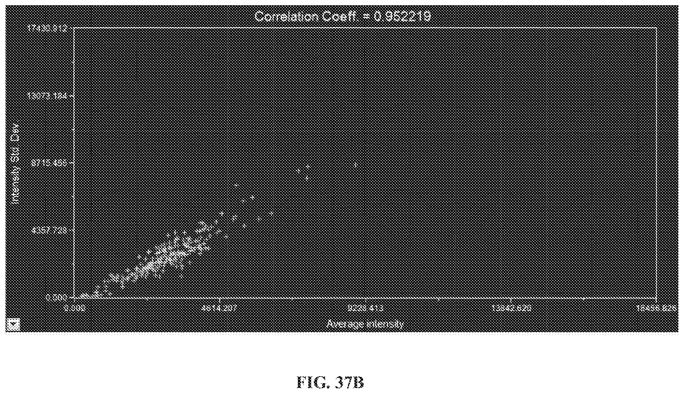

**Microscopy.** The CAR modified cells were fixed in 1% paraformaldehyde solution and analyzed by transmission electron microscopy using standard procedures. The fluorescence intensity of CAR modified and unmodified T cells was quantified using a Leica DMI 6000 inverted fluorescence microscope and Metamorph imaging software (Version 7.8) to determine whether high mitochondrial strength CAR-modified T cells (eT-cells) possess extra mitochondrial spare respiratory capacity (FIGS. 35, 36, 37A, and 37B).

CARs with high EYFP expression may have the requisite characteristics to by-pass apoptosis, up-regulate anti-apoptotic genes in CAR⁺ T cells, and persist *in vivo* to achieve clinically relevant anti-tumor effects. It may be desirable to transiently express the reporter gene from electro-transferred *in vitro*-transcribed mRNA species and/or express a cell-surface molecule (*e.g*., truncated nerve growth factor receptor) that can be used for paramagnetic bead-based selection. *In lieu* of sorting for human applications of a certain CAR design, the method provides an approach whereby large numbers of different CAR molecules (*e.g*., as produced by the EZ-CAR system) can be screened *in vitro* for co-expression of a reporter gene (*e.g*., EYFP) and thus selected based on a desired mitochondrial SRC.

### Example 8 - 4-1BB (CD137) mediated T-cell signaling

Gene modification and *ex vivo* propagation of CAR⁺ T cells cause altered mitochondrial metabolism and that 4-1BB mediated T-cell signaling promotes better survival of CAR⁺ T cells in a challenging culture environment.

Two different CARs targeting either ROR1 or HER1-3 were compared to determine the effect of signaling domain on growth of CAR-T cells in a limiting culture condition. CAR constructs were built on a sleeping beauty (SB) vector backbone as described previously. CARs employed either CD28-CD3z signaling or CD137 (4-1BB)-CD3z signaling domain in respective vector constructs. Besides targeting separate tumor antigens, CAR backbone remained constant as described earlier for CD19-specific CAR that utilizes a mouse scFv targeting tumor antigen and a human IgG4 Fc derived stalk to join CAR with transmembrane and signaling domain. The target antigens were described previously *i.e.* ROR1 on CLL (Deniger et al., PLoS One, 2015), and HER1-3 (Jena et al., ASH, 2014) on breast tumors. After SB-mediated gene modification, CAR-T cells were propagated on an irradiated universal activating and propagating cells (uAPC; Rushworth et al., J Immunothrer. 2014). CAR-T cells growth kinetics, mitochondrial structure and mitochondrial morphology was studied as described in Example 7.

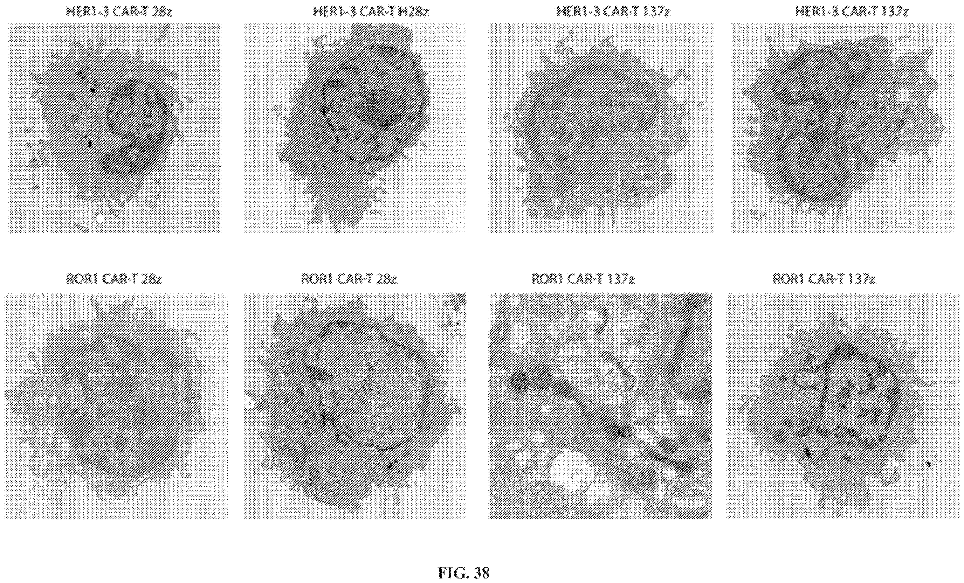

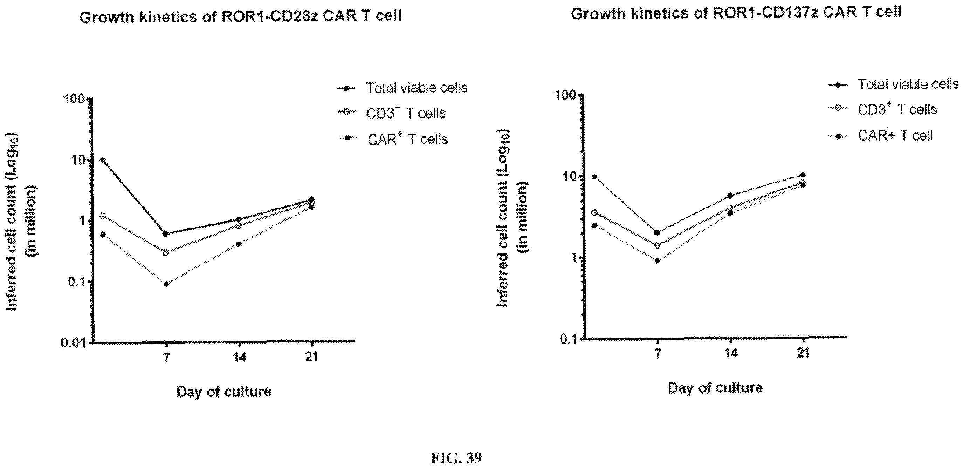

Growth and survival of CAR-T cells containing CD28-CD3z signaling is inferior as compared to CD137-CD3z signaling irrespective of the transposon load or target antigen employed (FIGS. 38 and 39). In total, 3 different CARs were tested in this study, namely, CD19-CAR, HER1-3 CAR, ROR1⁺ CAR containing either CD28z or CD137z signaling. All CARs were activated and expanded on universal AaPC, where the T cells signaled only through the CAR-stalk via a scFv ligand embedded on K562 cells. In the absence of specific co-stimulation CAR-T cells containing CD28 signaling rapidly underwent apoptosis, whereas CAR-T cells containing CD137z signaling persisted longer and consistently showed increased mitochondrial mass (as observed of high EYFP strength by confocal microscopy).

Mitochondrial numbers were few and scarcely distributed in CD28z-CAR-T cells whereas CD137z-CAR-T cells registered brighter and higher levels of EYFP signal. This attests to the high spare respiratory capacity (SRC) of the mitochondria and provides mechanistic evidence of CD137z-CAR T cells surviving in a limiting culture environment.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### REFERENCES

The following references, to the extent that they provide exemplary procedural or other details supplementary to those set forth herein, are specifically incorporated herein by reference.
U.S. Patent No. 4,690,915
U.S. Patent No. 6,225,042
U.S. Patent No. 6,355,479
U.S. Patent No. 6,362,001
U.S. Patent No. 6,410,319
U.S. Patent No. 6,790,662
U.S. Patent No. 7,109,304
U.S. Patent Application Publication No. 2009/0017000
U.S. Patent Application Publication No. 2009/0004142
PCT Publication No. WO2007/103009
PCT Application No. PCT/US2014/039365
PCT Application No. PCT/US14/39365
PCT Application No. PCT/US 14/38005
Altenschmidt et al., J. Mol. Med., 75:259, 1997.
Barthel and Goldfeld, J. Immunol., 171:3612-3619, 2003.
Brentjens et al., Blood, 118:4817-4828, 2011.
Brentjens et al., Sci. Transl. Med., 5:177ra138, 2013.
Brocker et al., Adv. Immunol., 68:257, 1998.
Cooper and Bollard, Blood, 119:2700-2702, 2012.
Cooper et al., Blood 105:1622-31, 2005.
Corrigan-Curay et al., Molecular Therapy. 22, 1564-1574, 2014.
Deniger et al., PLoS One. 2015;10(6):e0128151. doi: 10.1371/joumal.pone.0128151.
Eshhar et al., Proc. Natl. Acad. Sci. U.S.A., 90:720, 1993.
Eshhar, Cancer Immunol. Immunother., 45:131, 1997.
Fitzer-Attas et al., J. Immunol., 160:145, 1998.
Frauwirth et al., Immunity, 16:769-777, 2002.
Gross et al., FASEB J., 6:3370, 1992.
Grupp et al., New Eng. J. of Med., 18:1509-1518, 2013.
Hackett et al., Mol Ther 18:674-83,2010.
Hekele et al., Int. J. Cancer, 68:232, 1996.
Hwu et al., Cancer Res., 55:3369, 1995.
Jena et al., Blood, 116:1035-1044, 2010.
Jena et al., Curr. Hematol. Malig. Rep., 9:50-56, 2014.
Jena et al., 2014. Abstract. Blood. 124 (21).
Jin et al., Gene Ther 18:849-56, 2011.
Kalos et al., Sci. Transl. Med., 3:95ra73, 2011.
Kebriaei et al., Hum Gene Ther 23:444-50, 2012.
Kim et al., Nature, 22(4):403-410, 2004.
Kochenderfer et al., Blood, 116:4099-4102, 2010.
Kochenderfer et al., Blood, 119:2709-2720, 2012.
Krauss et al., Immunity, 15:497-502, 2001.
Marodon et al., Blood, 101:3416-3423, 2003.
Maude et al., N. Engl J. Med., 371:1507-1517, 2014.
Moritz et al., Proc. Natl. Acad. Sci. U. S. A., 91:4318, 1994.
Pearce et al., Science, 342:210, 2013.
Porter et al., N. Engl. J. Med., 365:725-733, 2011.
Roberts et al., Blood, 84:2878, 1994.
Rushworth et al., J. of Immunotherapy. 37:204-213, 2014.
Schneider, J. Embryol Exp. Morph., 27:353-365, 1972.
Stancovski et al., J. Immunol., 151:6577, 1993.
Till et al., Blood, 112:2261-2271, 2008.
Topalian and Rosenberg, Acta Haematol., 78(Suppl. 1):75-76, 1987.
van der Windt et al., Immunity, 36:68-78, 2012.
Weijtens et al., J. Immunol., 157:836, 1996.

The invention is also defined by the following embodiments:
1. A chimeric antigen receptor (CAR) polypeptide comprising an antigen binding domain; a hinge domain; a transmembrane domain and one or more intracellular signaling domain(s), said hinge domain comprising an IgG4-Fc sequence wherein, said IgG4-Fc sequence comprises at least one mutation relative to wild type IgG4-Fc that reduces Fc-receptor binding.
2. The CAR polypeptide of embodiment 1, wherein the IgG4-Fc sequence is at least about 90% identical to SEQ ID NO: 1.
3. The CAR polypeptide of embodiment 1, wherein the IgG4-Fc sequence comprises a mutation at position L235 or N297 relative to the wild type sequence.
4. The CAR polypeptide of embodiment 1, wherein the IgG4-Fc sequence comprises a mutation at positions L235 and N297 relative to the wild type sequence.
5. The CAR polypeptide of embodiment 1, wherein the IgG4-Fc sequence comprises a L235E or N297Q mutation relative to the wild type sequence.
6. The CAR polypeptide of embodiment 1, wherein the IgG4-Fc sequence comprises a L235E and N297Q mutation relative to the wild type sequence.
7. The CAR polypeptide of embodiment 1, wherein the IgG4-Fc sequence is identical to SEQ ID NO:1.
8. The CAR polypeptide of embodiment 1, wherein antigen binding domain comprises an scFv.
9. The CAR polypeptide of embodiment 1, wherein the intracellular cell signaling domain comprises a domain from CD3ζ.
10. The CAR polypeptide of embodiment 9, wherein the intracellular cell signaling domain further comprises an intracellular domain from CD28 or CD137 (4-1BB).
11. The CAR polypeptide of embodiment 1, wherein the transmembrane domain comprises a transmembrane domain of CD28, CD8a or CD137.
12. The CAR polypeptide of embodiment 1, wherein the antigen binding domain binds to an infectious disease antigen or a cancer-cell antigen.
13. The CAR polypeptide of embodiment 12, wherein the antigen binding domain binds to a cancer-cell antigen.
14. The CAR polypeptide of embodiment 13, wherein the cancer cell antigen is CD19 and the CAR is a CD19-targeted CAR.
15. A nucleic acid molecule encoding a CAR polypeptide in accordance with any one of embodiments 1-14.
16. An isolated immune effector cell comprising a CAR polypeptide in accordance with any one of embodiments 1-14 or a nucleic acid of embodiment 15.
17. The cell of embodiment 6, wherein the cell is a T-cell.
18. The cell of embodiment 6, wherein the cell is a human cell.
19. A pharmaceutical composition comprising a population of cells in accordance with embodiment 6 in a pharmaceutically acceptable carrier.
20. A method of treating a subject comprising administering an effective amount chimeric antigen receptor (CAR) T-cells that expresses a CAR polypeptide in accordance with any one of embodiments 1-14.
21. An engineered antigen presenting cell (APC) comprising a first transgene encoding a target antigen and a second transgene encoding a human leukocyte antigen (HLA), said HLA being expressed on the surface of the APC in complex with an epitope of the target antigen.
22. The engineered APC of embodiment 21, wherein the APC is not immortalized.
23. The engineered APC of embodiment 21, wherein the APC is a primary cell.
24. The engineered APC of embodiment 21, wherein the APC is a T-cell or T-cell progenitor.
25. The engineered APC of embodiment 21, wherein the APC is a T-cell expressing a TCRαβ or a TCRγδ.
26. The engineered APC of embodiment 21, further comprising at least a third transgene encoding a co-stimulatory molecule.
27. The engineered APC of embodiment 21, wherein the target antigen is NY-ESO-1.
28. A method of treating a subject having a disease comprising administering an effective amount of engineered APCs in accordance with any one of embodiments 21-26 to the subject, wherein said APCs express a target antigen associated with the disease.
29. The method of embodiment 28, wherein the subject has a cancer that expresses the target antigen encoded by the APCs.
30. The method of embodiment 28, wherein target antigen is NY-ESO-1.
31. A method of selecting CAR-modified T cells with high mitochondrial spare respiratory capacity comprising detecting an expression level of a mitochondrial reporter gene and selecting CAR-modified T cells with an elevated expression level of the mitochondrial reporter gene, thereby selecting CAR-modified T cells with mitochondrial spare respiratory capacity.
32. The method of embodiment 31, wherein the mitochondrial reporter gene is an endogenous gene.
33. The method of embodiment 31, wherein the mitochondrial reporter gene is an exogenous gene.
34. The method of embodiment 33, wherein the exogenous gene encodes a fluorescent reporter protein.
35. The method of embodiment 34, wherein the fluorescent reporter protein comprises a mitochondrial localization sequence.

## Claims

1. An engineered antigen presenting cell (APC) comprising a first transgene encoding a target antigen and a second transgene encoding a human leukocyte antigen (HLA), said HLA being expressed on the surface of the APC in complex with an epitope of the target antigen.

2. The engineered APC of claim 1, wherein the APC is not immortalized.

3. The engineered APC of claim 1, wherein the APC is a primary cell.

4. The engineered APC of claim 1, wherein the APC is a T-cell or T-cell progenitor.

5. The engineered APC of claim 1, wherein the APC is a T-cell expressing a TCRαβ or a TCRγδ.

6. The engineered APC of claim 1, further comprising at least a third transgene encoding a co-stimulatory molecule.

7. The engineered APC of claim 1, wherein the HLA is HLA-A2.

8. The engineered APC of claim 1, wherein the HLA is HLA-A, HLA-B, HLA-C or HLA-DRB 1.

9. The engineered APC of claim 1, wherein the target antigen is ALK, BCMA, CA9 (carbonic anhydrase IX), CD5, CD19, CD22, CD30, CD33, CD44, CD70, CD38, CD123, CD133, CD171, CD174, CD274, CD276, CEA, CEACAM5, CSPG4, c-Met, EGFR, EGFRvIII, EpCAM, EPHA2, ERBB2, FAP, Folate receptors, FOLH1, GD2, GD3, GFRalpha4, GM1, GPC3, GPRC5D, GPNMB, HER-2/neu, HERVs, HGFR, IL13Ralpha2, Ig kappa, KDR, LeY, L1CAM, Mesothelin, MUC1, MUC16, MUC16 glycosylated variants, NeuGc-GM3 (N-glycolyl GM3), O-acetyl-GD2, NKG2D ligand, PDCD1, PDGFR, Protein L, PSCA, PSMA, ROR1, SLAMF7, SSEA-4, TEM1, TRP-2, ULBP1, ULBP2, VEGFR2 or WT1.

10. The engineered APC of claim 6, wherein the co-stimulatory molecule is a cytokine.

11. The engineered APC of claim 10, wherein the cytokine is a membrane-bound Cγ cytokine.

12. The engineered APC of claim 11, wherein the cytokine is membrane bound IL-15.

13. The engineered APC of claim 1, further comprising an edited or deleted PD-1, LIM-3, CTLA-4 or TCR.

14. A method of propagating antigen-specific immune effector cells comprising culturing the antigen-specific immune effector cells with the engineered APC of any one of claims 1-13.

15. The method of claim 14, wherein the antigen-specific immune effector cells are T-cells, NK cells or NK T-cells.

16. The method of claim 14, wherein the antigen-specific immune effector cells are expressing a CAR or a TCR.
